(19) Europäisches Patentamt
European Patent Office
Office européen des brevets

(11) EP 4 455 142 A1

(12) EUROPEAN PATENT APPLICATION

(43) Date of publication:
**30.10.2024 Bulletin 2024/44**

(21) Application number: **23170082.4**

(22) Date of filing: **26.04.2023**

(51) International Patent Classification (IPC):
***C07D 413/14*** *(2006.01)* ***C07D 417/14*** *(2006.01)*
***A61P 35/00*** *(2006.01)* ***A61K 31/427*** *(2006.01)*
***A61K 31/422*** *(2006.01)*

(52) Cooperative Patent Classification (CPC):
**C07D 413/14; A61P 35/00; C07D 417/14**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Scenic Immunology B.V.**
**1098 XH Amsterdam (NL)**

(72) Inventor: **The designation of the inventor has not yet been filed**

(74) Representative: **Nederlandsch Octrooibureau**
**P.O. Box 29720**
**2502 LS The Hague (NL)**

Remarks:
The complete document including Reference Table(s) and the Sequence Listing(s) can be downloaded from the EPO website

# (54) NOVEL INHIBITOR OF QPCTL AND/OR QC

(57) The invention relates to a compound represented by formula (I), or a pharmaceutically acceptable salt, hydrate or solvate thereof, preferably for use as a medicament, more preferably for use in the treatment of cancer.

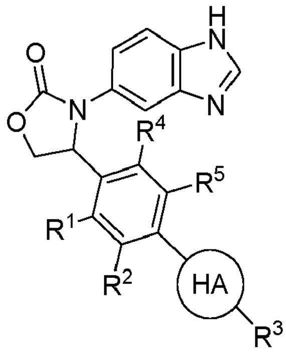

(I)

EP 4 455 142 A1

## Description

### Field

The invention relates to a compound represented by formula (I), or a pharmaceutically acceptable salt, hydrate or solvate thereof, preferably for use as a medicament, more preferably for use in the treatment of cancer.

### Background

Cancer is a leading cause of death worldwide. One treatment option may be cancer immunotherapy, which leverages the body's natural immune defences. It boosts the ability of the immune system, including the innate immune system, to specifically detect and destroy cancer cells (e.g. via phagocytosis) while leaving healthy cells unaffected.

However, cancer cells are able to evade immune surveillance in many ways, for instance, by evading phagocytosis by e.g., macrophages or neutrophils through the expression of so-called "anti-phagocytic" or "don't eat me" signals. One prominent example of such signal is the interaction between CD47 (overexpressed on the surface of tumor cells) and SIRPa (in the macrophage membrane).

It has been established that binding of SIRPa to CD47 depends on pyroglutamylation of the N-terminal glutamine moiety of CD47 by the glutaminyl-peptide cyclotransferase-like (isoQC) enzyme. Inhibition of isoQC may thus supress the anti-phagocytic signal shown by cancer cells eluding immunotherapy, thereby promoting an effective therapy.

Several small molecule isoQC inhibitors have been described (e.g. in WO2021009068), although the potency of most of these compounds is limited. Moreover these compounds often have low metabolic stability and low kinetic solubility, limiting their therapeutic application.

Hence, there is an ongoing need in the art for small molecule compounds that inhibit isoQC with high potency, metabolic stability and high kinetic solubility.

### Description of the invention

#### *Compounds*

In a first aspect, the invention provides a compound represented by formula (I), or a pharmaceutically acceptable salt, hydrate or solvate thereof:

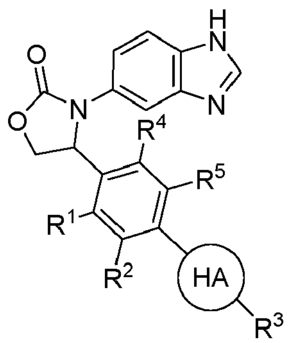

(I).

wherein $R^1$, $R^2$, $R^4$ and $R^5$ are independently H, a halogen, or a pseudohalogen, preferably H or a halogen;
wherein HA is a monocyclic heteroaryl;
wherein $R^3$ is a C1-C6 haloalkyl, preferably a C1-C4 haloalkyl.

A compound according to this first aspect may be called a compound according to or of the invention. Wherever a reference is made to a compound according to or of the invention, reference is made to both the compound and to a pharmaceutically acceptable salt, hydrate or solvate thereof.

A compound according to the invention has a high potency to inhibit isoQC and/or QC, as can be determined using Assay 1 (for isoQC), Assay 2 (for QC) and Assay 3 (pyroglutamylation in live cells) of Example 3. Moreover, a compound according to the invention has a high metabolic stability, as evidenced by microsomal stability in Example 5, and a high kinetic solubility, as evidenced in Example 6. Specifically, a compound according to the invention has a higher potency to inhibit isoQC and/or QC, a higher metabolic stability, and a higher kinetic solubility than reference compounds

(17)-(19) of WO2021009068.

*PHENYL GROUP SUBSTITUTION ($R^1$, $R^2$, $R^4$, $R^5$)*

A compound according to the invention comprises a phenyl group substituted by a 1,3-oxazolidin-2-one and HA in a para configuration. This phenyl group is further substituted with $R^1$, $R^2$, $R^4$ and $R^5$.

$R^1$, $R^2$, $R^4$ and $R^5$ are independently H, a halogen, or a pseudohalogen.

A halogen is F, Cl, Br, I, or At. Preferably, a halogen is F, Cl, Br, or I. More preferably, a halogen is F, Cl, or Br. Even more preferably, a halogen is F or Cl. Most preferably, a halogen is F.

A pseudohalogen is -CN, -CP, -NC, -OH, -SH, -SeH,-TeH, -OCN, -SCN, -NCS, -SeCN, -TeCN, $-N_3$, -NO, or -NOz. Preferably, a pseudohalogen is -CN, -NC, -OH, -SH, -OCN, - SCN, -NCS, $-N_3$, -NO, or -NOz.

Preferably, $R^1$, $R^2$, $R^4$ and $R^5$ are independently H or a halogen. Preferably, $R^1$, $R^2$, $R^4$ and $R^5$ are independently H, F, Cl, Br, or I. More preferably, $R^1$, $R^2$, $R^4$ and $R^5$ are independently H, F, Cl, or Br. Even more preferably, $R^1$, $R^2$, $R^4$ and $R^5$ are independently H, F, or Cl. Most preferably, $R^1$, $R^2$, $R^4$ and $R^5$ are independently H or F.

In embodiments, $R^1$ is H. Preferably, $R^1$ is H and $R^2$, $R^4$ and $R^5$ are independently a halogen or a pseudohalogen. More preferably, $R^1$ is H and $R^2$, $R^4$ and $R^5$ are independently a halogen. Even more preferably, $R^1$ is H and $R^2$, $R^4$ and $R^5$ are independently F or Cl. Most preferably, $R^1$ is H and $R^2$, $R^4$ and $R^5$ are F.

In embodiments, $R^2$ is H. Preferably, $R^2$ is H and $R^1$, $R^4$ and $R^5$ are independently a halogen or a pseudohalogen. More preferably, $R^2$ is H and $R^1$, $R^4$ and $R^5$ are independently a halogen. Even more preferably, $R^2$ is H and $R^1$, $R^4$ and $R^5$ are independently F or Cl. Most preferably, $R^2$ is H and $R^1$, $R^4$ and $R^5$ are F.

In embodiments, $R^4$ is H. Preferably, $R^4$ is H and $R^1$, $R^2$ and $R^5$ are independently a halogen or a pseudohalogen. More preferably, $R^4$ is H and $R^1$, $R^2$ and $R^5$ are independently a halogen. Even more preferably, $R^4$ is H and $R^1$, $R^2$ and $R^5$ are independently F or Cl. Most preferably, $R^4$ is H and $R^1$, $R^2$ and $R^5$ are F.

In embodiments, $R^5$ is H. Preferably, $R^5$ is H and $R^1$, $R^2$ and $R^4$ are independently a halogen or a pseudohalogen. More preferably, $R^5$ is H and $R^1$, $R^2$ and $R^4$ are independently a halogen. Even more preferably, $R^5$ is H and $R^1$, $R^2$ and $R^4$ are independently F or Cl. Most preferably, $R^5$ is H and $R^1$, $R^2$ and $R^4$ are F.

In embodiments, $R^1$ and $R^2$ are H. Preferably, $R^1$ and $R^2$ are H and $R^4$ and $R^5$ are independently a halogen or a pseudohalogen. More preferably, $R^1$ and $R^2$ are H and $R^4$ and $R^5$ are independently a halogen. Even more preferably, $R^1$ and $R^2$ are H and $R^4$ and $R^5$ are independently F or Cl. Most preferably, $R^1$ and $R^2$ are H and $R^4$ and $R^5$ are F.

In embodiments, $R^1$ and $R^4$ are H. Preferably, $R^1$ and $R^4$ are H and $R^2$ and $R^5$ are independently a halogen or a pseudohalogen. More preferably, $R^1$ and $R^4$ are H and $R^2$ and $R^5$ are independently a halogen. Even more preferably, $R^1$ and $R^4$ are H and $R^2$ and $R^5$ are independently F or Cl. Most preferably, $R^1$ and $R^4$ are H and $R^2$ and $R^5$ are F.

In embodiments, $R^1$ and $R^5$ are H. Preferably, $R^1$ and $R^5$ are H and $R^2$ and $R^4$ are independently a halogen or a pseudohalogen. More preferably, $R^1$ and $R^5$ are H and $R^2$ and $R^4$ are independently a halogen. Even more preferably, $R^1$ and $R^5$ are H and $R^2$ and $R^4$ are independently F or Cl. Most preferably, $R^1$ and $R^5$ are H and $R^2$ and $R^4$ are F.

In embodiments, $R^2$ and $R^4$ are H. Preferably, $R^2$ and $R^4$ are H and $R^1$ and $R^5$ are independently a halogen or a pseudohalogen. More preferably, $R^2$ and $R^4$ are H and $R^1$ and $R^5$ are independently a halogen. Even more preferably, $R^2$ and $R^4$ are H and $R^1$ and $R^5$ are independently F or Cl. Most preferably, $R^2$ and $R^4$ are H and $R^1$ and $R^5$ are F.

In embodiments, $R^2$ and $R^5$ are H. Preferably, $R^2$ and $R^5$ are H and $R^1$ and $R^4$ are independently a halogen or a pseudohalogen. More preferably, $R^2$ and $R^5$ are H and $R^1$ and $R^4$ are independently a halogen. Even more preferably, $R^2$ and $R^5$ are H and $R^1$ and $R^4$ are independently F or Cl. Most preferably, $R^2$ and $R^5$ are H and $R^1$ and $R^4$ are F.

In embodiments, $R^4$ and $R^5$ are H. Preferably, $R^4$ and $R^5$ are H and $R^1$ and $R^2$ are independently a halogen or a pseudohalogen. More preferably, $R^4$ and $R^5$ are H and $R^1$ and $R^2$ are independently a halogen. Even more preferably, $R^4$ and $R^5$ are H and $R^1$ and $R^2$ are independently F or Cl. Most preferably, $R^4$ and $R^5$ are H and $R^1$ and $R^2$ are F.

In embodiments, $R^1$, $R^2$, and $R^4$ are H. Preferably, $R^1$, $R^2$, and $R^4$ are H and $R^5$ is a halogen or a pseudohalogen. More preferably, $R^1$, $R^2$, and $R^4$ are H and $R^5$ is a halogen. Even more preferably, $R^1$, $R^2$, and $R^4$ are H and $R^5$ is For Cl. Most preferably, $R^1$, $R^2$, and $R^4$ are H and $R^5$ is F.

In embodiments, $R^1$, $R^2$, and $R^5$ are H. Preferably, $R^1$, $R^2$, and $R^5$ are H and $R^4$ is a halogen or a pseudohalogen. More preferably, $R^1$, $R^2$, and $R^5$ are H and $R^4$ is a halogen. Even more preferably, $R^1$, $R^2$, and $R^5$ are H and $R^4$ is For Cl. Most preferably, $R^1$, $R^2$, and $R^5$ are H and $R^4$ is F.

In embodiments, $R^1$, $R^4$, and $R^5$ are H. Preferably, $R^1$, $R^4$, and $R^5$ are H and $R^2$ is a halogen or a pseudohalogen. More preferably, $R^1$, $R^4$, and $R^5$ are H and $R^2$ is a halogen. Even more preferably, $R^1$, $R^4$, and $R^5$ are H and $R^2$ is For Cl. Most preferably, $R^1$, $R^4$, and $R^5$ are H and $R^2$ is F.

In embodiments, $R^2$, $R^4$, and $R^5$ are H. Preferably, $R^2$, $R^4$, and $R^5$ are H and $R^1$ is a halogen or a pseudohalogen. More preferably, $R^2$, $R^4$, and $R^5$ are H and $R^1$ is a halogen. Even more preferably, $R^2$, $R^4$, and $R^5$ are H and $R^1$ is For Cl. Most preferably, $R^2$, $R^4$, and $R^5$ are H and $R^1$ is F.

In embodiments, $R^1$, $R^2$, $R^4$, and $R^5$ are H.

In embodiments, at least one of $R^1$, $R^2$, $R^4$, and $R^5$ is a halogen or a pseudohalogen. Preferably, at least one

of $R^1$, $R^2$, $R^4$, and $R^5$ is a halogen, while the remainder of $R^1$, $R^2$, $R^4$, and $R^5$ are H. More preferably, at least one of $R^1$, $R^2$, $R^4$, and $R^5$ is F or Cl, while the remainder of $R^1$, $R^2$, $R^4$, and $R^5$ are H. Most preferably, at least one of $R^1$, $R^2$, $R^4$, and $R^5$ is F, while the remainder of $R^1$, $R^2$, $R^4$, and $R^5$ are H.

In embodiments, at least two of $R^1$, $R^2$, $R^4$, and $R^5$ are independently a halogen or a pseudohalogen. Preferably, at least two of $R^1$, $R^2$, $R^4$, and $R^5$ are a halogen, while the remainder of $R^1$, $R^2$, $R^4$, and $R^5$ are H. More preferably, at least two of $R^1$, $R^2$, $R^4$, and $R^5$ are independently F or Cl, while the remainder of $R^1$, $R^2$, $R^4$, and $R^5$ are H. Most preferably, at least two of $R^1$, $R^2$, $R^4$, and $R^5$ are F, while the remainder of $R^1$, $R^2$, $R^4$, and $R^5$ are H.

In embodiments, at least three of $R^1$, $R^2$, $R^4$, and $R^5$ are independently a halogen or a pseudohalogen. Preferably, at least three of $R^1$, $R^2$, $R^4$, and $R^5$ are a halogen, while the remainder of $R^1$, $R^2$, $R^4$, and $R^5$ are H. More preferably, at least three of $R^1$, $R^2$, $R^4$, and $R^5$ are independently F or Cl, while the remainder of $R^1$, $R^2$, $R^4$, and $R^5$ are H. Most preferably, at least three of $R^1$, $R^2$, $R^4$, and $R^5$ are F, while the remainder of $R^1$, $R^2$, $R^4$, and $R^5$ are H.

In embodiments, one of $R^1$, $R^2$, $R^4$, and $R^5$ is a halogen or a pseudohalogen, while the remainder of $R^1$, $R^2$, $R^4$, and $R^5$ are H. Preferably, one of $R^1$, $R^2$, $R^4$, and $R^5$ is For Cl, while the remainder of $R^1$, $R^2$, $R^4$, and $R^5$ are H. More preferably, one of $R^1$, $R^2$, $R^4$, and $R^5$ is F, while the remainder of $R^1$, $R^2$, $R^4$, and $R^5$ is H.

In embodiments, two of $R^1$, $R^2$, $R^4$, and $R^5$ are independently a halogen or a pseudohalogen, while the remainder of $R^1$, $R^2$, $R^4$, and $R^5$ are H. Preferably, two of $R^1$, $R^2$, $R^4$, and $R^5$ are independently F or Cl, while the remainder of $R^1$, $R^2$, $R^4$, and $R^5$ are H. More preferably, two of $R^1$, $R^2$, $R^4$, and $R^5$ are F, while the remainder of $R^1$, $R^2$, $R^4$, and $R^5$ is H.

In embodiments, three of $R^1$, $R^2$, $R^4$, and $R^5$ are independently a halogen or a pseudohalogen, while the remainder of $R^1$, $R^2$, $R^4$, and $R^5$ are H. Preferably, three of $R^1$, $R^2$, $R^4$, and $R^5$ are independently F or Cl, while the remainder of $R^1$, $R^2$, $R^4$, and $R^5$ are H. More preferably, three of $R^1$, $R^2$, $R^4$, and $R^5$ are F, while the remainder of $R^1$, $R^2$, $R^4$, and $R^5$ is H.

In embodiments, $R^1$ is a halogen or a pseudohalogen and $R^2$ is H; or $R^{1'}$ is H and $R^2$ is a halogen or a pseudohalogen; optionaly wherein $R^4$ is H and $R^5$ is H. Preferably, $R^1$ is a halogen and $R^2$ is H; or $R^1$ is H and $R^2$ is a halogen; optionaly wherein $R^4$ is H and $R^5$ is H. More preferably, $R^1$ is F or Cl and $R^2$ is H; or $R^1$ is H and $R^2$ is F or Cl; optionaly wherein $R^4$ is H and $R^5$ is H. Most preferably, $R^1$ is F and $R^2$ is H; or $R^1$ is H and $R^2$ is F; optionaly wherein $R^4$ is H and $R^5$ is H.

In embodiments, $R^4$ is a halogen or a pseudohalogen and $R^5$ is H; or $R^4$ is H and $R^5$ is a halogen or a pseudohalogen; optionaly wherein $R^1$ is H and $R^2$ is H. Preferably, $R^4$ is a halogen and $R^5$ is H; or $R^4$ is H and $R^5$ is a halogen; optionaly wherein $R^1$ is H and $R^2$ is H. More preferably, $R^4$ is F or Cl and $R^5$ is H; or $R^4$ is H and $R^5$ is F or Cl; optionaly wherein $R^1$ is H and $R^1$ is H. Most preferably, $R^4$ is F and $R^5$ is H; or $R^4$ is H and $R^5$ is F; optionaly wherein $R^1$ is H and $R^2$ is H.

***MONOCYCLIC HETEROARYL (HA)***

A compound according to the invention comprises a monocyclic heteroaryl represented by HA. The terms monocyclic heteroaryl and HA are used interchangeably herein.

In the context of this application, the monocyclic heteroaryl comprises a monocyclic heteroaromatic ring to which both the phenyl group and $R^3$ are directly and covalently bound. Heteroaromatic means that the ring must comprise at least one endocyclic atom which is not carbon. Monocyclic means that the heteroaromatic ring is part of a monocyclic aromatic system. The heteroaromatic ring may be further substituted, as described below. The term monocyclic does not rule out that this aromatic ring is further substituted by ring systems that do not form part of the same aromatic system. Likewise, heterocyclic does not imply the absence of substitution with carbon-only aromatic systems. As an example, HA may be a imidazolyl substituted with a phenyl group.

In embodiments, the heteroaromatic ring comprised in the monocyclic heteroaryl (HA) is formed by C, O, N and/or S atoms. Such monocyclic heteroaryls may be called monocyclic azaoxathia(hetero)aryls. In the context of this application, an aromatic ring formed by a given set of atoms refers to the endocyclic atoms (also called ring atoms) constituting the aromatic ring and evidently does not refer to any exocyclic substitutions or hydrogen atoms.

In embodiments, the heteroaromatic ring comprised in the monocyclic heteroaryl (HA) is formed by C, O and/or N atoms. Such monocyclic heteroaryls may be called monocyclic azaoxa(hetero)aryls.

In embodiments, the heteroaromatic ring comprised in the monocyclic heteroaryl (HA) is formed by C and O atoms. Such monocyclic heteroaryls may be called monocyclic oxa (hetero) aryls.

In embodiments, the heteroaromatic ring comprised in the monocyclic heteroaryl (HA) is formed by C and N atoms. Such monocyclic heteroaryls may be called monocyclic aza(hetero)aryls.

In embodiments, the heteroaromatic ring comprised in the monocyclic heteroaryl (HA) has 5 to 12 ring atoms. Such monocyclic heteroaryls (HA) may be called C5-C12 heteroaryls.

In embodiments, the heteroaromatic ring comprised in the monocyclic heteroaryl (HA) has 5 or 6 ring atoms. Such monocyclic heteroaryls (HA) may be called C5-C6 heteroaryls. Examples of C5-C6 heteroaryls include furanyl, thienyl, pyrrolyl, imidazolyl, oxazolyl, isoxazolyl, thiazolyl, isothiazolyl, pyrazolyl, triazolyl, oxadiazolyl, thiadiazolyl, pyridyl,

pyridazinyl, pyrimidinyl, and pyrazinyl.

In embodiments, the heteroaromatic ring comprised in the monocyclic heteroaryl (HA) has 5 ring atoms. Such monocyclic heteroaryls (HA) may be called C5 heteroaryls or five-membered heteroaryls. Examples of five-membered heteroaryls include furanyl, thienyl, pyrrolyl, imidazolyl, oxazolyl, isoxazolyl, thiazolyl, isothiazolyl, pyrazolyl, triazolyl, oxadiazolyl, and thiadiazolyl.

In embodiments, the heteroaromatic ring comprised in the monocyclic heteroaryl (HA) has 6 ring atoms. Such monocyclic heteroaryls (HA) may be called C6 heteroaryls or six-membered heteroaryls. Examples of six-membered heteroaryls include pyrazolyl, triazolyl, oxadiazolyl, and thiadiazolyl.

In embodiments, the heteroaromatic ring comprised in the monocyclic heteroaryl (HA) has 5 ring atoms and is formed by C, O, N and/or S atoms, preferably by C, O and/or N atoms.

In embodiments, the heteroaromatic ring comprised in the monocyclic heteroaryl (HA) has 6 ring atoms and is formed by C, O, N and/or S atoms, preferably by C, O and/or N atoms.

A compound according to the invention comprising a five-membered heteroaryl (HA) may be represented by formula (II) or by formula (II-ortho), depending on the relative position of the phenyl group and $R^3$ on HA:

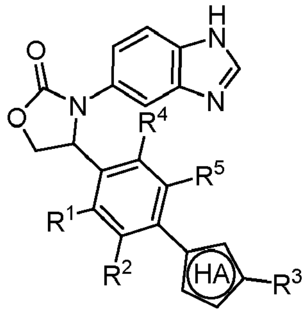

(II)

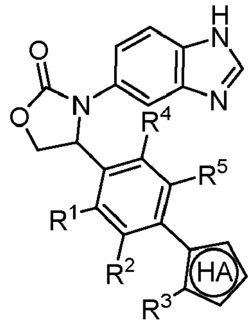

(II-ortho)

In embodiments, the compound according to the invention may be represented by formula (II), preferably wherein the heteroaromatic ring comprised in the monocyclic heteroaryl (HA) is formed by C, O, N and/or S atoms, preferably by C, O and/or N atoms.

In embodiments, the compound according to the invention may be represented by formula (II-ortho), preferably wherein the heteroaromatic ring comprised in the monocyclic heteroaryl (HA) is formed by C, O, N and/or S atoms, preferably by C, O and/or N atoms.

A compound according the invention represented by formula (II), wherein the heteroaromatic ring comprised in the monocyclic heteroaryl (HA) is formed by C, O, N and/or S atoms, may be represented by one of formulae (III), (IV), (V), (VI) and (VII), wherein each X is independently N or CH, and wherein each Z is independently NH, O or S:

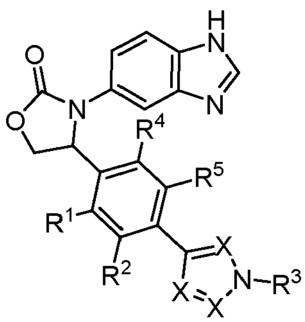

(III)

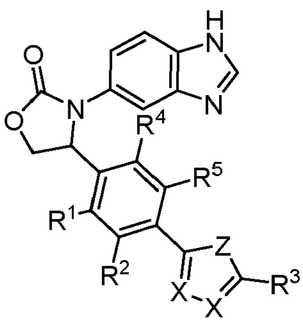

(IV)

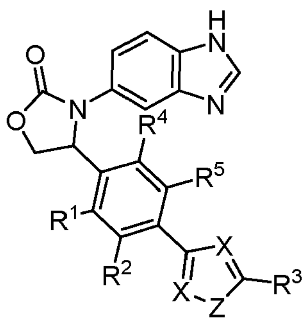

(V)

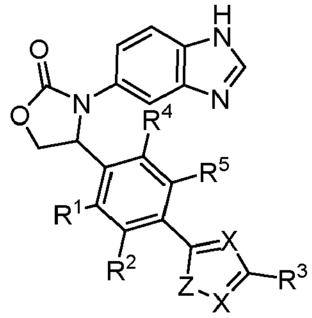

(VI)

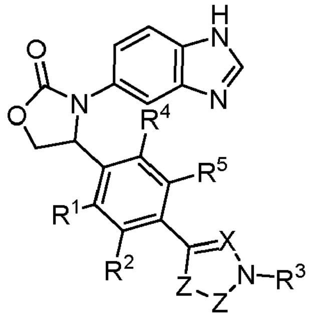

(VII)

In embodiments, the compound according the invention represented may be represented by formula (III), (IV), (V), (VI) or (VII), wherein each X is independently N or CH, and wherein each Z is independently NH, O or S.

In embodiments, the compound according the invention represented may be represented by formula (III), (IV), (V) or (VI), wherein each X is independently N or CH, and wherein each Z is independently NH, O or S.

In more specific embodiments, the compound according the invention represented may be represented by formula (III-a), (III-b), (III-c), (III-d), (III-e), (III-f), (III-g), or (III-h), wherein each X, denoted as X(A), X(B) and X(C) in the table below, is independently N or CH:

| | Formula | X(*A*) | X(*B*) | X(*C*) |
|---|---|---|---|---|
| 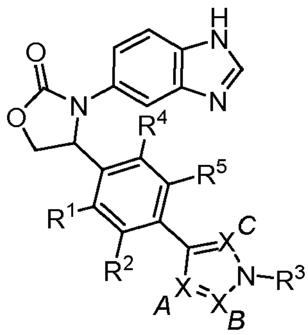 (III) | III | N or CH | N or CH | N or CH |
| | III-a | CH | CH | N |
| | III-b | CH | CH | CH |
| | III-c | CH | N | N |
| | III-d | CH | N | CH |
| | III-e | N | CH | N |
| | III-f | N | CH | CH |
| | III-g | N | N | N |
| | III-h | N | N | CH |

In more specific embodiments, the compound according the invention represented may be represented by formula (IV-a), (IV-b), (IV-c), (IV-d), (IV-e), (IV-f), (IV-g), (IV-h), (IV-i), (IV-j), (IV-k), or (IV-l), wherein each X, denoted as X(A) and X(B) in the table below, is independently N or CH, and wherein each Z is independently NH, O or S, denoted as Z(C) in the table below:

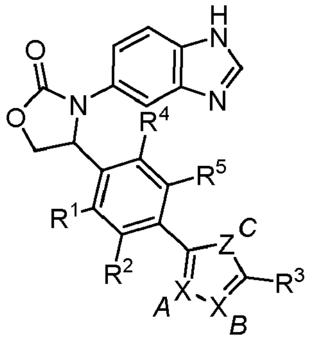

(IV)

| Formula | X(*A*) | X(*B*) | Z(*C*) |
|---|---|---|---|
| IV | N or CH | N or CH | NH, O or S |
| IV-a | CH | CH | NH |
| IV-b | CH | CH | O |
| IV-c | CH | CH | S |
| IV-d | CH | N | NH |
| IV-e | CH | N | O |
| IV-f | CH | N | S |
| IV-g | N | CH | NH |
| IV-h | N | CH | O |
| IV-i | N | CH | S |
| IV-j | N | N | NH |
| IV-k | N | N | O |
| IV-l | N | N | S |

In more specific embodiments, the compound according the invention represented may be represented by formula (V-a), (V-b), (V-c), (V-d), (V-e), (V-f), (V-g), (V-h), (V-i), (V-j), (V-k), or (V-l), wherein each X, denoted as X(A) and X(C) in the table below, is independently N or CH, and wherein each Z is independently NH, O or S, denoted as Z(8) in the table below:

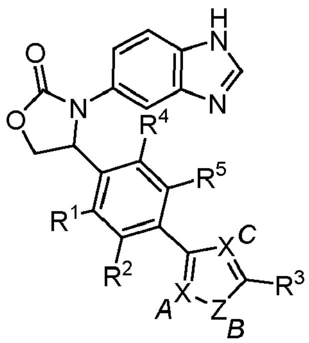

(V)

| Formula | X(*A*) | Z(*B*) | X(*C*) |
|---|---|---|---|
| V | N or CH | NH, O or S | N or CH |
| V-a | CH | NH | CH |
| V-b | CH | O | CH |
| V-c | CH | S | CH |
| V-d | CH | NH | N |
| V-e | CH | O | N |
| V-f | CH | S | N |
| V-g | N | NH | CH |
| V-h | N | O | CH |
| V-i | N | S | CH |
| V-j | N | NH | N |
| V-k | N | O | N |
| V-l | N | S | N |

In more specific embodiments, the compound according the invention represented may be represented by formula (VI-a), (VI-b), (VI-c), (VI-d), (VI-e), (VI-f), (VI-g), (VI-h), (VI-i), (VI-j), (VI-k), or (VI-l), wherein each X, denoted as X(B) and X(C) in the table below, is independently N or CH, and wherein each Z is independently NH, O or S, denoted as *Z(A)* in the table below:

| 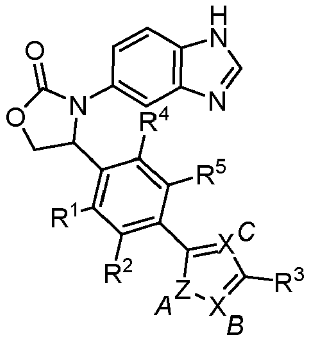 (VI) | Formula | Z(A) | X(B) | X(C) |
|---|---|---|---|---|
| | VI | NH, O or S | N or CH | N or CH |
| | VI-a | NH | CH | CH |
| | VI-b | O | CH | CH |
| | VI-c | S | CH | CH |
| | VI-d | NH | CH | N |
| | VI-e | O | CH | N |
| | VI-f | S | CH | N |
| | VI-g | NH | N | CH |
| | VI-h | O | N | CH |
| | VI-i | S | N | CH |
| | VI-j | NH | N | N |
| | VI-k | O | N | N |
| | VI-l | S | N | N |

In more specific embodiments, the compound according the invention represented may be represented by formula (VII-a), (VII-b), (VII-c), (VII-d), (VII-e), (VII-f), (VII-g), (VII-h), (VII-i), (VII-j), (VII-k), (VII-l), (VII-m), (VII-n), (VII-o), (VII-p), (VII-q), or (VII-r), wherein each X, denoted as X*(C)* in the table below, is independently N or CH, and wherein each Z is independently NH, O or S, denoted as Z*(A)* and Z*(B)* in the table below:

| 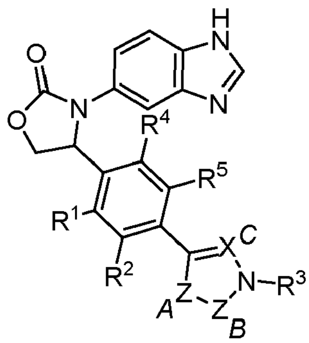 (VII) | Formula | Z*(A)* | Z*(B)* | X*(C)* |
|---|---|---|---|---|
| | VI | NH, O or S | NH, O or S | N or CH |
| | VI-a | NH | NH | N |
| | VI-b | NH | NH | CH |
| | VI-c | NH | O | N |
| | VI-d | NH | O | CH |
| | VI-e | NH | S | N |
| | VI-f | NH | S | CH |
| | VI-g | O | NH | N |
| | VI-h | O | NH | CH |
| | VI-i | O | O | N |
| | VI-j | O | O | CH |
| | VI-k | O | S | N |
| | VI-l | O | S | CH |
| | VI-m | S | NH | N |
| | VI-n | S | NH | CH |
| | VI-o | S | O | N |
| | VI-p | S | O | CH |
| | VI-q | S | S | N |
| | VI-r | S | S | CH |

In embodiments, the compound according the invention represented may be represented by formula (III-a),

(III-c), (III-d), (III-f), (IV-b), (IV-c), (V-k), (VI-f), or (VI-h).

The monocyclic heteroaryl (HA) is directly and covalently attached to both the phenyl group and $R^3$ as explained above. However, HA may be further substituted, i.e. covalently attached to, further groups insofar as the heteroaromatic group comprised therein can still be considered a monocyclic heteroaromatic group.

In embodiments, HA is substituted by one or more groups independently selected from alkyl, halogen, pseudohalogen, -O-alkyl, -NH-alkyl, $-N(alkyl)_2$, -C(=O)O-alkyl, -C(=O)NH-alkyl, $-C(=O)N(alkyl)_2$, -NHC(=O)-alkyl, -S(=O)zNH-alkyl, -S(=O)zN(alkyl)z, -NHS(=O)z-alkyl, and -S-alkyl, preferably wherein each alkyl group may be substituted with one or more halogens or pseudohalogens. A pseudohalogen is defined above.

In embodiments, HA is substituted by one or more groups independently selected from alkyl, halogen, pseudohalogen, -O-alkyl, -NH-alkyl, $-N(alkyl)_2$, -C(=O)O-alkyl, -C(=O)NH-alkyl, $-C(=O)N(alkyl)_2$, -NHC(=O)-alkyl, -S(=O)zNH-alkyl, -S(=O)zN(alkyl)z, -NHS(=O)z-alkyl, and -S-alkyl, wherein each alkyl group is a C1-C4 alkyl group, preferably wherein each alkyl group may be substituted with one or more halogens or pseudohalogens.

A halogen is F, Cl, Br, I, or At. Preferably, a halogen is F, Cl, Br, or I. More preferably, a halogen is F, Cl, or Br. Even more preferably, a halogen is F or Cl. Most preferably, a halogen is F. A pseudohalogen is -CN, -CP, -NC, -OH, -SH, -SeH,-TeH, -OCN, -SCN, - NCS, -SeCN, -TeCN, $-N_3$, -NO, or -NOz. Preferably, a pseudohalogen is -CN, -NC, -OH, - SH, -OCN, -SCN, -NCS, $-N_3$, -NO, or -NOz.

*MONOCYCLIC HETEROARYL SUBSTITUTION ($R^3$)*

The monocyclic heteroaryl (HA) comprised in a compound according to the invention is substituted by $R^3$, being a C1-C6 haloalkyl, preferably a C1-C4 haloalkyl.

In embodiments, $R^3$ is a C1-C5 haloalkyl, a C1-C4 haloalkyl, a C1-C3 haloalkyl, a C1-C2 haloalkyl, or a C1 haloalkyl.

In embodiments, $R^3$ is a C1-C4 haloalkyl, wherein the alkyl is methyl ($-CH_3$), ethyl ($-CH_2CH_3$), n-propyl ($-CH_2CH_2CH_3$), iso-propyl ($-CH(CH_3)_2$), cyclo-propyl, n-butyl ($-CH_2CH_2CH_2CH_3$), s-butyl ($-CH(CH_3)CH_2CH_3$), t-butyl ($-C(CH_3)_3$), iso-butyl ($-CH_2CH(CH_3)_2$), or cyclo-butyl.

In embodiments, $R^3$ is a C1-C3 haloalkyl, wherein the alkyl is methyl ($-CH_3$), ethyl ($-CH_2CH_3$), n-propyl ($-CH_2CH_2CH_3$), iso-propyl ($-CH(CH_3)_2$), or cyclo-propyl.

In embodiments, $R^3$ is a C1-C2 haloalkyl, wherein the alkyl is methyl ($-CH_3$), or ethyl ($-CH_2CH_3$).

In embodiments, $R^3$ comprises 1, 2, 3, 4, 5, 6, 7, 8, or 9 halogen atoms.

In embodiments, $R^3$ comprises between 1 to 4 halogens atoms, preferably 2 to 4.

In embodiments, $R^3$ is a C1-C6 perhaloalkyl, a C1-C5 perhaloalkyl, a C1-C4 perhaloalkyl, a C1-C3 perhaloalkyl, a C1-C2 perhaloalkyl, or a C1 perhaloalkyl.

In embodiments, $R^3$ is a fluoroalkyl, a chloroalkyl, a bromoalkyl, an iodoalkyl, or an astatoalkyl. Preferably, $R^3$ is a fluoroalkyl, a chloroalkyl, a bromoalkyl, or an iodoalkyl. More preferably, $R^3$ is a fluoroalkyl, a chloroalkyl, or a bromoalkyl. Even more preferably, $R^3$ is a fluoroalkyl, or a chloroalkyl. Most preferably, $R^3$ is a fluoroalkyl.

In embodiments, $R^3$ is a C1-C5 fluoroalkyl, a C1-C4 fluoroalkyl, a C1-C3 fluoroalkyl, a C1-C2 fluoroalkyl, or a C1 fluoroalkyl.

In embodiments, $R^3$ is a C1-C4 fluoroalkyl such as, but not being limited to, $-CF_3$, $-CHF_2$, $-CH_2CF_3$, $-CH_2CH_2F$, $-CH_2CHF_2$, $-CH(CH_3)CF_3$, $-CH_2C(CH_3)_2F$, $-CH_2CF_2CH_3$, $-CH_2CH_2CF_2CH_3$, $-CH_2CH_2CHF_2$, $-CH_2CH_2CF_3$,

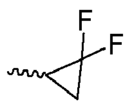, and 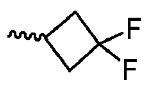.

In embodiments, $R^3$ is $-CF_3$, $-CH_2CHF_2$,

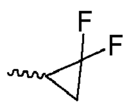, or 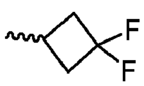.

In embodiments, $R^3$ is -CFs.

***ISOMERY***

Certain compounds may exist in one or more particular geometric, optical, enantiomeric, diastereoisomeric,

epimeric, stereoisomeric, tautomeric, (de)protonated, isotopomeric, isotopologues, isotopologic, conformational, or anomeric forms, including but not limited to, cis- and trans-forms; E- and Z-forms; c-, t-, and r- forms; endo- and exo-forms; R-, S-, and meso-forms; D- and L-forms; d- and l-forms; (+) and (-) forms; keto-, enol-, and enolate-forms; syn- and anti-forms; synclinal- and anticlinal-forms; α- and β-forms; axial and equatorial forms; boat-, chair-, twist-, envelope-, and halfchair-forms; and combinations thereof, hereinafter collectively referred to as "isomers" (or "isomeric forms").

Any reference to a compounds or a class of compounds, either by a name or by a formula, is meant as a reference to the set of all isomers falling in that class, unless explicitly mentioned otherwise. This includes both the isomers mentioned above and all structural isomers, unless it is clear that they are explicitly excluded. For example, a C1-C4 alkyl may refer to n-butyl and tert-butyl, together with all their isomers as mentioned above. A reference to n-butyl, on the other hand, only refers to n-butyl and its stereoisomers, isotopomers, isotopologues, etc., and not to tert-butyl.

If a compound or a class of compounds refers to multiple species, reference is made to both the isolated species and to any equimolar or non-equimolar mixture of the species.

It is reiterated that a formula also refers to all tautomers thereof. Specifically, formula (I) also refers to (I-6-yl):

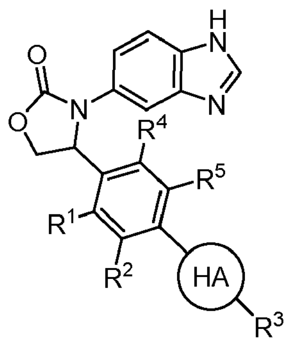

(I)

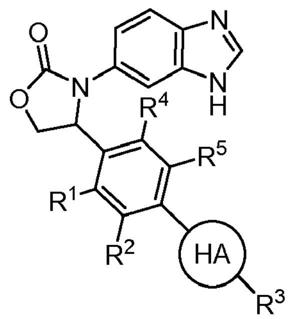

(I-6-yl)

A compound according to the invention represented by (I) has at least one stereocenter. Hence, the compound may be present in either one of two configurations, represented by either formula (I-S) or (I-R), respectively. As noted above, the general formula (I) may refer to either (I-S), (I-R), or any equimolar or non-equimolar mixtures of these. It is further understood that all other formulae in this application may be denoted in the fashion. For example, a compound represented by (III-a) and (I-S) may be referred to a (III-a-S).

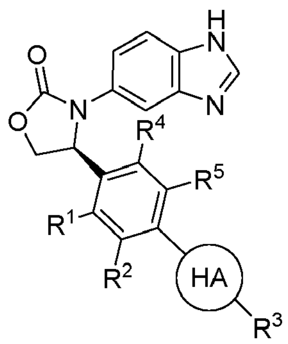

(I-S)

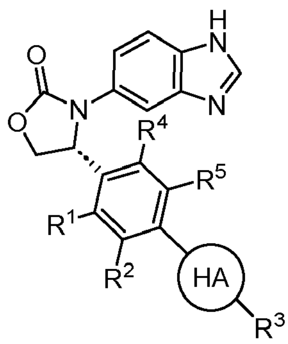

(I-R)

In embodiments, the compound according to the invention may be represented by formula (I-S).

In embodiments, the compound according to the invention may be represented by formula (I-R).

In embodiments, the compound according to the invention is a mixture, preferably a racemic mixture, of compounds represented by formulae (I-S) and (I-R), respectively. A racemic mixture is equimolar of essentially equimolar mixture of two enantiomers, in casu (I-S) and (I-R).

## *PREFERRED COMPOUNDS*

In embodiments, the compound according to the invention may be represented by one or more of formulae (VIII), (VIII-S), (IX), (IX-S), (X), (X-S), (XI), (XI-S), (XII), (XII-S), (XIII), (XIII-S), (XIV), or (XIV-S).

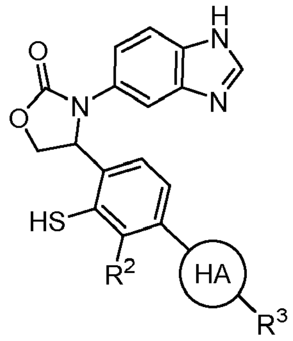

(VIII)

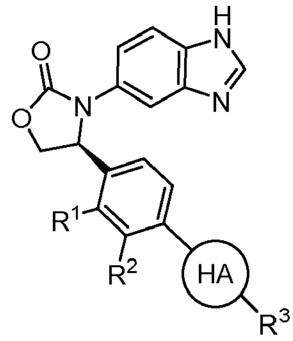

(VIII-S)

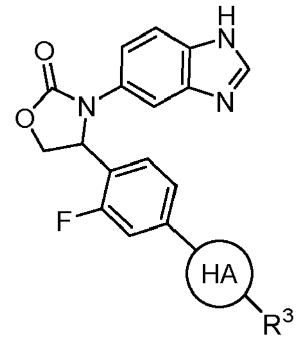

(IX)

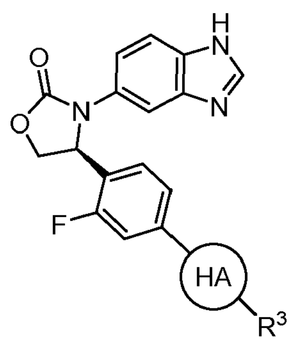

(IX-S)

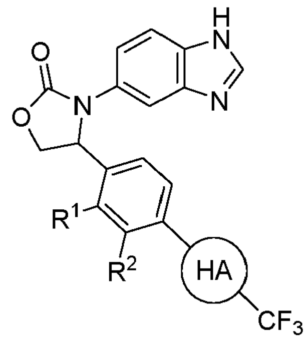

(X)

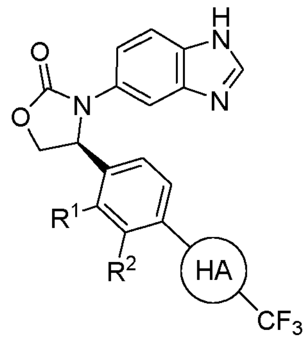

(X-S)

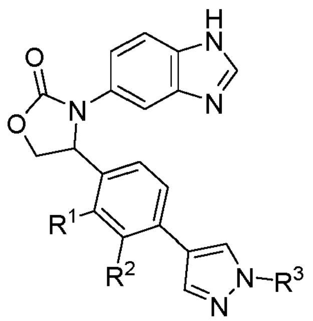

(XI)

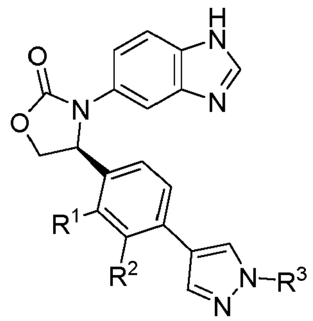

(XI-S)

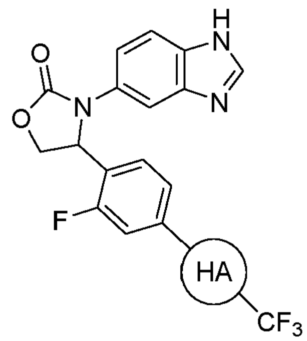

(XII)

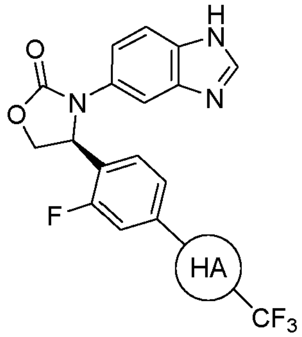

(XII-S)

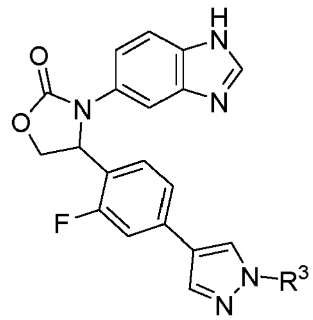

(XIII)

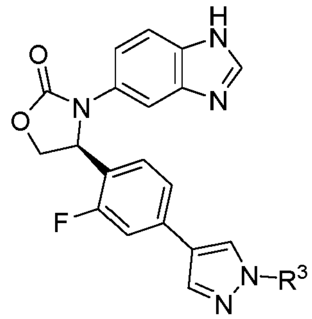

(XIII-S)

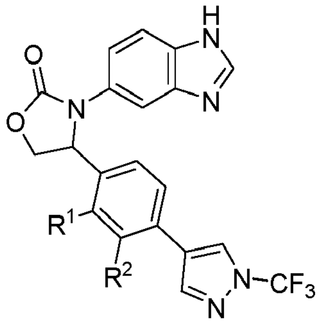

(XIV)

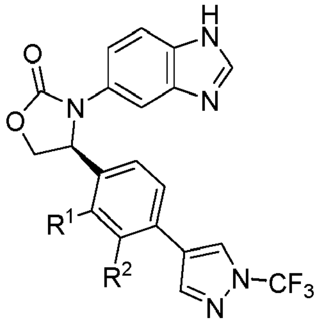

(XIV-S)

In embodiments, the compound according to the invention may be represented by formula (1), (1-S), (2), (2-S), (3), (3-S), (4), (4-S), (5), (5-S), (6), (6-S), (7), (7-S), (8), (8-S), (9), (9-S), (10), (10-S), (11), (11-S), (12), (12-S), (13), (13-S), (14), (14-S), (15), (15-S), (16), or (16-S).

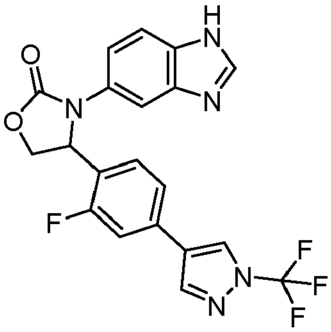

(1)

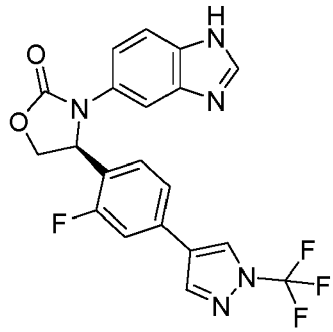

(1-S)

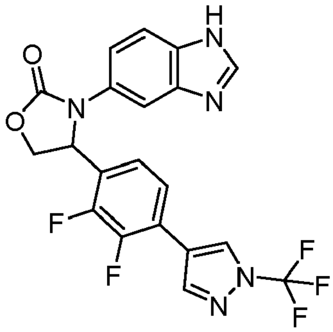

(2)

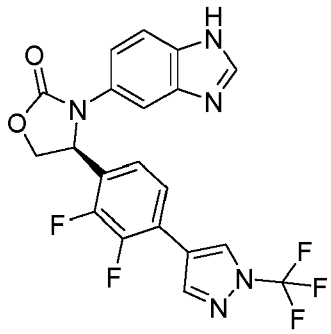

(2-S)

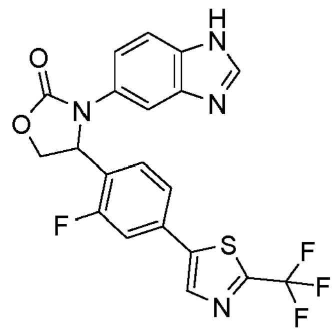

(3)

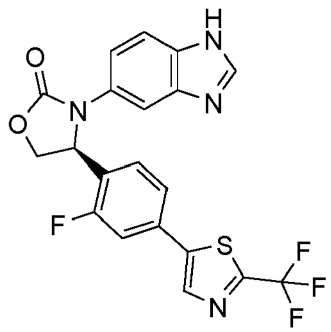

(3-S)

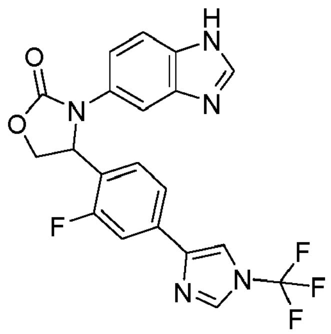

(4)

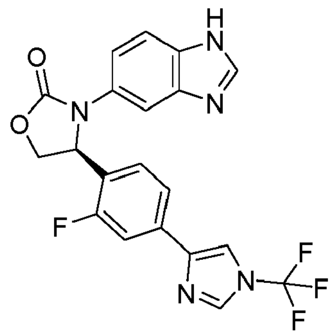

(4-S)

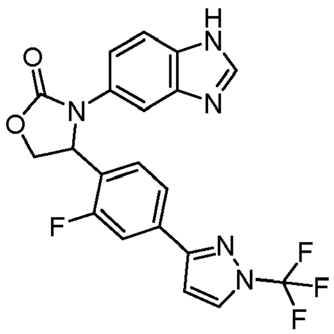

(5)

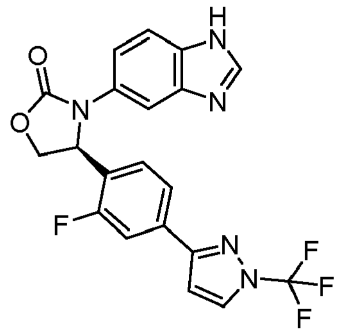

(5-S)

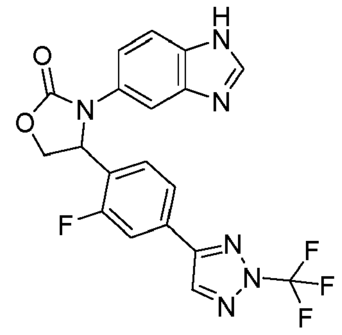

(6)

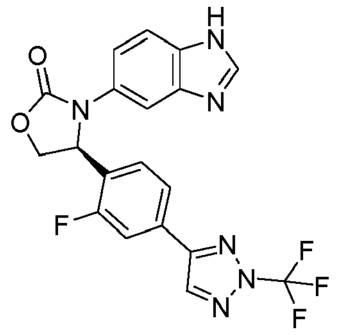

(6-S)

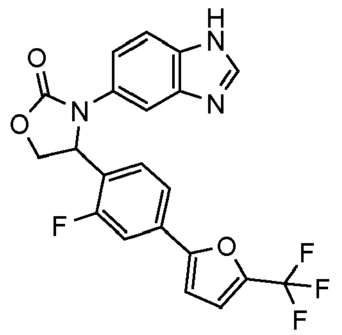

(7)

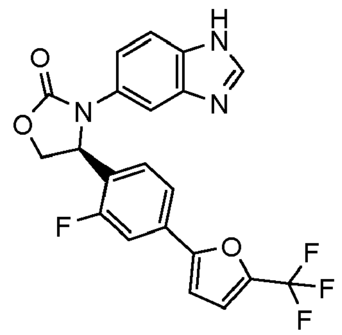

(7-S)

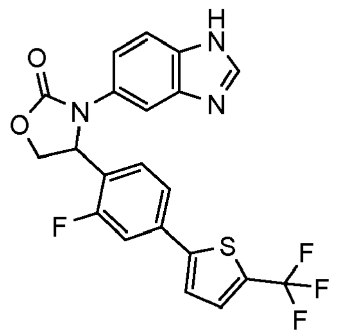

(8)

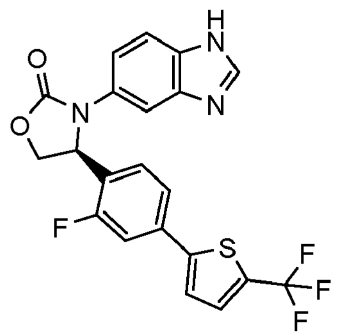

(8-S)

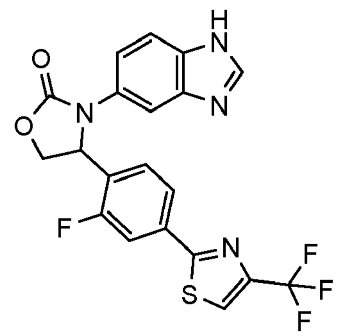

(9)

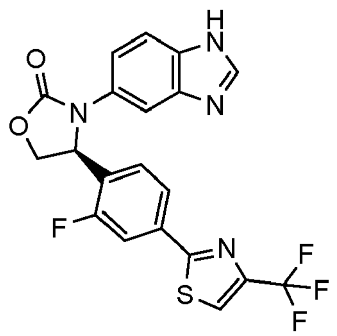

(9-S)

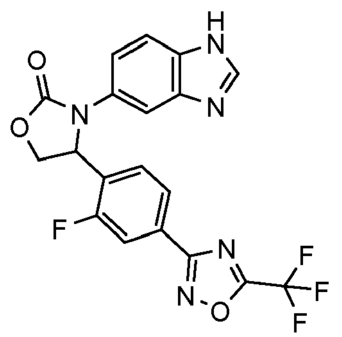

(10)

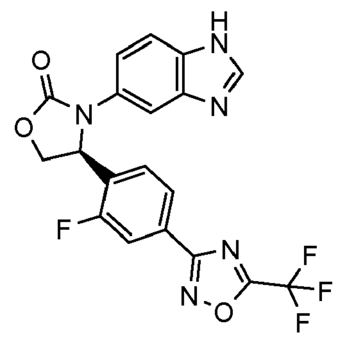

(10-S)

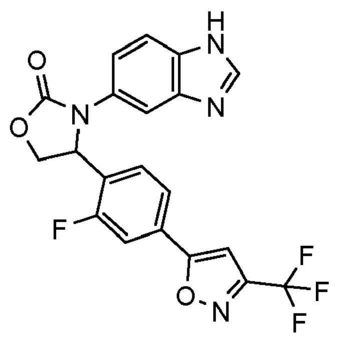

(11)

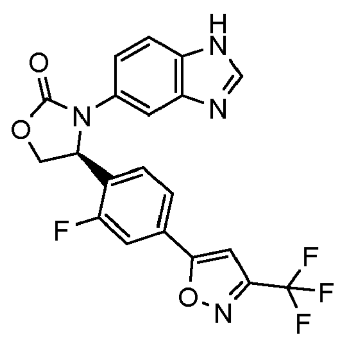

(11-S)

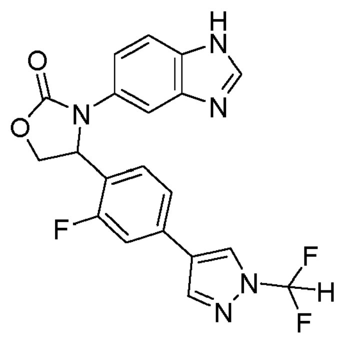

(12)

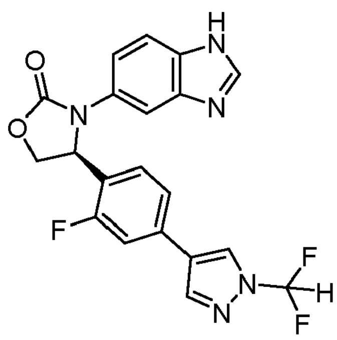

(12-S)

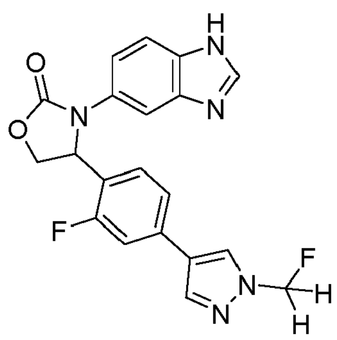

(13)

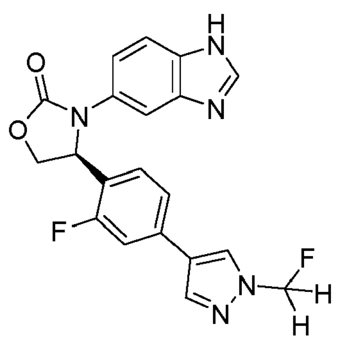

(13-S)

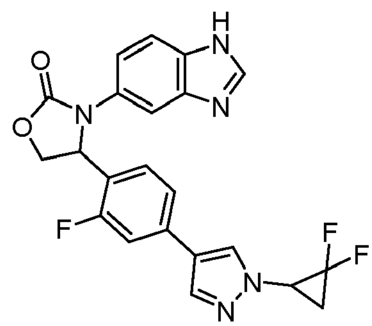

(14)

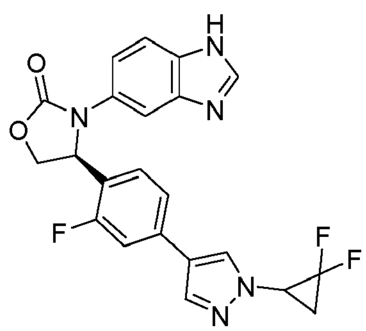

(14-S)

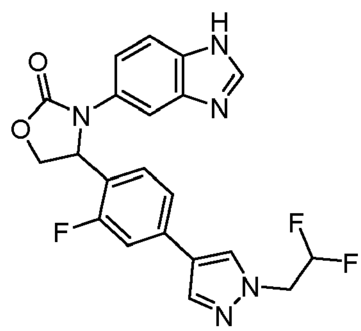

(15)

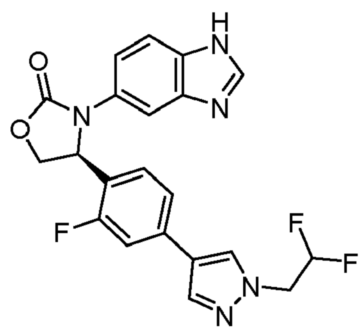

(15-S)

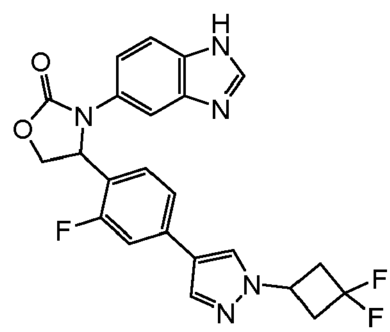

(16)

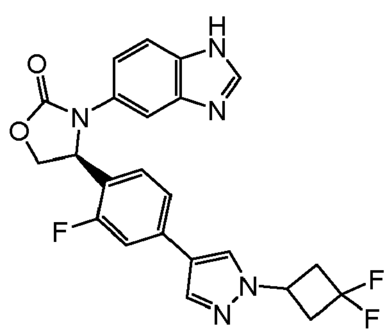

(16-S)

In embodiments, the compound according to the invention may be represented by formula (1) or (1-S).

### *SALTS*

A compound according to the invention may be present as a pharmaceutically acceptable salt.

Examples of pharmaceutically acceptable salts are discussed in Berge et al., 1977, "Pharmaceutically Acceptable Salts," J. Pharm. Sci., Vol. 66, pp. 1-19.

For example, if a compound according to the invention is anionic, or has a functional group, which may be anionic (e.g., -COOH may be $-COO^-$), then a salt may be formed with a suitable cation. Examples of suitable inorganic cations include, but are not limited to, alkali metal ions such as $Na^+$ and $K^+$, alkaline earth cations such as $Ca^{2+}$ and $Mg^{2+}$, and other cations such as $Al^{3+}$ as well as the ammonium ion (i.e., $NH_4^+$). Examples of suitable organic cations include, but are not limited to substituted ammonium ions (e.g., $NH_3R^+$, $NH_2R_2^+$, $NHR_3^+$, $NR_4^+$), for example, where each R is independently linear or branched saturated $C_{1-18}$alkyl, $C_{3-8}$cycloalkyl, $C_{3-8}$cycloalkyl-$C_{1-6}$alkyl, and phenyl-$C_{1-6}$alkyl, wherein the phenyl group is optionally substituted. Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is $N(CH_3)_4^+$.

If a compound according to the invention is cationic, or has a functional group, which upon protonation may become cationic (e.g., $-NH_2$ may become $-NH_3^+$), then a salt may be formed with a suitable anion.

For example, if a compound according to the invention contains a cationic group (e.g., $-NMe_2^+$), or has a functional group, which upon protonation may become cationic (e.g., $-NH_2$ may become $-NH_3^+$), then a salt may be formed with a suitable anion. In the case of a quaternary ammonium compound a counter-anion is generally always present in order to balance the positive charge. If, in addition to a cationic group (e.g., $-NMe_2^+$, $-NH_3^+$), the compound also contains a group capable of forming an anion (e.g., -COOH), then an inner salt (also referred to as a zwitterion) may be formed.

For example, in the benzimidazolyl group comprised in a compound according to the invention, the -NH- group in the imidazole ring may be protonated, and a salt may be formed with a suitable anion.

Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyloxybenzoic, acetic, trifluoroacetic, ascorbic, aspartic, benzoic, camphorsulfonic, cinnamic, citric, edetic, 1,2-ethanedisulfonic, ethanesulfonic, fumaric, glucoheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric. Examples of suitable polymeric organic anions include, but are not limited to, those derived from the following polymeric acids: tannic acid, carboxymethyl cellulose.

### *SOLVATES AND HYDRATES*

A compound according to the invention may be present as a pharmaceutically acceptable solvate or hydrate.

The term solvate is used herein in the conventional sense to refer to a complex of solute (e.g., compound, salt of compound) and solvent. If the solvent is water, the solvate may be conveniently referred to as a hydrate, for example, a mono-hydrate, a di-hydrate, a trihydrate, etc.

### *Compositions*

An aspect of the invention pertains to a composition (e.g., a pharmaceutical composition) comprising a compound according to the invention, and a pharmaceutically acceptable carrier, diluent, or excipient. Such a composition may be called a composition according to the invention herein.

Another aspect of the invention pertains to a method of preparing a composition according to the invention.

### *Kits*

An aspect of the invention pertains to a kit comprising (a) a compound according to the invention, preferably provided as a composition (e.g., a pharmaceutical composition) and in a suitable container and/or with suitable packaging; and (b) instructions for use, for example, in a method of treatment of a disorder (e.g., a disease) as described herein, for example, written instructions on how to administer the compound.

The written instructions may also include a list of indications for which the compound according to the invention is a suitable treatment.

***Purified forms***

An aspect of the invention pertains to compounds according to the invention in substantially purified form and/or in a form substantially free from contaminants.

In one embodiment, the substantially purified form is at least 50% by weight, e.g., at least 60% by weight, e.g., at least 70% by weight, e.g., at least 80% by weight, e.g., at least 90% by weight, e.g., at least 95% by weight, e.g., at least 97% by weight, e.g., at least 98% by weight, e.g., at least 99% by weight.

Unless otherwise specified, the substantially purified form refers to the compound in any stereoisomeric or enantiomeric form. For example, in one embodiment, the substantially purified form refers to a mixture of stereoisomers, i.e., purified with respect to other compounds. In one embodiment, the substantially purified form refers to one stereoisomer, e.g., optically pure stereoisomer. In one embodiment, the substantially purified form refers to a mixture of enantiomers. In one embodiment, the substantially purified form refers to a equimolar mixture of enantiomers (i.e., a racemic mixture, a racemate). In one embodiment, the substantially purified form refers to one enantiomer, e.g., optically pure enantiomer.

In one embodiment, the contaminants represent no more than 50% by weight, e.g., no more than 40% by weight, e.g., no more than 30% by weight, e.g., no more than 20% by weight, e.g., no more than 10% by weight, e.g., no more than 5% by weight, e.g., no more than 3% by weight, e.g., no more than 2% by weight, e.g., no more than 1% by weight.

Unless specified, the contaminants refer to other compounds, that is, other than stereoisomers or enantiomers. In one embodiment, the contaminants refer to other compounds and other stereoisomers. In one embodiment, the contaminants refer to other compounds and the other enantiomer.

In one embodiment, the substantially purified form is at least 60% optically pure (i.e., 60% of the compound, on a molar basis, is the desired stereoisomer or enantiomer, and 40% is the undesired stereoisomer or enantiomer), e.g., at least 70% optically pure, e.g., at least 80% optically pure, e.g., at least 90% optically pure, e.g., at least 95% optically pure, e.g., at least 97% optically pure, e.g., at least 98% optically pure, e.g., at least 99% optically pure.

***Chemically protected forms***

It may be convenient or desirable to prepare, purify, and/or handle the compound according to the invention in a chemically protected form. The term "chemically protected form" is used herein in the conventional chemical sense and pertains to a compound in which one or more reactive functional groups are protected from undesirable chemical reactions under specified conditions (e.g., pH, temperature, radiation, solvent, reactive chemical reagents, and the like). In practice, well-known chemical methods are employed to reversibly render unreactive a functional group, which otherwise would be reactive, under specified conditions. In a chemically protected form, one or more reactive functional groups are in the form of a protected or protecting group (alternatively as a masked or masking group or a blocked or blocking group). By protecting a reactive functional group, reactions involving other unprotected reactive functional groups can be performed, without affecting the protected group; the protecting group may be removed or the masking group transformed, usually in a subsequent step, without substantially affecting the remainder of the molecule. See, for example, Protective Groups in Organic Synthesis (T. Green and P. Wuts; 4th Edition; John Wiley and Sons, 2006).

A wide variety of such "protecting," "blocking," or "masking" methods are widely used and well known in organic synthesis. For example, a compound which has two nonequivalent reactive functional groups, both of which would be reactive under specified conditions, may be derivatized to render one of the functional groups "protected," and therefore unreactive, under the specified conditions; so protected, the compound may be used as a reactant which has effectively only one reactive functional group. After the desired reaction (involving the other functional group) is complete, the protected group may be "deprotected" to return it to its original functionality.

For example, a hydroxy group may be protected as an ether (-OR) or an ester (-OC(=O)R), for example, as: a t-butyl ether; a benzyl, benzhydryl (diphenylmethyl), or trityl (triphenylmethyl) ether; a trimethylsilyl or t-butyldimethylsilyl ether; or an acetyl ester (-OC(=O)$CH_3$, -OAc).

For example, an amine group may be protected, for example, as an amide (-NRCO-R), for example: as an acetamide (-NHCO-CHs); or as a carbamate (-NRCO-OR), for example: as a benzyloxy carbamate (-NHCO-$OCH_2C_6H_5$, -NH-Cbz), as a t-butoxy carbamate (-NHCO-$OC(CH_3)_3$, -NH-Boc); as a 2-biphenyl-2-propoxy carbamate (-NHCO-$OC(CH_3)_2C_6H_4C_6H_5$, -NH-Bpoc), as a 9-fluorenylmethoxy carbamate (-NH-Fmoc), as a 6-nitroveratryloxy carbamate (-NH-Nvoc), as a 2-trimethylsilylethyloxy carbamate (-NH-Teoc), a 2,2,2-trichloroethyloxy carbamate (-NH-Troc), as an allyloxy amide (-NH-Alloc), or as a 2(-phenylsulfonyl)ethyloxy carbamate (-NH-Psec); or, in suitable cases (e.g., cyclic amines), as a nitroxide radical (>N-O•); or, in suitable cases (e.g., heterocyclic nitrogens), as a 2-trimethylsilylethoxymethyl (N-SEM).

### *Prodrugs*

It may be convenient or desirable to prepare, purify, and/or handle the compound according to the invention in the form of a prodrug. The term "prodrug," as used herein, pertains to a compound, which yields the desired active compound *in vivo.* Typically, the prodrug is inactive, or less active than the desired active compound, but may provide advantageous handling, administration, or metabolic properties.

For example, some prodrugs are esters of the active compound (e.g., a physiologically acceptable metabolically labile ester). During metabolism, the ester group (-C(=O)OR) is cleaved to yield the active drug. Such esters may be formed by esterification, for example, of any of the carboxylic acid groups (-C(=O)OH) in the parent compound, with, where appropriate, prior protection of any other reactive groups present in the parent compound, followed by deprotection if required.

Also, some prodrugs are activated enzymatically to yield the active compound, or a compound, which, upon further chemical reaction, yields the active compound (for example, as in antibody directed enzyme prodrug therapy (ADEPT), gene directed enzyme prodrug therapy (GDEPT), lipid directed enzyme prodrug therapy (LIDEPT), *etc.).* For example, the prodrug may be a sugar derivative or other glycoside conjugate, or may be an amino acid ester derivative.

### *Therapeutic use*

An aspect of the invention pertains to a compound according to the invention for use as a medicament, i.e. for treating (i.e. for use in a method of treatment) of the human or animal body by therapy, for example, for use in a method of treatment of a disorder (e.g., a disease) as described herein.

An aspect of the invention pertains to a method of treatment, for example, a method of treatment of a disorder (*e.g*., a disease) as described herein, comprising administering to a subject in need of treatment a compound according to the invention, preferably in the form of a pharmaceutical composition.

An aspect of the invention pertains to use of a compound according to the invention in the manufacture of a medicament, for example, for use in a method of treatment, for example, for use in a method of treatment of a disorder (e.g., a disease) as described herein.

The disorders and other therapeutic parameters below may be applied to all therapeutic aspects above, i.e. to the compound for use in treatment, to the method of treatment, and to the use of the compound for the manufacture of a medicament.

### *ISOQC, QC AND THE CD47-SIRPA SIGNALLING AXIS*

In embodiments, the compounds according to the invention inhibit glutaminyl-peptide cyclotransferase-like (isoQC) enzyme (e.g., inhibit or reduce or block the activity or function of isoQC enzyme).

In embodiments, the compounds according to the invention inhibit glutaminyl-peptide cyclotransferase (QC) enzyme (e.g., inhibit or reduce or block the activity or function of QC enzyme).

In embodiments, the compounds according to the invention inhibit *both* glutaminyl-peptide cyclotransferase-like (isoQC) enzyme *and* glutaminyl-peptide cyclotransferase (QC) enzyme (e.g., inhibit or reduce or block the activity or function of isoQC and/or QC enzyme).

Compounds according to the invention are characterized by a high potency to inhibit isoQC and/or QC. Example 3 show that compounds according to the invention are highly potent. The potency can be measured as an IC50 value according to Assay 1 (for isoQC), Assay 2 (for QC) and Assay 3 (pyroglutamylation in live cells). Compared to reference compounds (17)-(19) of WO2021009068, the compounds of the invention are more potent, as evidenced by lower $IC_{50}$ values.

Accordingly, the compounds according to the invention may be useful, for example, in the treatment of disorders (e.g., diseases) that are ameliorated by the inhibition of isoQC and/or QC enzyme (e.g., by the inhibition or reduction or blockage of the activity or function of isoQC and/or QC enzyme).

The disorder (e.g., a disease) is preferably ameliorated by the inhibition of isoQC and/or QC enzyme (e.g., by the inhibition or reduction or blockage of the activity or function of isoQC and/or QC enzyme). Compounds according to the invention inhibit the glutaminyl-peptide cyclotransferase-like (isoQC) enzyme and/or the glutaminyl-peptide cyclotransferase (QC) enzyme (e.g., inhibit or reduce or block the activity or function of isoQC and/or QC enzyme).

In embodiments, the disorder is, for example, cancer, atherosclerosis, fibrotic diseases, infectious diseases, Alzheimer's disease *etc.,* as described herein.

In embodiments, the disorders which may be treated by the compound according to the invention include those the aetiology of which involve or requires the CD47-SIRP$\alpha$ signalling axis. Such diseases include those in which diseased cells, or other undesirable cells, evade immune surveillance by expression or over-expression of CD47. Such diseases may thus be treated by reducing pyroglutamylation of CD47 in such cells, resulting in reduced binding between CD47

on the surface of such cells and SIRPα on the surface of a second cell, such as an immune cell. The interplay between the CD47-SIRPα signalling axis, isoQC and QC is described below.

The term "Cluster of Differentiation 47" (abbreviated as "CD47") as used herein refers to a 50 kDa transmembrane protein (receptor) encoded by the CD47 gene (Ensembl reference: ENSG00000196776 in human). CD47 is also known as integrin associated protein (IAP). CD47 is expressed by many cells in the body as revealed by CD47 mRNA expression and CD47 immunohistochemical staining studies (see, e.g., Wiersma *et al.,* 2015; Lindberg *et al.,* 1993). CD47 has been implicated in a range of cellular processes, including apoptosis, proliferation, adhesion, and migration as well as angiogenic and immune responses. CD47 binds to or interacts with several ligands with signal-regulatory protein alpha (SIRPα) being considered a main ligand for CD47.

The term "signal-regulatory protein alpha" (abbreviated "SIRPα" or "SIRPa", also termed CD172a or SHPS-1) as used herein refers to a regulatory transmembrane glycoprotein from the SIRP family, which is encoded by the SIRPα gene (Ensembl reference: ENSG00000198053 in human). SIRPα is an inhibitory transmembrane receptor of various cell types, including myeloid cells (e.g., macrophages, monocytes, neutrophils, basophils, eosinophils, dendritic cells), neurons, and (*in vitro*) cardiomyocytes derived from induced pluripotent stem cells (see, e.g., Matozaki *et al.,* 2009; Dubois *et al.,* 2011).

The interaction between CD47 and SIRPα mediates or conveys "anti-phagocytic" or "don't eat me" signals between two cells, which ultimately inhibit phagocytosis and other cytotoxic effects. When CD47 interacts or binds with SIRPα, it initiates a cascade of signalling events in the cells (i.e., the cell expressing CD47 and the cell expressing SIRPα). Specifically, CD47-SIRPα interaction causes tyrosine phosphorylation of SIRPα cytoplasmic immunoreceptor tyrosine-based inhibitory motifs (ITIM) motifs, which in turn leads to concomitant recruitment of Src homology 2 domain tyrosine phosphatase 1 (SHP-1) and Src homology 2 domain tyrosine phosphatase 2 (SHP-2). SHP-1 and SHP-2 are cytoplasmic protein tyrosine phosphatases, which mediate signalling events causing inhibition of phagocytosis by for instance dephosphorylating myosin-IIA (see, e.g., Wiersma *et al.,* 2015).

Because it triggers a cascade of signalling events leading to inhibition of phagocytosis, the binding or interacting of CD47 with SIRPα is often referred to as a "don't eat me signal" or "anti-phagocytic signal". Importantly, the binding of CD47 to SIRPα can also inhibit death of CD47 expressing cells by other mechanisms, such as antibody dependent cellular cytotoxicity (ADCC). Together, blocking of the CD47-SIRPα interaction between target cells and immune cells may be exploited to enhance death of the CD47 expressing cells.

It has been reported that cancer cells upregulate the expression of CD47 at their cell surface, which results in CD47 levels which are higher compared to CD47 levels found in normal cells (see, e.g., Majeti *et al.,* 2009; Chao *et al.,* 2012). As a result, cancer cells can evade destruction by the immune system or evade immune surveillance, e.g., by evading phagocytosis by immune cells such as phagocyte cells (e.g., macrophages, neutrophils). It was also found that diseased cells in conditions other than cancer, such as, e.g., atherosclerosis, fibrotic diseases, infectious diseases caused by pathogens (e.g., viruses), *etc.,* upregulate the expression of CD47 at their cell surface compared to the CD47 levels found in normal/healthy cells to evade phagocytosis by phagocytes. Hence, inhibition of the CD47-SIRPα interaction may also be therapeutically exploited in these and yet other, to be discovered, disease areas. However, also in conditions where CD47 is not specifically upregulated, blocking of the CD47-SIRPα interaction may be therapeutically beneficial by shifting the balance towards phagocytosis of target cells.

In the case of cancer, several approaches to interfere with the CD47-SIRPα interaction have principally targeted CD47. For instance, several anti-CD47 antibodies and recombinant SIRPα proteins, either or not fused to immunomodulatory peptide sequences, aimed at interfering or blocking CD47-SIRPα interactions are currently being developed or tested in clinical trials. Although promising, such strategies have important drawbacks. For instance, antibodies and large polypeptides are known to have poor tissue penetration, especially into solid tumors, as compared to small molecule inhibitors. Furthermore, since CD47 is widely distributed throughout the body, including healthy tissues, the available pool of molecules able to bind to the intended cells is limited. This is referred to as the antigen sink effect. Other disadvantages associated with the use of anti-CD47 antibodies include the lack of oral bioavailability and undesirable side effects such as the development of anemia (which may occur as a result of a dose-dependent loss of red blood cells) as well as hemagglutination (clumping of red blood cells) and thrombocytopenia (lack of blood platelets). Alternative therapeutic strategies that do not rely on large biomolecules (e.g., antibodies or recombinant proteins) could yield improved efficacy, less toxic side-effects, and increased ease of use.

Recently, it has been demonstrated that binding of SIRPα to CD47 depends on pyroglutamylation of the N-terminal glutamine moiety of CD47. Pyroglutamylation is a post-translational modification in which either a glutamine or glutamate amino acid is converted into a pyroglutamate moiety. The human genome contains two genes that encode two enzymes that catalyse this N-terminal pyroglutamylation reaction, i.e., the glutaminyl-peptide cyclotransferase-like (QPCTL) gene encoding the glutaminyl-peptide cyclotransferase-like (isoQC) protein / enzyme and the glutaminyl-peptide cyclotransferase (QPCT) gene encoding the glutaminyl-peptide cyclotransferase (QC) protein / enzyme. While isoQC is localized in the Golgi apparatus and QC is secreted, there is an overlap in substrate preference and enzymatic characteristics.

In case of the interaction between SIRPα and CD47, this interaction was shown to primarily depend on the glutaminyl cyclase activity of the isoQC protein, encoded by the glutaminyl-peptide cyclotransferase-like (QPCTL) gene. As such, it was shown that chemical inhibition of isoQC or genetic depletion of QPCTL reduced SIRPα binding to CD47. Furthermore, increased phagocytosis of antibody opsonized cells *in vitro* and increased clearance of opsonized tumour cells *in vivo* were shown to be effects of blocking isoQC activity or production (see, e.g., Logtenberg *et al.,* 2019). Due to the overlap in enzymatic characteristics and substrate preference, it is possible that, in certain circumstances, glutaminyl cyclase activity of the QC protein can also play a role in the interaction between SIRPα and CD47 and therefore in the CD47-SIRPα signalling axis.

Accordingly, reducing or blocking or inhibiting the activity of the enzyme referred to as glutaminyl-peptide cyclotransferase-like (isoQC) and/or the enzyme referred to as glutaminyl-peptide cyclotransferase (QC) is associated with a reduction or inhibition or blockade of the interaction or binding between CD47 and SIRPα. This reduction of interaction or binding between CD47 and SIRPα results in a reduction or inhibition or blockade of the CD47-SIRPα signalling axis.

Generation of a proficient "anti-phagocytic signal" or "do not eat me signal" by a cell (for instance a cell in a disease or condition involving the CD47-SIRPα signalling axis, such as a cancer cell) could therefore be blocked by interfering with the enzyme(s) responsible for the pyroglutamylation of CD47, including isoQC and possibly under certain conditions QC.

In addition to CD47, QC and isoQC pyroglutamylate other proteins. For example, the amyloid beta protein involved in Alzheimer's disease is known to be pyroglutamylated by QC and the C-C Motif Chemokine Ligand 2 (CCL2) protein is known to be pyroglutamylated by isoQC. Due to the large overlap in enzymatic characteristics and substrate preference it can, however, not be ruled out that there is a certain amount of functional overlap between QC and isoQC in pyroglutamylating these targets.

***CANCER***

In embodiments, the disorder is cancer.

Compounds according to the invention are effective for the treatment of cancer. This is evidenced for compounds (1) in the Raji xenograft mouse model of Example 8 and the Pan02 syngeneic mouse model of Example 9. Their therapeutic usefulness is corroborated by their high metabolic stability, as evidenced by the microsomal stability in Example 5, and high kinetic solubility, as evidenced in Example 6.

In embodiments, the disorder is: leukemia, acute myeloid leukemia (AML), acute promyelocytic leukemia (APL), chronic myeloid leukemia (CML), acute lymphoblastic leukemia (ALL), T-cell acute lymphoblastic leukemia (T-ALL), lymphoma, B-cell lymphoma, T-cell lymphoma, Hodgkin's disease, non-Hodgkin's lymphoma (NHL), hairy cell lymphoma, Burkett's lymphoma, multiple myeloma (MM), myelodysplastic syndrome, lung cancer, adenocarcinoma, small cell lung cancer (SCLC), non-small cell lung cancer (NSCLC), mediastinum cancer, peritoneal cancer, mesothelioma, gastrointestinal cancer, gastric cancer, stomach cancer, bowel cancer, small bowel cancer, large bowel cancer, colon cancer, colon adenocarcinoma, colon adenoma, rectal cancer, colorectal cancer, leiomyosarcoma, breast cancer, gynaecological cancer, genito-urinary cancer, ovarian cancer, endometrial cancer, cervical cancer, prostate cancer, testicular cancer, seminoma, teratocarcinoma, liver cancer, kidney cancer, bladder cancer, urothelial cancer, biliary tract cancer, pancreatic cancer, exocrine pancreatic carcinoma, esophageal cancer, nasopharyngeal cancer, head and neck squamous cell carcinoma (HNSCC), skin cancer, squamous cancer, squamous cell carcinoma, Kaposi's sarcoma, melanoma, malignant melanoma, xeroderma pigmentosum, keratoacanthoma, bone cancer, bone sarcoma, osteosarcoma, rhabdomyosarcoma, fibrosarcoma, thyroid gland cancer, thyroid follicular cancer, adrenal gland cancer, nervous system cancer, brain cancer, astrocytoma, neuroblastoma, glioma, schwannoma, glioblastoma, or sarcoma.

In embodiments, the disorder is: leukemia, acute myeloid leukemia (AML), acute promyelocytic leukemia (APL), chronic myeloid leukemia (CML), acute lymphoblastic leukemia (ALL), T cell acute lymphoblastic leukemia (T ALL), lymphoma, B-cell lymphoma, T-cell lymphoma, Hodgkin's disease, non-Hodgkin's lymphoma (NHL), hairy cell lymphoma, Burkett's lymphoma, multiple myeloma (MM), myelodysplastic syndrome, lung cancer, adenocarcinoma, small cell lung cancer (SCLC), non-small cell lung cancer (NSCLC), gastrointestinal cancer, gastric cancer, stomach cancer, bowel cancer, small bowel cancer, large bowel cancer, colon cancer, colon adenocarcinoma, colon adenoma, rectal cancer, colorectal cancer, breast cancer, gynaecological cancer, ovarian cancer, prostate cancer, bladder cancer, pancreatic cancer, exocrine pancreatic carcinoma, esophageal cancer, head and neck squamous cell carcinoma (HNSCC), skin cancer, squamous cancer, squamous cell carcinoma, Kaposi's sarcoma, melanoma, malignant melanoma, xenoderoma pigmentoum, osteosarcoma, nervous system cancer, brain cancer, astrocytoma, neuroblastoma, glioma, schwannoma, glioblastoma, or sarcoma.

In embodiments, the disorder is: gastrointestinal cancer, gastric cancer, stomach cancer, esophageal cancer, head and neck squamous cell carcinoma (HNSCC), non-small cell lung cancer (NSCLC), breast cancer, colorectal cancer, bowel cancer, large bowel cancer, colon cancer, colon adenocarcinoma, colon adenoma, rectal cancer, ovarian

cancer, pancreatic cancer, exocrine pancreatic carcinoma, leukemia, acute myeloid leukemia (AML), myelodysplastic syndrome, lymphoma, B-cell lymphoma, non-Hodgkin's lymphoma (NHL), urothelial cancer, or peritoneal cancer.

### *PROLIFERATIVE DISORDERS*

In embodiments, the disorder is: a proliferative disorder.

The term "proliferative disorder," as used herein, pertains to an unwanted or uncontrolled cellular proliferation of excessive or abnormal cells which is undesired, such as neoplastic or hyperplastic growth.

In embodiments, the proliferative disorder is characterised by benign, pre-malignant, malignant, pre-metastatic, metastatic, or non-metastatic cellular proliferation, including for example: neoplasms, hyperplasias, and tumours (e.g., histocytoma, glioma, astrocyoma, osteoma), cancers (see below), psoriasis, bone diseases, fibroproliferative disorders (e.g., of connective tissues), pulmonary fibrosis, atherosclerosis, smooth muscle cell proliferation in the blood vessels, such as stenosis or restenosis following angioplasty.

### *ATHEROSCLEROSIS*

In embodiments, the disorder is: atherosclerosis.

The term "atherosclerosis" as used herein refers to condition recognized as the main disease process underlying heart attack and stroke. More specifically, atherosclerosis is characterized as a systemic, progressive disease process in which the arterial wall thickens through a pathological process involving inflammation, oxidative stress, and dyslipidemia. This pathological process leads to plaque formation and flow limitation in the vessel lumen of subjects afflicted with the condition.

The mechanisms underlying atherosclerosis are being actively studied. For example, it has been reported that the accumulation of diseased vascular cells (e.g., diseased vascular smooth muscle cells), diseased endothelial cells, and apoptotic cellular debris in the vessel lumen debris contributes to worsen the pathological process leading to plaque formation. A recent study has revealed that diseased cells (such as diseased vascular smooth muscle cells and diseased endothelial cells) upregulate the expression of CD47 at their cell surface thereby conveying a "don't eat me signal", which allows said diseased cells to evade phagocytosis by phagocyte cells, e.g., macrophages, so that diseased cells are not cleared by the immune system (see, e.g., Kojima *et al.,* 2016). This is consistent with the observation that CD47 is consistently upregulated in human atherosclerotic plaque compared to non-atherosclerotic vascular tissue, and in subjects with symptomatic cerebrovascular disease (stroke or transient ischemic attack) as compared to those with stable asymptomatic lesions (see, e.g., Kojima *et al.,* 2016). It was further reported that inhibiting the CD47-SIRP$\alpha$ axis by administration of an anti-CD47 antibody improved clearance of diseased cells by phagocyte cells and ameliorated atherosclerosis (see, e.g., Kojima *et al.,* 2016). Accordingly, isoQC and/or QC inhibitors, such as those described herein, may be useful therapeutic agents for the treatment of atherosclerosis.

### *FIBROTIC DISEASES*

In embodiments, the disorder is: a fibrotic disease.

In embodiments, the disorder is: scleroderma, idiopathic pulmonary fibrosis, liver cirrhosis, kidney fibrosis, lung fibrosis, bladder fibrosis, heart fibrosis, pancreas fibrosis, or myelofibrosis.

The term "fibrotic disease" as used herein refers to a condition that is characterized by the accumulation of excess extracellular matrix components (e.g., collagen, fibronectin) that forms fibrous connective tissue in and around an inflamed or damaged tissue. Fibrosis may cause overgrowth, hardening, and/or scarring that disrupts the architecture of the underlying organ or tissue. While controlled tissue remodeling and scarring is part of the normal wound healing process promoted by transdifferentiation of fibroblasts into myofibroblasts, excessive and persistent scarring due to severe or repetitive injury or dysregulated wound healing (e.g., persistence of myofibroblasts) can eventually result in permanent scarring, organ dysfunction and failure, and even death.

Fibrotic changes can occur in vascular disorders (e.g., peripheral vascular disease, cardiac disease, cerebral disease, *etc.)* and in all main tissue and organ systems (e.g., lung, liver, kidney, heart, skin, pancreas). Fibrotic disorders include a wide range of clinical presentations, including multisystemic disorders, such as systemic sclerosis, multifocal fibrosclerosis, scleroderma, myelofibrosis, and organ-specific disorders, such as pulmonary (e.g., idiopathic pulmonary fibrosis (IPF)), liver fibrosis, kidney fibrosis, pancreas fibrosis, heart fibrosis, and bladder fibrosis (see, e.g., Rosenbloom *et al.,* 2010; Wynn *et al.,* 2004; Wernig *et al.,* 2017). The mechanisms underlying fibrotic diseases are being actively studied. For example, it has been reported that diseased cells such as diseased fibroblasts upregulate the expression of CD47 at their cell surface thereby conveying a "don't eat me signal", which allows the diseased cells to evade phagocytosis by phagocyte cells, e.g., macrophages and/or neutrophils, so that diseased cells are not cleared by the immune system (see, e.g., Wernig *et al.,* 2017). It was further found that inhibiting the CD47-SIRP$\alpha$ axis by treatment

with an anti-CD47 antibody led to an increased phagocytosed diseased fibroblast, which in turn reduced fibrosis in the tissue (see, e.g., Wernig *et al.,* 2017). Accordingly, isoQC and/or QC inhibitors, such as those described herein, may be useful therapeutic agents for the treatment of fibrotic diseases.

### *INFECTIOUS DISEASES*

In embodiments, the disorder is: an infectious disease (e.g., an infection).

In embodiments, the disorder is: an infectious disease caused by a virus, bacterium, or protozoan.

In embodiments, the disorder is: an infectious disease caused by a virus.

In embodiments, the disorder is: an infectious disease caused by a bacterium.

In embodiments, the disorder is: an infectious disease caused by a protozoan.

In embodiments, the disorder is: an infectious disease caused by a pathogen selected from: a lentivirus, human T-lymphotropic virus (HTLV), an hepadna virus, hepatitis B virus, a herpes virus, human papilloma virus, la crosse virus, Yersinia sp., Yersinia pestis, Yersinia pseudotuberculosis, Yersinia enterocolitica, Franciscella sp., Helicobacter sp., Helicobacter pylori, Pasturella sp., Vibrio sp., Vibrio cholerae, Vibrio parahemolyticus, Legionella sp., Legionella pneumophila, Listeria sp., Listeria monocytogenes, Mycoplasma sp., Mycoplasma hominis, Mycoplasma pneumoniae, Mycobacterium sp., Mycobacterium tuberculosis, Mycobacterium leprae, Rickettsia sp., Rickettsia rickettsii, Rickettsia typhi, a Plasmodium, a Trypanosoma, a Giardia, a Toxoplasma, and a Leishmania.

Since the physiological function of the SIRPa-CD47 axis is thought to allow the immune system to discriminate self from non-self, inhibition of isoQC and/or QC in cells that, due to an infection, possess a pro-phagocytic signal may help to combat the infection. See, e.g., van den Berg *et al.,* 2008. Accordingly, isoQC and/or QC inhibitors, such as those described herein, may be useful therapeutic agents for the treatment of infectious diseases (e.g., infections).

### *ALZHEIMER'S DISEASE (AD)*

In embodiments, the disorder is: Alzheimer's disease.

Schilling *et al.,* 2008, demonstrates that pyroglutamylation of the Abeta protein is important in the pathogenesis of Alzheimer's disease. Accordingly, isoQC and/or QC inhibitors, such as those described herein, may be useful therapeutic agents for the treatment of Alzheimer's disease.

### *NON-ALCOHOLIC STEATOHEPATITIS (NASH)*

In embodiments, the disorder is: non-alcoholic steatohepatitis (NASH).

Cynis *et al.,* 2013, describes the potential application of pyroglutamylation inhibitors for the treatment of non-alcoholic steatohepatitis (NASH) through inhibition of CCL2 mediated inflammation. Accordingly, isoQC and/or QC inhibitors, such as those described herein, may be useful therapeutic agents for the treatment of NASH.

### *SEPTIC ARTHRITIS*

In embodiments, the disorder is: septic arthritis.

Hellvard *et al.,* 2012, describe the use of glutaminyl cyclase inhibitors for the treatment of septic arthritis. Accordingly, isoQC and/or QC inhibitors, such as those described herein, may be useful therapeutic agents for the treatment of septic arthritis.

### *COPD /ASTHMA /ALLERGIES*

In embodiments, the disorder is: chronic obstructive pulmonary disease (COPD), asthma, or an allergy.

In embodiments, the disorder is: chronic obstructive pulmonary disease (COPD).

In embodiments, the disorder is: asthma.

In embodiments, the disorder is: an allergy.

Raymond *et al.,* 2009, demonstrate that the CD47-SIRP$\alpha$ axis is important for Th2 chronic inflammation and that this is attenuated in CD47 deficiency. Accordingly, isoQC and/or QC inhibitors, such as those described herein, may be useful therapeutic agents for the treatment of COPD, asthma, and allergies.

### *PARASITIC INFECTIONS*

In embodiments, the disorder is: a parasitic infection.

In embodiments, the disorder is: malaria.

Nagaoka *et al.,* 2019, demonstrate an interaction between a malarial protein and erythrocyte CD47 is important for malaria infection. As this interaction may depend on CD47 to be pyroglutamylated, isoQC and/or QC inhibitors, such as those described herein, may be useful therapeutic agents for the treatment of parasitic infections, for example, malaria.

### *SICKLE-CELL ANEMIA*

In embodiments, the disorder is: sickle-cell anemia.
TSP-1 interaction with CD47 of sickle cell anemia patients makes the RBCs adhere to the vascular wall, which causes vaso-occlusion and other problems. Novelli *et al.,* June 2019, show that interfering with this may be of therapeutic use. As the interaction between TSP-1 and CD47 may be dependent on pyroglutamylation, isoQC and/or QC inhibitors, such as those described herein, may be useful therapeutic agents for the treatment of sickle-cell anemia.

### *HUNTINGTON'S DISEASE*

In embodiments, the disorder is: Huntington's disease.
Jimenez-Sanchez *et al.,* 2015, describes how glutaminyl cyclase inhibition suppresses mutant HTT induced toxicity. Accordingly, isoQC and/or QC inhibitors, such as those described herein, may be useful therapeutic agents for the treatment of Huntington's disease.

### *ISCHEMIA / REPERFUSION INJURY (RENAL /MYOCARDIAL /LIVER / CEREBRAL)*

In embodiments, the disorder is: ischemia or reperfusion injury (also known as ischemia-reperfusion injury).
In embodiments, the disorder is: ischemia.
In embodiments, the disorder is: reperfusion injury.
In embodiments, the disorder is: renal ischemia or reperfusion injury; myocardial ischemia or reperfusion injury; liver ischemia or reperfusion injury; or cerebral ischemia or reperfusion injury.
In embodiments, the disorder is: renal ischemia.
In embodiments, the disorder is: renal reperfusion injury.
In embodiments, the disorder is: myocardial ischemia.
In embodiments, the disorder is: myocardial reperfusion injury.
In embodiments, the disorder is: liver ischemia.
In embodiments, the disorder is: liver reperfusion injury.
In embodiments, the disorder is: cerebral ischemia.
In embodiments, the disorder is: cerebral reperfusion injury.
When the supply of oxygen is returned after ischemia, ensuing tissue damage leads to prolonged inflammation if the damaged cells are not efficiently removed by phagocytosis. The efficiency of this removal can be greatly inhibited by active CD47-SIRPa signalling, and the inhibition of this axis can therefore limit reperfusion injury after ischemia. See, e.g., Li *et al.,* April 2019; Isenberg *et al.,* 2018; Xu *et al.,* 2017; Wang *et al.,* 2017; Zhang *et al.,* 2017; Wang *et al.,* 2016; Xiao *et al.,* 2016; Rogers *et al.,* 2016; Xiao *et al.,* 2014; Lin *et al.,* 2014; Zhou *et al.,* 2014; Rogers *et al.,* 2012; Jin *et al.,* 2009; Isenberg *et al.,* 2007. Accordingly, isoQC and/or QC inhibitors, such as those described herein, may be useful therapeutic agents for the treatment of ischemia and reperfusion injury.

### *TREATMENT*

The term "treatment," as used herein in the context of treating a disorder, pertains generally to treatment of a human or an animal (e.g., in veterinary applications), in which some desired therapeutic effect is achieved, for example, the inhibition of the progress of the disorder, and includes a reduction in the rate of progress, a halt in the rate of progress, alleviation of symptoms of the disorder, amelioration of the disorder, and cure of the disorder. Treatment as a prophylactic measure (i.e., prophylaxis) is also included. For example, use with patients who have not yet developed the disorder, but who are at risk of developing the disorder, is encompassed by the term "treatment."
For example, treatment of cancer includes the prophylaxis of cancer, reducing the incidence of cancer, alleviating the symptoms of cancer, etc.
The term "therapeutically-effective amount," as used herein, pertains to that amount of a compound, or a material, composition, or dosage form comprising a compound, which is effective for producing some desired therapeutic effect, commensurate with a reasonable benefit/risk ratio, when administered in accordance with a desired treatment regimen.

***COMBINATION THERAPIES***

The compounds according to the invention may be used as monotherapies, for example where inhibition of pyroglutamylation of CD47 or other proteins provides a therapeutic benefit *per se,* as described herein e.g. to promote phagocytosis.

However, the term "treatment" includes combination treatments and therapies, in which two or more treatments or therapies are combined, for example, sequentially or simultaneously. For example, the compounds according to the invention may also be used in combination therapies, e.g., in conjunction with other agents.

Combination treatments or therapies include (without limitation) the following combinations of pairs of agents or modalities. It will be understood that second agents or modalities may fall into one or more of these categories:

- Compounds according to the invention and second agents which target (inhibit) the CD47-SIRPα signalling axis, such as agents which bind to CD47 or SIRPα. Such combinations may provide enhanced inhibition of that axis. Non-limiting examples include antibodies such as anti-CD47 antibodies and anti-SIRPα antibodies, and recombinant Fc-fusion proteins such as CD47-Fc and SIRPa-Fc;
- Compounds according to the invention and second agents which induce prophagocytic signals whether by virtue of the CD47-SIRPα signalling axis or otherwise, such as treatments based on Ionising Radiation (IR) or anthracyclines or anti-CD38 antibodies. Non-limiting examples include the anthracycline derivatives Doxorubicin (DOX), Daunarubicin (DNR), Epirubicin (EPI) and Idarubicin (IDA) Daratumumab;
- Compounds according to the invention and second agents which are independently suitable for treating any of the indications described herein. Such combinations may provide enhanced efficacy in treating such indications, particularly by targeting different aetiologies of the disorders. Non-limiting examples include Temozolomide (TMZ), used to treat, e.g., glioblastoma multiforme (GBM); Carfilzomib (Kyprolis®); Azacytidine; decitabine, etc.
- Compounds according to the invention and second agents which are large molecules such as proteins, e.g., antibodies, or different treatment modalities such as IR. Combinations of different types of treatments administered in different ways may provide advantages in terms of tolerance and compliance. Non-limiting examples include not only antibodies approved for use in the relevant indications, but also antibodies induced by vaccines, anti neoantigen antibodies, antibodies which have proven safe but ineffective as monotherapies; antibodies used at sub-effective (when used as monotherapy) dosages.

Combination treatments or therapies also include (without limitation) triple combinations of agents or modalities. Second and third agents may each fall into one or more of the categories of second agents described above.

In embodiments, compounds according to the invention are used to complement or enhance the effects of a monotherapy therapeutic antibody treatment, or of IR.

In embodiments, compounds according to the invention are used in combination with a therapeutic antibody which is an anti-CD47 antibody and/or anti-SIRPα antibody. Examples include Hu5F9-G4, ALX148, CC-95251, CC-90002, and IBI-188.

In embodiments, compounds according to the invention are used in combination with a recombinant Fc-fusion protein. Examples include TTI-621 and TTI-622.

In embodiments, compounds according to the invention are used in combination with a therapeutic antibody which is a PD1 or PD-L1 inhibitor such as an anti PD1 or anti PD-L1 antibody. Examples include Atezolizumab, Avelumab, and Durvalumab.

In embodiments, compounds according to the invention are used in combination with a therapeutic antibody which is selected from the list consisting of: an anti-Her2 antibody, an anti-EGFR antibody, and an anti-PDGFR antibody; an anti-GD2 (Ganglioside G2) antibody. Examples include Dinutuximab, Olaratumab, Trastuzumab, Pertuzumab, Ertumaxomab, Cetuximab, Necitumumab, Nimotuzumab, Panitumumab. Such combinations may be particularly beneficial when targeting solid tumors.

In embodiments, compounds according to the invention are used in combination with a therapeutic antibody which is selected from the list consisting of: an anti-CD19 antibody; an anti-CD20 antibody; an anti-CD38 antibody; an anti-SLAMF7 antibody; an anti-CCR40 antibody. Examples include Rituximab, Tafasitamab, Daratumumab, Elotuzumab, Mogamulizumab, Ofatumumab, Tositumomab, Obinutuzumab. Such combinations may be particularly beneficial when targeting liquid tumors.

In embodiments, compounds according to the invention are used in combination with a therapeutic antibody which is an anti-CD56 antibody or an anti-CD271-sporin antibody.

In embodiments, compounds according to the invention are used in combination with a therapeutic modality shown in the following table, i.e., as a double or triple combination, optionally for treating the disorder ("indication") shown therein. See, e.g., see Uger et al., 2020.

| Combination Therapies | | | | |
|---|---|---|---|---|
| Compound (Company) | Target | Format | Indication | Combination Cohorts |
| Hu5F9-G4 (Forty Seven) | CD47 | Antibody | Solid tumors | N/A |
| | | | AML, MDS | N/A |
| | | | B-cell NHL | Rituximab |
| | | | Solid tumors and colorectal cancer | Cetuximab |
| | | | Hematological malignancies | Azacitidine |
| | | | NHL | Rituximab + Acalabrutinib |
| | | | Ovarian cancer | Avelumab |
| | | | Urothelial carcinoma | Atezolizumab |
| | | | AML | Atezolizumab |
| CC-90002 (Celgene) | CD47 | Antibody | AML, MDS | N/A |
| | | | Solid and hematologic cancers | Rituximab |
| TTI-621 (Trillium Therapeutics) | CD47 | SIRPaFc | Hematologic malignancies, selected solid tumors | Rituximab Nivolumab |
| | | | Solid tumors, mycosis fungoides | Anti-PD-1/PD-L1 Peg IFN-α2a T-Vec Radiation |
| ALX148 (ALX Oncology) | CD47 | Mutated SIRPaFc | Solid tumors, lymphoma | Pembrolizumab Trastuzumab Rituximab |
| IBI188 (Innovent Biologics) | CD47 | Antibody | Advanced malignancies | Rituximab |
| | | | Advanced malignant tumors and lymphoma | N/A |
| SRF231 (Surface Oncology) | CD47 | Antibody | Solid and hematologic cancers | N/A |
| TTI-622 (Trillium Therapeutics) | CD47 | SIRPaFc | Lymphoma, multiple myeloma | Rituximab Anti-PD-1/PD-L1 Proteasome-Inhibitor |
| CC-95251 (Celgene) | SIRPα | Antibody | Solid tumors | Cetuximab |
| AO-176 (Arch Oncology) | CD47 | Antibody | Solid tumors | N/A |
| TJ011133 (I-Mab Biopharma) | CD47 | Antibody | Solid tumors, lymphoma | Pembrolizumab Rituximab |
| TG-1801 (TG Therapeutics) | CD47/CD19 | Bispecific antibody | B-cell lymphoma | N/A |
| BI 765063 (Boehringer Ingelheim/OSE) | SIRPα | Antibody | Solid tumors | Anti-PD-1 |
| SGN-CD47M (Seattle Genetics) | CD47 | Antibody | Solid tumors | N/A |

As used herein, the term "antibody" is used in a general sense to include any polypeptide or protein comprising an antibody antigen-binding site described herein, including Fab, $Fab_2$, $Fab_3$, diabodies, triabodies, tetrabodies, mini-

bodies and single-domain antibodies, as well as whole antibodies of any isotype or sub-class.

Both monospecific and bispecific antibodies are included. An example of a bispecific antibody is an anti-CD20-CD47 bispecific antibody or anti-CD19-CD47 bispecific antibody.

An antibody may, for example, be a single-chain variable fragment (scFv) or single-chain antibody (scAb). An scFv fragment is a fusion of a variable heavy (VH) and variable light (VL) chain. A scAb has a constant light (CL) chain fused to the VL chain of an scFv fragment. The CL chain is optionally the human kappa light chain (HuCκ). A single chain Fv (scFv) may be comprised within a mini-immunoglobulin or small immunoprotein (SIP), e.g., as described in Li *et al.,* 1997. A SIP may comprise an scFv molecule fused to the CH4 domain of the human IgE secretory isoform IgE-S2 ($\varepsilon_{S2}$-CH4; see, e.g., Batista *et al.,* 1996) forming an homo-dimeric mini-immunoglobulin antibody molecule.

Antibodies and methods for their construction and use are known in the art and described, in for example, Holliger *et al.,* 2005 and Liu *et al.,* 2020.

Antibodies used in the treatments herein may lack antibody constant regions.

However in preferred embodiments, antibodies are whole antibodies. For example, the antibody may be an IgG, IgA, IgE or IgM or any of the isotype sub-classes, particularly IgG1.

In embodiments, compounds according to the invention are used in combination with a therapeutic antibody which is a monoclonal antibody, optionally a human or humanised monoclonal antibody.

In embodiments, compounds according to the invention are used in combination with a therapeutic antibody which is an IgG antibody.

In embodiments, compounds according to the invention are used in combination with a therapeutic antibody which is an IgA antibody.

One aspect of the present invention pertains to a compound according to the invention in combination with one or more (e.g., 1, 2, 3, 4, *etc.)* additional therapeutic agents.

The particular combination would be at the discretion of the physician who would select dosages using their common general knowledge and dosing regimens known to a skilled practitioner.

The agents (e.g., the compound according to the invention, plus one or more other agents) may be administered simultaneously or sequentially, and may be administered in individually varying dose schedules and via different routes. For example, when administered sequentially, the agents can be administered at closely spaced intervals (e.g., over a period of 5-10 minutes) or at longer intervals (e.g., 1, 2, 3, 4 or more hours apart, or even longer periods apart where required), the precise dosage regimen being commensurate with the properties of the therapeutic agent(s).

The agents (e.g., the compound according to the invention, plus one or more other agents) may be formulated together in a single dosage form, or alternatively, the individual agents may be formulated separately, and may optionally be presented together in the form of a kit, optionally with instructions for their use.

### *ROUTES OF ADMINISTRATION*

The compound according to the invention or pharmaceutical composition comprising the compound according to the invention may be administered to a subject by any convenient route of administration, whether systemically/peripherally or topically (i.e., at the site of desired action).

Routes of administration include, but are not limited to, oral (e.g., by ingestion); buccal; sublingual; transdermal (including, e.g., by a patch, plaster, *etc*.); transmucosal (including, e.g., by a patch, plaster, *etc.*); intranasal (e.g., by nasal spray); ocular (e.g., by eye drops); pulmonary (e.g., by inhalation or insufflation therapy using, e.g., via an aerosol, e.g., through the mouth or nose); rectal (e.g., by suppository or enema); vaginal (e.g., by pessary); parenteral, for example, by injection, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal; by implant of a depot or reservoir, for example, subcutaneously or intramuscularly.

### *THE SUBJECT/PATIENT*

Groups, sub-groups or cohorts of subjects/patients who may particularly benefit from the treatments of the present invention may be selected for treatment with one or more compounds according to the invention. Such selection may be performed as an active step in any of the aspects or embodiments of the invention relating to treatment. For example such subjects/patients may be those suffering a disorder which may be associated with aberrant expression (e.g., upregulation) of CD47 on diseased or other undesirable cells, and the existence of such aberrant expression may be used as a selection criterion. Selected subjects/patients may additionally or alternatively be those who would benefit from reduced signalling or binding between CD47 on the surface of a first cell and SIRPα on the surface of a second cell e.g. where it is undesirable to utilise antibody therapies, or other protein or large biomolecule therapies, targeting CD47 and/or SIRPα e.g. as the subject/patient is refractory to such therapies, or they are otherwise unsuitable for the subject/patient (for example for the reasons explained in the section entitled "The CD47-SIRPα Signalling Axis" above).

Since the compounds according to the invention may demonstrate benefit via mechanisms such as phagocytosis, ADCC or ADCP, subjects/patients may additionally or alternatively be selected according to immune status to ensure such immunotherapies are most likely to succeed - for example subjects/patients having or demonstrating high levels of prophagocytic signals/macrophage infiltration e.g. in disease tissues.

The subject/patient may be a chordate, a vertebrate, a mammal, a placental mammal, a marsupial (e.g., kangaroo, wombat), a rodent (e.g., a guinea pig, a hamster, a rat, a mouse), murine (e.g., a mouse), a lagomorph (e.g., a rabbit), avian (e.g., a bird), canine (e.g., a dog), feline (e.g., a cat), equine (e.g., a horse), porcine (e.g., a pig), ovine (e.g., a sheep), bovine (e.g., a cow), a primate, simian (e.g., a monkey or ape), a monkey (e.g., marmoset, baboon), an ape (e.g., gorilla, chimpanzee, orangutan, gibbon), or a human.

Furthermore, the subject/patient may be any of its forms of development, for example, a foetus.

In embodiments, the subject/patient is a human.

***FORMULATIONS***

While it is possible for a compound according to the invention to be administered alone, it is preferable to present it as a pharmaceutical formulation (e.g., composition, preparation, medicament) comprising at least one compound according to the invention, as described herein, together with one or more other pharmaceutically acceptable ingredients well known to those skilled in the art, including, but not limited to, pharmaceutically acceptable carriers, diluents, excipients, adjuvants, fillers, buffers, preservatives, anti-oxidants, lubricants, stabilisers, solubilisers, surfactants (e.g., wetting agents), masking agents, colouring agents, flavouring agents, and sweetening agents. The formulation may further comprise other active agents, for example, other therapeutic or prophylactic agents.

Thus, also described herein are pharmaceutical compositions, as defined above, and methods of making a pharmaceutical composition comprising mixing at least one compound according to the invention together with one or more other pharmaceutically acceptable ingredients well known to those skilled in the art, e.g., carriers, diluents, excipients, *etc.* If formulated as discrete units (e.g., tablets, *etc*.), each unit contains a predetermined amount (dosage) of the compound.

The term "pharmaceutically acceptable," as used herein, pertains to compounds, ingredients, materials, compositions, dosage forms, *etc.,* which are, within the scope of sound medical judgment, suitable for use in contact with the tissues of the subject in question (e.g., human) without excessive toxicity, irritation, allergic response, or other problem or complication, commensurate with a reasonable benefit/risk ratio. Each carrier, diluent, excipient, *etc.* must also be "acceptable" in the sense of being compatible with the other ingredients of the formulation.

Suitable carriers, diluents, excipients, *etc.* can be found in standard pharmaceutical texts, for example, Remington: The Science and Practice of Pharmacy, 21st edition, Lippinott Williams and Wilkins, 2005; Remington: The Science and Practice of Pharmacy, 22nd edition, Pharmaceutical Press, 2012; and Handbook of Pharmaceutical Excipients, 7th edition, Pharmaceutical Press, 2012.

The formulations may be prepared by any methods well known in the art of pharmacy. Such methods include the step of bringing into association the compound with a carrier which constitutes one or more accessory ingredients. In general, the formulations are prepared by uniformly and intimately bringing into association the compound with carriers (e.g., liquid carriers, finely divided solid carrier, *etc*.), and then shaping the product, if necessary.

The formulation may be prepared to provide for rapid or slow release; immediate, delayed, timed, or sustained release; or a combination thereof.

Formulations may suitably be in the form of liquids, solutions (e.g., aqueous, non-aqueous), suspensions (e.g., aqueous, non-aqueous), emulsions (e.g., oil-in-water, water-in-oil), elixirs, syrups, electuaries, mouthwashes, drops, tablets (including, e.g., coated tablets), granules, powders, losenges, pastilles, capsules (including, e.g., hard and soft gelatin capsules), cachets, pills, ampoules, boluses, suppositories, pessaries, tinctures, gels, pastes, ointments, creams, lotions, oils, foams, sprays, mists, or aerosols.

Formulations may suitably be provided as a patch, adhesive plaster, bandage, dressing, or the like which is impregnated with one or more compounds and optionally one or more other pharmaceutically acceptable ingredients, including, for example, penetration, permeation, and absorption enhancers. Formulations may also suitably be provided in the form of a depot or reservoir.

The compound according to the invention may be dissolved in, suspended in, or mixed with one or more other pharmaceutically acceptable ingredients. The compound may be presented in a liposome or other microparticulate which is designed to target the compound, for example, to blood components or one or more organs.

Formulations suitable for oral administration (e.g., by ingestion) include liquids, solutions (e.g., aqueous, non-aqueous), suspensions (e.g., aqueous, non-aqueous), emulsions (e.g., oil-in-water, water-in-oil), elixirs, syrups, electuaries, tablets, granules, powders, capsules, cachets, pills, ampoules, boluses.

Formulations suitable for buccal administration include mouthwashes, losenges, pastilles, as well as patches, adhesive plasters, depots, and reservoirs. Losenges typically comprise the compound in a flavoured basis, usually

sucrose and acacia or tragacanth. Pastilles typically comprise the compound in an inert matrix, such as gelatin and glycerin, or sucrose and acacia. Mouthwashes typically comprise the compound in a suitable liquid carrier.

Formulations suitable for sublingual administration include tablets, losenges, pastilles, capsules, and pills.

Formulations suitable for oral transmucosal administration include liquids, solutions (e.g., aqueous, non-aqueous), suspensions (e.g., aqueous, non-aqueous), emulsions (e.g., oil-in-water, water-in-oil), mouthwashes, losenges, pastilles, as well as patches, adhesive plasters, depots, and reservoirs.

Formulations suitable for non-oral transmucosal administration include liquids, solutions (e.g., aqueous, non-aqueous), suspensions (e.g., aqueous, non-aqueous), emulsions (e.g., oil-in-water, water-in-oil), suppositories, pessaries, gels, pastes, ointments, creams, lotions, oils, as well as patches, adhesive plasters, depots, and reservoirs.

Formulations suitable for transdermal administration include gels, pastes, ointments, creams, lotions, and oils, as well as patches, adhesive plasters, bandages, dressings, depots, and reservoirs.

Tablets may be made by conventional means, e.g., compression or moulding, optionally with one or more accessory ingredients. Compressed tablets may be prepared by compressing in a suitable machine the compound in a free-flowing form such as a powder or granules, optionally mixed with one or more binders (e.g., povidone, gelatin, acacia, sorbitol, tragacanth, hydroxypropylmethyl cellulose); fillers or diluents (e.g., lactose, microcrystalline cellulose, calcium hydrogen phosphate); lubricants (e.g., magnesium stearate, talc, silica); disintegrants (e.g., sodium starch glycolate, cross-linked povidone, cross-linked sodium carboxymethyl cellulose); surface-active or dispersing or wetting agents (e.g., sodium lauryl sulfate); preservatives (e.g., methyl p-hydroxybenzoate, propyl p-hydroxybenzoate, sorbic acid); flavours, flavour enhancing agents, and sweeteners. Moulded tablets may be made by moulding in a suitable machine a mixture of the powdered compound moistened with an inert liquid diluent. The tablets may optionally be coated or scored and may be formulated so as to provide slow or controlled release of the compound therein using, for example, hydroxypropylmethyl cellulose in varying proportions to provide the desired release profile. Tablets may optionally be provided with a coating, for example, to affect release, for example an enteric coating, to provide release in parts of the gut other than the stomach.

Ointments are typically prepared from the compound and a paraffinic or a water-miscible ointment base.

Creams are typically prepared from the compound and an oil-in-water cream base. If desired, the aqueous phase of the cream base may include, for example, at least about 30% w/w of a polyhydric alcohol, i.e., an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the compound through the skin or other affected areas. Examples of such dermal penetration enhancers include dimethylsulfoxide and related analogues.

Emulsions are typically prepared from the compound and an oily phase, which may optionally comprise merely an emulsifier (otherwise known as an emulgent), or it may comprise a mixture of at least one emulsifier with a fat or an oil or with both a fat and an oil. Preferably, a hydrophilic emulsifier is included together with a lipophilic emulsifier which acts as a stabiliser. It is also preferred to include both an oil and a fat. Together, the emulsifier(s) with or without stabiliser(s) make up the so-called emulsifying wax, and the wax together with the oil and/or fat make up the so-called emulsifying ointment base which forms the oily dispersed phase of the cream formulations.

Suitable emulgents and emulsion stabilisers include Tween 60, Span 80, cetostearyl alcohol, myristyl alcohol, glyceryl monostearate and sodium lauryl sulfate. The choice of suitable oils or fats for the formulation is based on achieving the desired cosmetic properties, since the solubility of the compound in most oils likely to be used in pharmaceutical emulsion formulations may be very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for intranasal administration, where the carrier is a liquid, include, for example, nasal spray, nasal drops, or by aerosol administration by nebuliser, include aqueous or oily solutions of the compound.

Formulations suitable for intranasal administration, where the carrier is a solid, include, for example, those presented as a coarse powder having a particle size, for example, in the range of about 20 to about 500 microns which is administered in the manner in which snuff is taken, i.e., by rapid inhalation through the nasal passage from a container of the powder held close up to the nose.

Formulations suitable for pulmonary administration (e.g., by inhalation or insufflation therapy) include those presented as an aerosol spray from a pressurised pack, with the use of a suitable propellant, such as dichlorodifluoromethane, trichlorofluoromethane, dichorotetrafluoroethane, carbon dioxide, or other suitable gases.

Formulations suitable for ocular administration include eye drops wherein the compound is dissolved or suspended in a suitable carrier, especially an aqueous solvent for the compound.

Formulations suitable for rectal administration may be presented as a suppository with a suitable base comprising, for example, natural or hardened oils, waxes, fats, semi-liquid or liquid polyols, for example, cocoa butter or a salicylate; or as a solution or suspension for treatment by enema.
Formulations suitable for vaginal administration may be presented as pessaries, tampons, creams, gels, pastes, foams or spray formulations containing in addition to the compound, such carriers as are known in the art to be appropriate.
Formulations suitable for parenteral administration (e.g., by injection), include aqueous or non-aqueous, isotonic, pyrogen-free, sterile liquids (e.g., solutions, suspensions), in which the compound is dissolved, suspended, or otherwise provided (e.g., in a liposome or other microparticulate). Such liquids may additionally contain other pharmaceutically acceptable ingredients, such as anti-oxidants, buffers, preservatives, stabilisers, bacteriostats, suspending agents, thickening agents, and solutes which render the formulation isotonic with the blood (or other relevant bodily fluid) of the intended recipient. Examples of excipients include, for example, water, alcohols, polyols, glycerol, vegetable oils, and the like. Examples of suitable isotonic carriers for use in such formulations include Sodium Chloride Injection, Ringer's Solution, or Lactated Ringer's Injection. Typically, the concentration of the compound in the liquid is from about 1 ng/mL to about 10 μg/mL, for example from about 10 ng/mL to about 1 μg/mL. The formulations may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets.

***DOSAGE***

It will be appreciated by one of skill in the art that appropriate dosages of the compound according to the invention, and compositions comprising the compound according to the invention, can vary from patient to patient. Determining the optimal dosage will generally involve the balancing of the level of therapeutic benefit against any risk or deleterious side effects. The selected dosage level will depend on a variety of factors including, but not limited to, the activity of the particular compound according to the invention, the route of administration, the time of administration, the rate of excretion of the compound according to the invention, the duration of the treatment, other drugs, compounds, and/or materials used in combination, the severity of the disorder, and the species, sex, age, weight, condition, general health, and prior medical history of the patient. The amount of compound according to the invention and route of administration will ultimately be at the discretion of the physician, veterinarian, or clinician, although generally the dosage will be selected to achieve local concentrations at the site of action which achieve the desired effect without causing substantial harmful or deleterious side-effects.
Administration can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell(s) being treated, and the subject being treated. Single or multiple administrations can be carried out with the dose level and pattern being selected by the treating physician, veterinarian, or clinician.
In general, a suitable dose of the compound according to the invention is in the range of about 0.01 mg to about 5000 mg (more typically about 0.1 mg to about 300 mg) per kilogram body weight of the subject per day.
Where the compound is a salt, an ester, an amide, a prodrug, or the like, the amount administered is calculated on the basis of the parent compound and so the actual weight to be used is increased proportionately.

***Other therapeutic and non-therapeutic uses***

An aspect the invention pertains to a method of inhibiting glutaminyl-peptide cyclotransferase-like (isoQC) enzyme and/or glutaminyl-peptide cyclotransferase (QC) enzyme (e.g., inhibiting or reducing or blocking the activity or function of isoQC and/or QC enzyme), *in vitro* or *in vivo,* comprising contacting the isoQC and/or QC enzyme with an effective amount of a compound according to the invention.
An aspect of the invention pertains to a method of inhibiting glutaminyl-peptide cyclotransferase-like (isoQC) enzyme and/or glutaminyl-peptide cyclotransferase (QC) enzyme (e.g., inhibiting or reducing or blocking the activity or function of isoQC and/or QC enzyme) in a cell, *in vitro* or *in vivo,* comprising contacting the cell with an effective amount of a compound according to the invention.
In one embodiment, the method is performed *in vitro.*
In one embodiment, the method is performed *in vivo.*
In one embodiment, the compound according to the invention is provided in the form of a pharmaceutically acceptable composition.
One of ordinary skill in the art is readily able to determine whether or not a candidate compound inhibits isoQC and/or QC enzyme (e.g., inhibits or reduces or blocks or the activity or function of isoQC and/or QC enzyme). For

example, suitable assays are described herein and/or are known in the art.

One of ordinary skill in the art is readily able to determine whether or not a candidate compound inhibits isoQC and/or QC enzyme (e.g., inhibits or reduces or blocks or the activity or function of isoQC and/or QC enzyme) in a cell. For example, a sample of cells may be grown *in vitro* and a compound brought into contact with said cells, and the effect of the compound on those cells observed. As an example of "effect," the morphological status of the cells (e.g., alive or dead, *etc.)* may be determined. Where the compound is found to exert an influence on the cells, this may be used as a prognostic or diagnostic marker of the efficacy of the compound in methods of treating a patient carrying cells of the same cellular type.

The compounds according to the invention may e.g., (a) regulate (e.g., inhibit) cell proliferation; (b) inhibit cell cycle progression; (c) promote apoptosis; or (d) a combination of one or more of these.

An aspect of the invention pertains to a method of regulating (e.g., inhibiting) cell proliferation (e.g., proliferation of a cell), inhibiting cell cycle progression, promoting apoptosis, or a combination of one or more these, *in vitro* or *in vivo,* comprising contacting a cell with an effective amount of a compound according to the invention.

The compounds according to the invention described herein may be used in methods to reduce the formation of a pyroglutamyl residue at the N-terminus of CD47 expressed at the surface of a first cell in the subject. This can result in a reduction or inhibition of the binding or other interaction between the CD47 on the surface of the first cell, and SIRP$\alpha$ on the surface of a second cell.

The compounds according to the invention described herein may be used in methods to reduce binding or other interaction between CD47 on the surface of a first cell and SIRP$\alpha$ on the surface of a second cell.

The first cell with CD47 on the surface may be a cell which is a diseased cell, or other undesirable cell. The cell may be selected from: a cancer cell expressing or overexpressing CD47, a vascular smooth muscle cells expressing or overexpressing CD47, a diseased endothelial cell expressing or overexpressing CD47, a diseased cell infected by a pathogen, which is optionally a virus, expressing or overexpressing CD47, and a diseased cell undergoing fibrosis expressing or overexpressing CD47 on its cell surface.

The first cell may overexpress CD47 on its surface (i.e., it is upregulated).

The second cell expressing the SIRP$\alpha$ on its surface may be an immune cell such as a phagocyte cell (e.g., macrophage, neutrophil). Optionally the second cell is a myeloid cell, which is optionally selected from the group consisting of a macrophage, monocyte, neutrophil, basophil, eosinophil, and dendritic cell.

As explained above, reducing binding or other interaction may be achieved by inhibiting QPCTL in the first cell, and thereby inhibiting pyroglutamylation of the N-terminal glutamine moiety of CD47 on the surface of the first cell.

Reducing binding between said CD47 on the surface of said first cell and said SIRP$\alpha$ on the surface of said second cell may target said first cell with CD47 on the surface for phagocytosis, antibody-dependent cellular cytotoxicity (ADCC) or antibody-dependent cellular phagocytosis (abbreviated ADCP). In particular, the recognition of tumor-specific antigens (or other antigens associated with diseased or undesirable cells) by therapeutic antibodies can result in the coating, or opsonization, of the cells, and this can lead to ADCP.

The compounds according to the invention may be used in methods to promote immunotherapy.

The compounds according to the invention may be used in methods to promote immune-cell mediated killing of diseased or other undesirable cells expressing, or upregulating CD47.

The diseased or other undesirable cells may be those discussed above.

The immune cells may be those discussed above.

In one embodiment, the method is performed *in vitro.*

In one embodiment, the method is performed *in vivo.*

In one embodiment, the compound according to the invention is provided in the form of a pharmaceutically acceptable composition.

Any type of cell may be treated or targeted, including but not limited to, blood (including, e.g., neutrophils, eosinophils, basophils, lymphocytes, monocytes, erythrocytes, thrombocytes), lung, gastrointestinal (including, e.g., bowel, colon), breast (mammary), ovarian, prostate, liver (hepatic), kidney (renal), bladder, pancreas, brain, and skin cells.

One of ordinary skill in the art is readily able to determine whether or not a candidate compound regulates (e.g., inhibits) cell proliferation, *etc.* For example, assays which may conveniently be used to assess the activity offered by a particular compound are described herein and/or are known in the art.

In the light of the disclosure herein, one of ordinary skill in the art is readily able to confirm that a candidate compound inhibits formation of a pyroglutamyl residue at the N-terminus of CD47 and/or inhibits binding between said CD47 on the surface of said first cell and said SIRP$\alpha$ on the surface of said second cell. For example, assays which may conveniently be used to assess or confirm the activity offered by a particular compound are described herein and/or are known in the art. Assays may be performed *in vitro* e.g. using purified enzymes. Alternatively assays may be cell-based. For example they may assess whether the compound reduces the formation of a pyroglutamyl residue at the N-terminus of CD47 expressed at the surface of a first cell, with a resultant reduction or inhibition of the binding or other interaction between the CD47 on the surface of that first cell, and SIRP$\alpha$ on the surface of a second cell.

Alternatively, for example, a sample of cells (e.g., from a tumour) may be grown *in vitro* and a compound brought into contact with said cells, and the effect of the compound on those cells observed. As an example of "effect," the morphological status of the cells (e.g., alive or dead, *etc.)* may be determined. Where the compound is found to exert an influence on the cells, this may be used as a prognostic or diagnostic marker of the efficacy of the compound in methods of treating a patient carrying cells of the same cellular type.

The compounds according to the invention may also be used as cell culture additives to inhibit glutaminyl-peptide cyclotransferase-like (isoQC) enzyme and/or glutaminyl-peptide cyclotransferase (QC) enzyme (e.g., to inhibit or reduce or block the activity or function of isoQC and/or QC enzyme).

The compounds according to the invention may also be used as part of an *in vitro* assay, for example, in order to determine whether a candidate host is likely to benefit from treatment with the compound in question.

The compounds according to the invention may also be used as a standard, for example, in an assay, in order to identify other active compounds, other isoQC and/or QC enzyme inhibitors, *etc.*

**Definitions**

In this document and in its claims, the verb "to comprise" and its conjugations is used in its non-limiting sense to mean that items following the word are included, but items not specifically mentioned are not excluded. In addition the verb "to consist" may be replaced by "to consist essentially of" meaning that a product, an assay device respectively a method or a use as defined herein may comprise additional component(s) respectively additional step(s) than the ones specifically identified, said additional component(s), respectively step(s) not altering the unique characteristic of the invention.

In addition, reference to an element by the indefinite article "a" or "an" does not exclude the possibility that more than one of the element is present, unless the context clearly requires that there be one and only one of the elements. The indefinite article "a" or "an" thus usually means "at least one".

An alkyl refers to a saturated hydrocarbon radical (i.e. substituent), which may be linear, branched or cyclic.

A Cn-Cm alkyl refers to an alkyl, wherein the number of carbon atoms comprised in the radical is n to m.

A (Cn-Cm) haloalkyl refers to a (Cn-Cm) alkyl wherein one or more hydrogen atoms are substituted by a halogen atom. A fluoro-, chloro-, bromo-, iodo- and astato(-Cn-Cm)-)alkyl is a (Cn-Cm) haloalkyl wherein the hydrogen atoms are substituted by one or more fluorine, chlorine, iodine or astatine atoms, respectively.

A (Cn-Cm) perhaloalkyl refers to a (Cn-Cm) alkyl wherein all hydrogen atoms are substituted by a halogen atom. A perfluoro-, perchloro-, perbromo-, periodo- and perastatoalkyl is a (Cn-Cm) alkyl wherein all hydrogen atoms are substituted by a more fluorine, chlorine, iodine or astatine atom, respectively.

A halogen is F, Cl, Br, I, or At.

A pseudohalogen is -CN, -CP, -NC, -OH, -SH, -SeH,-TeH, -OCN, -SCN, -NCS, -SeCN, -TeCN, $-N_3$, -NO, or -NOz.

All patent and literature references cited in the present specification are hereby incorporated by reference in their entirety.

**Examples**

The invention is explained in more detail below with a number of examples, which are not to be construed as limiting the scope of the invention. The invention is not limited to the forms of implementation described in the cases given as examples. The invention also extends to each combination of measures as described above, independently from each other.

***Example 1 - Synthesis of compounds (1)-(16) according to the invention***

The following synthetic schemes are provided for purpose of illustration, not limitation. The following examples illustrate the various methods of making the compounds described herein. It is understood that one skilled in the art may be able to make these compounds by similar methods or by combining other methods known to one skilled in the art. In general, starting materials and reagents can be obtained from commercial vendors or synthesized according to known sources to those skilled in the art or prepared as described herein. Solvents and commercial starting materials were used without further purification unless stated otherwise.

Intermediate compounds are denoted by Arabic numerals, whereas compounds according to the invention are denoted by Arabic numerals between parantheses. Reference compounds used to benchmark compounds according to the invention are also denoted by Arabic numerals between parantheses.

***EXAMPLE 1.1 - SYNTHESIS OF COMPOUND (1)***

***SYNTHESIS OF COMPOUND 2***

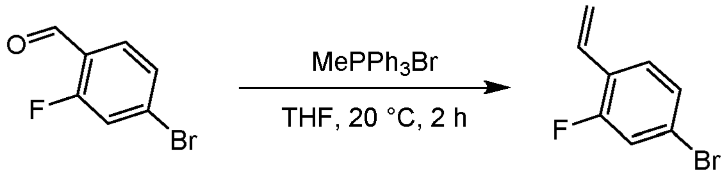


**1** **2**

To a mixture of **compound 1** (20 g, 98.52 mmol, 1 eq) and bromo-methyl-triphenyl-A5-phosphane (42.23 g, 118.22 mmol, 1.2 eq) in THF (300 mL) was added t-BuOK (13.27 g, 118.22 mmol, 1.2 eq) slowly under N2 and the mixture was stirred at 25 °C for 2 h. The mixture was quenched with water (1000 mL) and the resulting mixture was extracted with MTBE (300 mL*2). The combined organic phase was dried over $Na_2SO_4$, filtered and concentrated at reduced pressure to give a residue. The residue was purified by silica gel chromatography (100 - 200 mesh silica gel, Petroleum ether/Ethyl acetate = 1/0) to give **compound 2** (20 g, crude) as colorless liquid.

***SYNTHESIS OF COMPOUND 4***

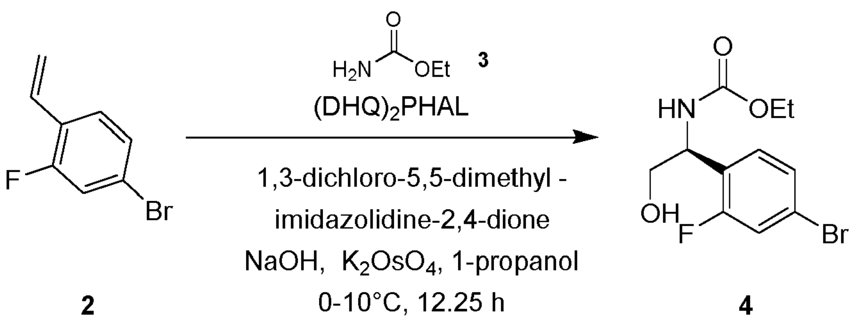


**2** **4**

To a solution of **compound 3** (19.94 g, 223.84 mmol, 3 eq) in 1 -PROPANOL (150 mL) and NaOH (0.5 M, 447.68 mL, 3 eq) was added 1, 3 -dichloro- 5, 5 -dimethyl-imidazolidine-2, 4 -dione (22.05 g, 111.92 mmol, 1.5 eq) at 0 °C in portions and the resulting mixture was stirred at 0 °C for 15 min. Then a solution of 4 - [ (R) - [ (2R, 4R, 5S) - 5 -ethylquinuclidin-2 -yl] - (6 -methoxy- 4 -quinolyl) methoxy] - 1 - [ (R) - [ (2R, 4S, 5R) - 5 -ethylquinuclidin- 2 -yl] - (6 -methoxy- 4 -quinolyl) methoxy] phthalazine (5.81 g, 7.46 mmol, 0.1 eq) which was dissolved with 1-propanol (150 mL) was added to the mixture, followed by adding a solution of **compound 2** (15 g, 74.61 mmol, 1 eq) which was dissolved with 1 -propanol (150 mL), then $K_2OsO_4.2H_2O$ (1.92 g, 5.22 mmol, 0.07 eq) which was dissolved with NaOH (0.5 M, 14.92 mL, 0.1 eq) was added and the resulting mixture was stirred at 25 °C for 12 h. The mixture was diluted with water (1000 mL) and the resulting mixture was extracted with ethyl acetate (500 mL*3), the combined organic phase was dried over Na2SO4, filtered and concentrated at reduced pressure to give a residue. The residue was purified by silica gel chromatography (100 - 200 mesh silica gel, Petroleum ether/Ethyl acetate = 10/1, 3/1) to give **compound 4** (16.4 g, crude) as white solid.

***SYNTHESIS OF COMPOUND 5***

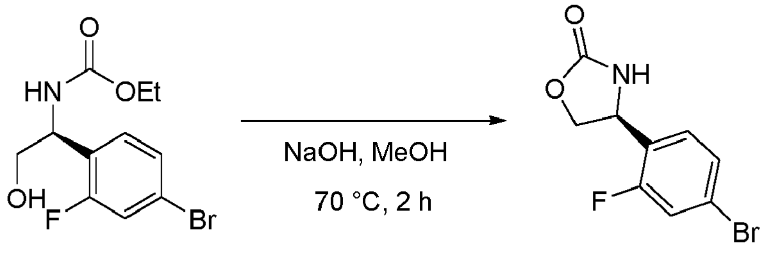


4 5

To a solution of **compound 4** (16.4 g, 53.57 mmol, 1 eq) in MeOH (720 mL) was added NaOH (0.2 M, 723.23 mL, 2.7 eq). The mixture was stirred at 70 °C for 1 h. The mixture was diluted with water (1500 mL) and the resulting mixture was extracted with ethyl acetate (500 mL*3). The combined organic phase was dried over Na2SO4, filtered and concentrated at reduced pressure to give a residue. The residue was purified with prep-HPLC (column: UniSil 10 - 120 C18 70x250 mm; mobile phase: [water (FA) -ACN]; B%: 15%- 45%, 30 min) to give **compound 5** (7 g, 26.92 mmol, 50.24% yield) as colorless oil. $^{1}H$ NMR: (400 MHz, CHLOROFORM-d) $\delta$ = 8.02 (s, 1H), 7.40 - 7.36 (m, 1H), 7.35 - 7.28 (m, 2H), 6.66 - 6.08 (m, 1H), 5.27 - 5.20 (m, 1H), 4.82 (t, J = 8.8 Hz, 1H), 4.20 (dd, J = 6.3, 8.6 Hz, 1H).

***SYNTHESIS OF COMPOUND 7***

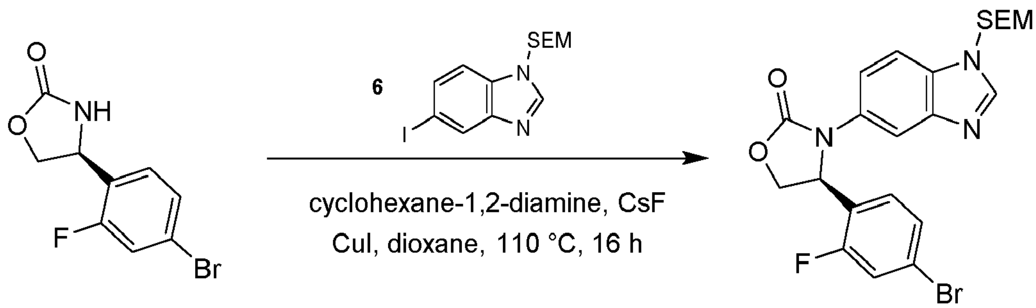


5 7

To a solution of **compound 5** (5.8 g, 22.30 mmol, 1 eq) and compound 6 (6.68 g, 17.84 mmol, 0.8 eq) in dioxane (90 mL) was added CuI (1.27 g, 6.69 mmol, 0.3 eq), CsF (6.78 g, 44.61 mmol, 1.64 mL, 2 eq) and (1S, 2S) -cyclohexane-1, 2 -diamine (764.02 mg, 6.69 mmol, 820.64 uL, 0.3 eq) under N2. The mixture was stirred at 110 °C for 16 h under N2. The reaction mixture was concentrated under vacuum to give a residue. The residue was purified by column chromatography (100 mesh silica gel, petroleum ether: ethyl acetate = 1: 0 to 1: 1) to give **compound 7** (9.4 g, crude) as yellow gum. LCMS: RT =0.537 min, m/z =508.3 $(M+H)^{+}$.

***SYNTHESIS OF COMPOUND 9***

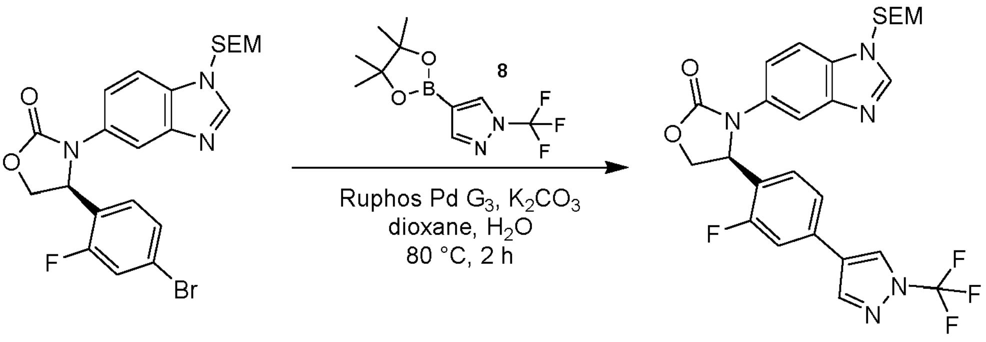


7 9

To a mixture of **compound 7** (1 g, 1.97 mmol, 1 eq) and **compound 8** (569.15 mg, 2.17 mmol, 1.1 eq) in dioxane (9 mL) and H2O (3 mL) was added RuPhos Pd G3 (165.15 mg, 197.46 μmol, 0.1 eq) and K2CO3 (545.80 mg, 3.95 mmol, 2 eq) under N2. The mixture was stirred at 80 °C for 2 h under N2.

To a mixture of **compound 7** (8.4 g, 16.59 mmol, 1 eq) and **compound 8** (4.78 g, 18.25 mmol, 1.1 eq) in dioxane (90 mL) and H2O (30 mL) was added RuPhos Pd G3 (1.39 g, 1.66 mmol, 0.1 eq) and K2CO3 (4.58 g, 33.17 mmol, 2 eq) under N2. The mixture was stirred at 80 °C for 2 h under N2.

The reaction two batches of mixture were combined, the combined mixture was diluted with ethyl acetate (200 mL). The organic layer was washed with brine (100 mL*2), dried over $Na_2SO_4$, filtered and the filtrate was concentrated under vacuum to give a residue. The residue was purified by column chromatography (100 mesh silica gel, petroleum ether: ethyl acetate = 1: 0 to 1: 1) to give **compound 9** (9.1 g, crude) as yellow gum. LCMS: RT =0.543 min, m/z =562.4 $(M+H)^+$.

***SYNTHESIS OF COMPOUND (1)***

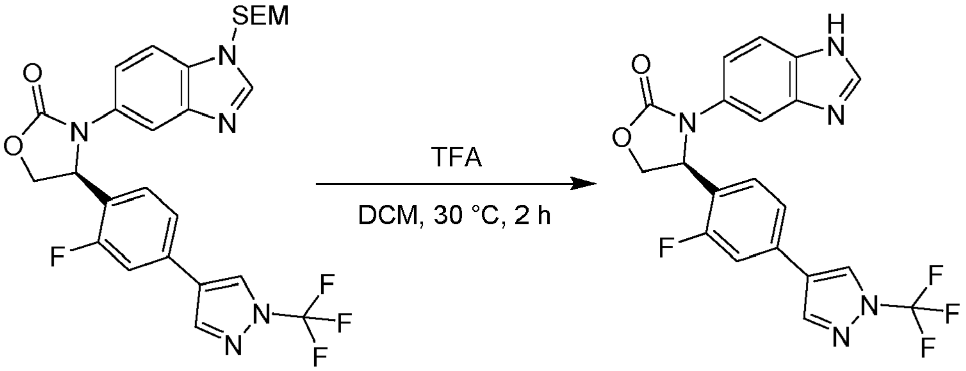


9 **(1)**

To a solution of **compound 9** (8.6 g, 15.31 mmol, 1 eq) in DCM (80 mL) was added TFA (80 mL), the mixture was stirred at 30 °C for 2 h. The reaction mixture was concentrated under vacuum to give a residue. The residue was purified by prep-HPLC (column: Phenomenex luna C18 (250*70 mm, 10 um); mobile phase: [water (FA) -ACN]; B%: 20%- 50%, 15 min) to give 7.2 g white solid. The 7.2 g white solid was diluted with ethyl acetate (100 mL) and washed with saturated NaHCOs aqueous solution (30 mL*2) and brine (30 mL). The organic layer was dried over Na2SO4, filtered and the filtrate was concentrated under vacuum to give a residue. The residue was diluted with water (30 mL) and lyophilized to give **(1)** (4.75 g, 10.69 mmol, 69.78% yield, 97% purity) as white solid.

**$^1$H NMR:** (400 MHz, METHANOL-$d_4$) $\delta$ = 8.61 - 8.54 (m, 1H), 8.17 (br d, J = 4.0 Hz, 1H), 8.13 (d, J = 1.3 Hz, 1H), 7.66 (d, J = 1.9 Hz, 1H), 7.55 - 7.30 (m, 5H), 5.93 (br dd, J = 3.4, 5.4 Hz, 1H), 4.94 (dt, J = 2.1, 8.8 Hz, 1H), 4.45 - 4.36 (m, 1H)

**LCMS:** RT =0.427 min, m/z =432.1 $(M+H)^+$.
**SFC:** RT =2.261 min

### *EXAMPLE 1.2 - SYNTHESIS OF COMPOUND (2)*

### *GENERAL PROCEDURE FOR PREPARATION OF COMPOUND 2*

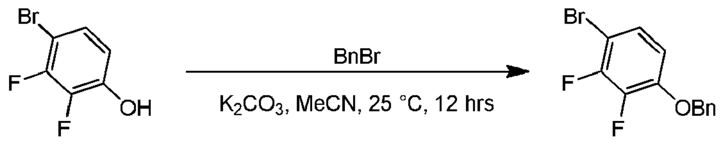


**1** **2**

To a solution of compound **1** (20.0 g, 95.7 mmol, 1.00 eq) in MeCN (200 mL) was added $K_2CO_3$ (26.4 g, 191 mmol, 2.00 eq) and BnBr (19.6 g, 114 mmol, 13.64 mL, 1.20 eq). The mixture was stirred at 25 °C for 12 hrs. TLC (Petroleum ether: Ethyl acetate = 10:1) showed compound **1** ($R_f$ = 0.3) was consumed and new spots ($R_f$ = 0.6, 0.75) formed. The mixture was filtrated and the filtrate was concentrated to give a residue. The residue was purified by column chromatography (silica gel, Petroleum ether: Ethyl acetate = 1:0 to 10:1) to give compound **2** ($R_f$ = 0.6) (25.0 g, 83.6 mmol, 87.3% yield) as a white solid. $^1$H NMR (400 MHz $CDCl_3$) δ 7.45-7.39 (m, 5H), 7.21-7.19 (m, 1H), 6.73-6.72 (m, 1H), 5.16 (s, 2H).

### *GENERAL PROCEDURE FOR PREPARATION OF COMPOUND 3*

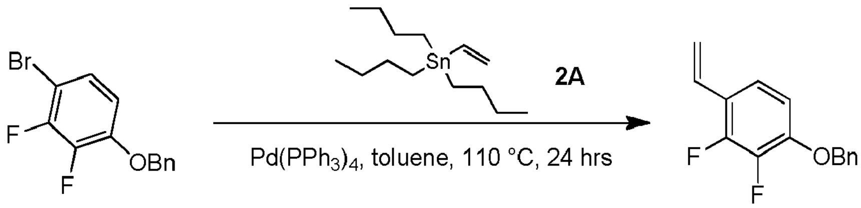


**2** **3**

To a solution of compound **2** (24.0 g, 80.2 mmol, 1.00 eq.) and compound **2A** (90.5 g, 285 mmol, 83.06 mL, 3.56 eq.) in toluene (250 mL) was added $Pd(PPh_3)_4$ (4.80 g, 4.15 mmol, 0.05 eq.). The reaction mixture was stirred at 110 °C for 24 hrs under $N_2$ atmosphere. The mixture was added to an aqueous KF solution (500 mL) and stirred at 25 °C for 0.5 hr. The mixture was extracted with EtOAc (300 mL * 3). The combined organic layers were dried over anhydrous sodium sulphate, filtrated and concentrated to give a residue. The residue was purified by column chromatography (silica gel, Petroleum ether: Ethyl acetate = 1 : 0 to 20:1 ) to get compound **3** (Petroleum ether: Ethyl acetate = 10:1, $R_f$ = 0.6) (18.0 g, crude) as a white solid. $^1$H NMR (400 MHz $CDCl_3$) δ 7.47-7.36 (m, 5H), 7.12-7.10 (m, 1H), 6.81-6.74 (m, 2H), 5.78-5.74 (m, 1H), 5.36-5.33 (m, 1H), 5.17 (s, 2H).

### *GENERAL PROCEDURE FOR PREPARATION OF COMPOUND 4*

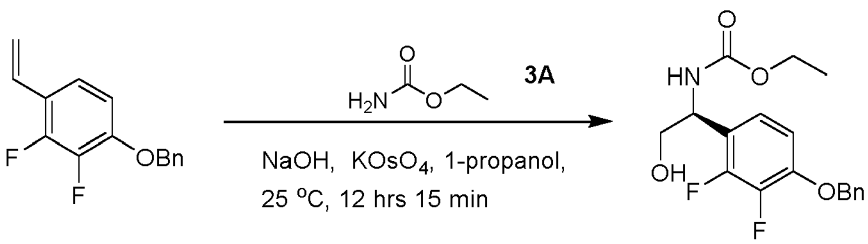


**3** **4**

Compound **3A** (19.5 g, 219 mmol, 3.00 eq) was dissolved in 1-propanol (180 mL) and NaOH (0.5 M, 441 mL, 3.02 eq). The mixture was stirred at 25 °C for 5 mins. Then 1,3-dichloro-5,5-dimethyl -imidazolidine-2,4-dione (21.6 g, 109 mmol, 1.50 eq) was added to the mixture and the mixture was stirred at 25 °C for 10 mins. A mixture of 4-[(R)-[(2R,4R,5S)-5-ethylquinuclidin-2-yl]-(6-methoxy-4-quinolyl) methoxy]-1-[(R)-[(2R,4S,5R)-5-ethylquinuclidin-2-yl]-(6-methoxy-4-quinolyl)methoxy]phthalazine (3.60 g, 4.62 mmol, 0.06 eq) in 1-propanol (180 mL) was added to the mixture. Then compound **3** (18.0 g, 73.1 mmol, 1.00 eq) in 1-propanol (180 mL) was added to the mixture followed by added a mixture of dipotassium;dioxido(dioxo)osmium;dihydrate (1.08 g, 2.93 mmol, 0.04 eq) in NaOH (0.5 M, 18.0 mL, 0.1 eq). The reaction mixture was stirred at 25 °C for 12 hrs. The mixture was concentrated and added water (500 mL), extracted with EtOAc (500 mL * 2). The combined organic layers were dried over anhydrous sodium sulphate, filtrated and concentrated to give a residue. The crude product was purified by reversed-phase HPLC (0.1% $NH_3$•$H_2O$ in acetonitrile) to get compound **4** (12.0 g, 34.1 mmol, 46.7% yield) as a white solid. $^1H$ NMR: (400 MHz $CDCl_3$) δ 7.44-7.36 (m, 5H), 6.96-6.79 (m, 1H), 6.77-6.76 (m, 1H), 5.47-5.45 (m, 1H), 5.14 (s, 2H), 5.05-5.00 (m, 1H), 4.16-4.09 (m, 2H), 3.89-3.85 (m, 2H), 2.05 (s, 1H), 1.26-1.23 (m, 3H).

***GENERAL PROCEDURE FOR PREPARATION OF COMPOUND 5***

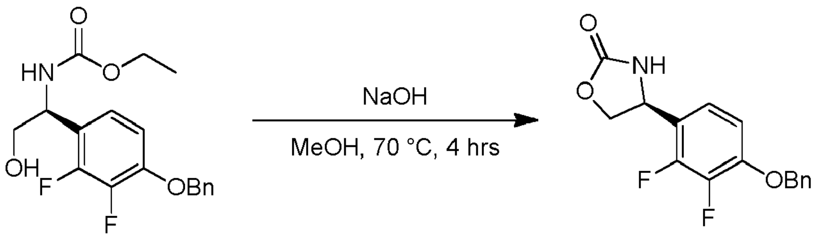


**4** **5**

To a solution of compound **4** (12.0 g, 34.1 mmol, 1.00 eq) in MeOH (460 mL) was added NaOH (0.2 M, 460 mL, 2.69 eq). The mixture was stirred at 70 °C for 4 hrs. The mixture was concentrated to remove MeOH and the mixture was extracted with EtOAc (500 mL * 2). The combined organic layers were dried over anhydrous sodium sulphate, filtrated and concentrated to give a residue. The crude product was purified by reversed-phase HPLC (0.1% FA condition in acetonitrile) to get compound **5** (6.40 g, 19.7 mmol, 57.8% yield, 94.1% purity) as a white solid. $^1H$ NMR (400 MHz $CDCl_3$) δ 7.44-7.35 (m, 5H), 7.06-6.85 (m, 1H), 6.84-6.79 (m, 1H), 6.08 (s, 1H), 5.22-5.14 (m, 3H), 4.81-4.76 (m, 1H), 4.23-4.16 (m, 1H).

***GENERAL PROCEDURE FOR PREPARATION OF COMPOUND 5A***

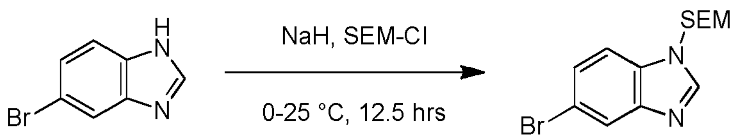


**A** **5A**

To a solution of compound **A** (10.0 g, 50.7 mmol, 1.00 eq) in THF (100 mL) was added NaH (2.23 g, 55.8 mmol, 60% purity, 1.10 eq) at 0 °C. The mixture was stirred at 0 °C for 0.5 hr, then SEM-Cl (10.1 g, 60.9 mmol, 10.7 mL, 1.20 eq) was added to the mixture and the mixture was stirred at 25 °C for 12 hrs. TLC (Petroleum ether: Ethyl acetate = 0:1) showed compound **A** (Rf = 0.3) was consumed and new spot (Rf = 0.65) formed. The mixture was quenched with water (300 mL) and extracted with EtOAc (200 ml * 2). The combined organic layers were dried over anhydrous sodium sulphate, filtrated and concentrated to give a residue. The residue was purified by column chromatography (silica gel, Petroleum ether: Ethyl acetate = 20:1 to 5:1) to get compound **5A** (Petroleum ether: Ethyl acetate = 0:1, Rf = 0.65) (16.0 g, crude) as a yellow oil. $^1H$ NMR (400 MHz $CDCl_3$) δ 7.94-7.93 (m, 1H), 7.69-7.65 (m, 1H), 7.41-7.39 (m, 2H), 5.50-5.48 (m, 2H), 3.51-3.46 (m, 2H), 0.90-0.86 (m, 2H), -0.05 - -0.06 (m, 9H).

***General procedure for preparation of compound 6***

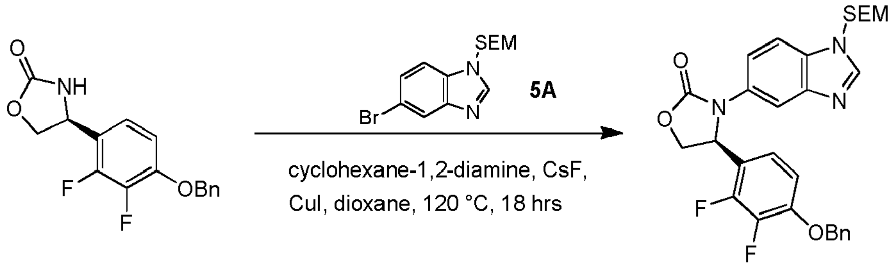


**5** **6**

To a solution of compound **5** (6.30 g, 19.4 mmol, 94.1% purity, 1.00 eq) and compound **5A** (6.52 g, 19.9 mmol, 1.03 eq) in dioxane (60 mL) was added CsF (5.93 g, 39.0 mmol, 1.44 mL, 2.01 eq), cyclohexane-1, 2-diamine (665 mg, 5.83 mmol, 715 μL, 0.30 eq) and CuI (1.11 g, 5.83 mmol, 0.30 eq). The mixture was stirred at 120 °C for 18 hrs. The mixture was filtrated and the filtrate was concentrated to give a residue. The residue was purified by column chromatography (silica gel, Petroleum ether: Ethyl acetate = 10:1 to 1:1) to get compound 6 (Petroleum ether: Ethyl acetate = 0:1, Rf = 0.3) (8.70 g, 15.8 mmol, 81.2% yield) as a yellow oil. $^1$H NMR (400 MHz $CDCl_3$) δ 7.92-7.91 (m, 1H), 7.72-7.71 (m, 1H), 7.59-7.40 (m, 1H), 7.38-7.11 (m, 6H), 6.65-6.64 (m, 1H), 6.63-6.61 (m, 1H), 5.67-5.63 (m, 1H), 5.40-5.38 (m, 2H), 4.98 (s, 2H), 4.77-4.72 (m, 1H), 4.22-4.18 (m, 1H), 3.44-3.40 (m, 2H), 0.83-0.78 (m, 2H), -0.12 - -0.15 (m, 9H).

***General procedure for preparation of compound 7***

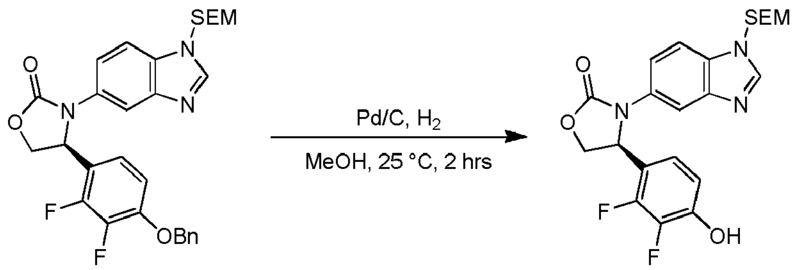


**6** **7**

To a solution of compound **6** (8.70 g, 15.8 mmol, 1.00 eq) in MeOH (150 mL) was added Pd/C (1.50 g, 150 μmol, 10% purity) under $N_2$. The suspension was degassed under vacuum and purged with Hz several times. The mixture was stirred under $H_2$ (15 psi) at 25 °C for 2 hours. The mixture was filtrated and the filtrate was concentrated to give compound **7** (6.50 g, 14.1 mmol, 89.3% yield) as a white solid which was used in the next step without purification. $^1$H NMR (400 MHz $CDCl_3$) δ 8.03-8.01 (m, 1H), 7.92-7.57 (m, 1H), 7.52-7.50 (m, 2H), 7.08-7.05 (m, 1H), 6.87-6.80 (m, 1H), 6.67-6.65 (m, 1H), 5.74-5.68 (m, 1H), 5.53-5.46 (m, 2H), 4.85-4.76 (m, 1H), 4.33-4.29 (m, 1H), 3.54-3.50 (m, 2H), 0.92-0.87 (m, 2H), -0.02 - -0.06 (m, 9H).

***GENERAL PROCEDURE FOR PREPARATION OF COMPOUND 950-1***

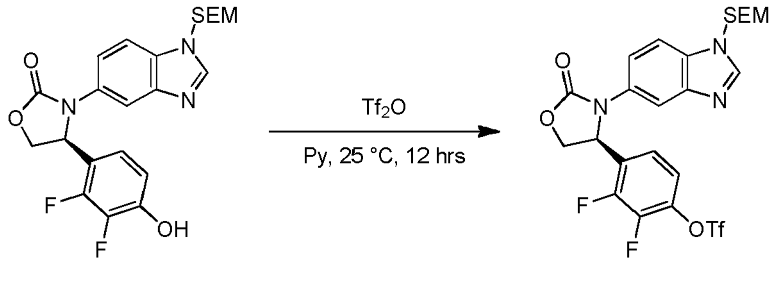


7 950-1

To a solution of compound **7** (2.00 g, 4.33 mmol, 1.00 eq) in Py (20 mL) was added trifluoromethylsulfonyl trifluoromethanesulfonate (1.37 g, 4.85 mmol, 0.8 mL, 1.12 eq). The mixture was stirred at 25 °C for 12 hrs. The mixture was diluted with EtOAc (30 mL) and washed with water (50 ml). The organic layer was dried over anhydrous sodium sulphate, filtrated and concentrated to give a residue. The residue was purified by column chromatography (silica gel, Petroleum ether: Ethyl acetate = 10:1 to 1:1) to get compound **950-1** (Rf = 0.4, Petroleum ether: Ethyl acetate = 0:1) (500 mg, 842 μmol, 19.4% yield) as a yellow solid. $^1H$ NMR (400 MHz $CDCl_3$) δ 7.99-7.79 (m, 1H), 7.73-7.61 (m, 1H), 7.59-7.53 (m, 1H), 7.20-7.10 (m, 3H), 5.86-5.83 (m, 1H), 5.62-5.49 (m, 2H), 4.93-4.89 (m, 1H), 4.33-4.28 (m, 1H), 3.59-3.46 (m, 2H), 0.92-0.86 (m, 2H), 0.03 - -0.06 (m, 9H).

***GENERAL PROCEDURE FOR PREPARATION OF COMPOUND 950-2***

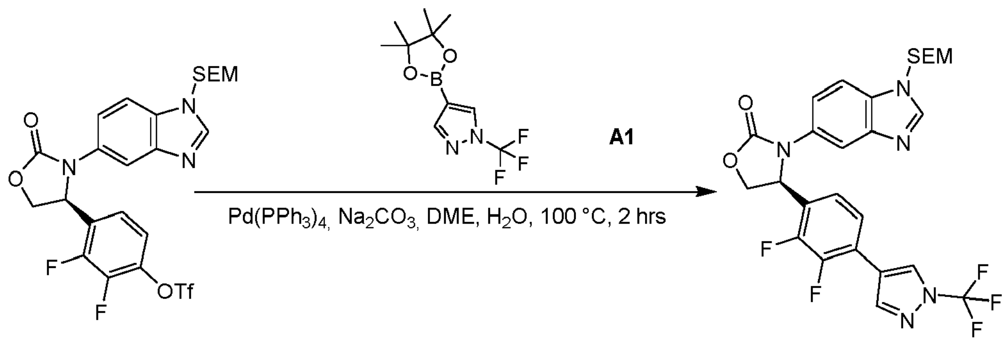


950-1 950-2

To a solution of compound **950-1** (150 mg, 253 μmol, 1.00 eq) and compound **A1** (70.0 mg, 267 μmol, 1.06 eq) in DME (5 mL) and $H_2O$ (1 mL) was added Pd $(PPh_3)_4$ (15.0 mg, 12.9 μmol, 0.05 eq) and $Na_2CO_3$ (60.0 mg, 566 μmol, 2.24 eq). The mixture was stirred at 100 °C for 2 hrs. The mixture was concentrated to give a residue. The crude product was purified by reversed-phase HPLC (0.1% FA condition in acetonitrile) to get compound **950-**2 (60.0 mg, 103 μmol, 40.9% yield) as a colorless solid. $^1H$ NMR (400 MHz $CDCl_3$) δ 8.16-8.04 (m, 1H), 7.97-7.87 (m, 1H), 7.72-7.70 (m, 1H), 7.64-7.62 (m, 1H), 7.52-7.49 (m, 1H), 7.19-7.17 (m, 1H), 7.15-7.13 (m, 2H), 5.90-5.83 (m, 1H), 5.53-5.46 (m, 2H), 4.93-4.89 (m, 1H), 4.36-4.32 (m, 1H), 3.56-3.45 (m, 2H), 0.90-0.86 (m, 2H), 0.02- -0.08 (m, 9H).

***GENERAL PROCEDURE FOR PREPARATION OF COMPOUND (2)***

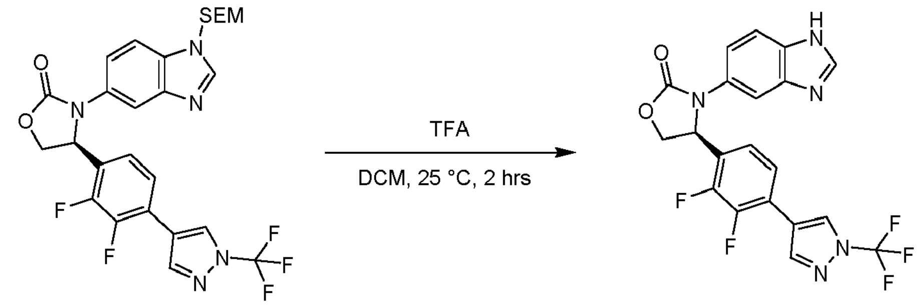


950-2 (2)

To a solution of compound **950-2** (50.0 mg, 86.3 μmol, 1.00 eq) in DCM (1 mL) was added TFA (1.54 g, 13.5 mmol, 1 mL, 156 eq). The mixture was stirred at 25 °C for 2 hrs. The mixture was diluted with DCM (10 mL) and adjusted to pH = 8 with NazCOs solution, the mixture was extracted with DCM (10 mL * 2). The combined organic layers were concentrated to give a residue. The residue was purified by prep-HPLC (basic condition, column: Phenomenex Gemini-NX C18 75 * 30mm * 3um; mobile phase: [water (0.05% ammonia hydroxide v/v)-ACN]; B%: 24%-54%, 7min) to get (2) (30.10 mg, 66.4 μmol, 77.0% yield, 99.2% purity) as a white solid.

**LCMS:** RT = 0.888 min, m/z = 450.1 $(M+1)^+$
**HPLC:** RT = 2.145 min
**SFC:** RT = 1.709 min

**$^1$H NMR** (400 MHz DMSO-d6) δ 8.88 (s, 1H), 8.37 (s, 1H), 8.21 (s, 1H), 7.65 (s, 1H), 7.58-7.51 (m, 2H), 7.30-7.27 (m, 2H), 6.04-6.01 (m, 1H), 4.92-4.88 (m, 1H), 4.40-4.36 (m, 1H).

***EXAMPLE 1.3 - SYNTHESIS OF COMPOUND (3)***

***SYNTHESIS OF COMPOUND 2***

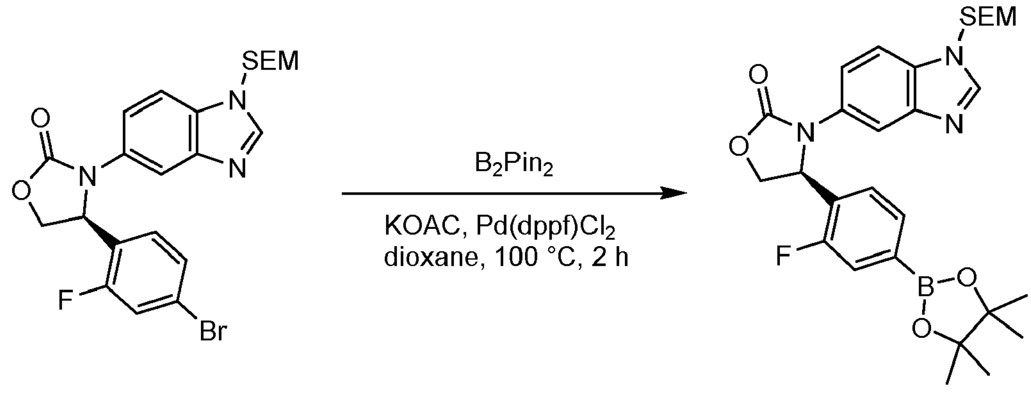


1 2

To a mixture of **compound 1** (500 mg, 987.29 μmol, 1 eq) and 4, 4, 5, 5 -tetramethyl-2 - (4, 4, 5, 5 -tetramethyl-1, 3, 2 -dioxaborolan- 2 -yl) - 1, 3, 2 -dioxaborolane (376.06 mg, 1.48 mmol, 1.5 eq) in dioxane (2 mL) was added KOAc (290.68 mg, 2.96 mmol, 3 eq), $Pd_2(dba)_3$ (90.41 mg, 98.73 μmol, 0.1 eq) and XPhos (94.13 mg, 197.46 μmol, 0.2 eq) under $N_2$. The mixture was stirred at 100 °C for 2 h under $N_2$. The reaction mixture was diluted with ethyl acetate (100 mL), the organic layer was washed with brine (50 mL), dried over $Na_2SO_4$, filtered and the filtrate was concentrated under vacuum to give a residue. The residue was purified by column chromatography (100 mesh silica gel, petroleum ether: ethyl acetate = 1: 0 to 1: 1) to give **compound 2** (310 mg, crude) as yellow gum.

***SYNTHESIS OF COMPOUND 6***

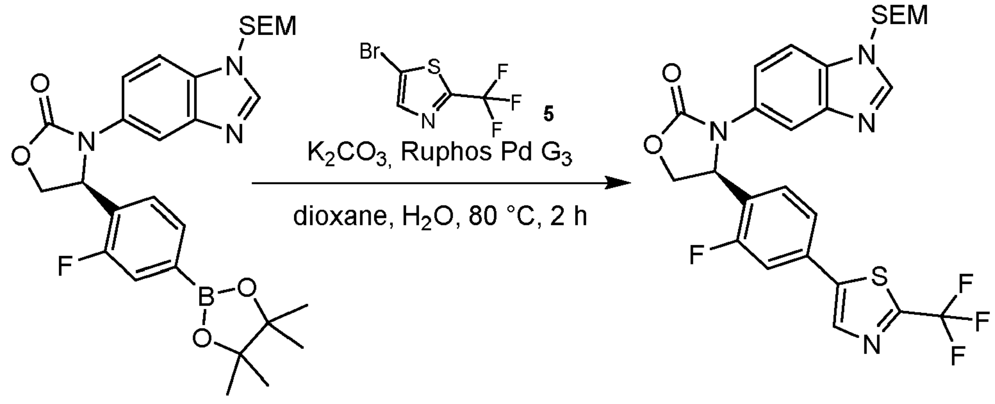


**2** **6**

To a mixture of **compound 2**(143.13 mg, 258.60 μmol, 1 eq) and **compound 5** (60 mg, 258.60 μmol, 1 eq) in dioxane (2 mL) and $H_2O$ (0.5 mL) was added $K_2CO_3$ (107.22 mg, 775.79 μmol, 3 eq) and RuPhos Pd Gs (21.63 mg, 25.86 μmol, 0.1 eq) under $N_2$. The mixture was stirred at 80 °C for 2 h under $N_2$. The reaction mixture was concentrated under vacuum to give a residue. The residue was purified by column chromatography (100 mesh silica gel, petroleum ether: ethyl acetate = 1: 0 to 1: 1) to give **compound 6** (70 mg, crude) as yellow gum.

***SYNTHESIS OF COMPOUND (4)***

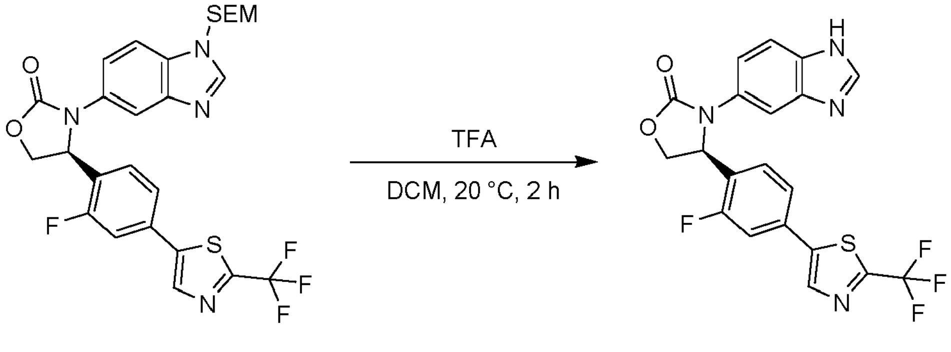


**6** **(4)**

To a solution of **compound 6**(70 mg, 120.97 μmol, 1 eq) in DCM (1 mL) was added TFA (1 mL), the mixture was stirred at 20 °C for 2 h. The reaction mixture was concentrated under vacuum to give a residue. The residue was diluted with ethyl acetate (30 mL), the organic layer was washed with saturated NaHCOs (20 mL*2) aqueous solution and brine (30 mL), dried over anhydrous sodium sulphate, filtered and concentrated under vacuum to give a residue. The residue was purified by prep-HPLC (colum: Phenomenex luna C18 150*25 mm* 10um;mobile phase: [water (FA) -ACN] ;B%: 15%- 45%, 10 min) to give (4) (20.96 mg, 44.41 μmol, 36.71% yield, 95% purity) as white solid.

$^{1}$**H NMR:** (400 MHz, METHANOL-$d_4$) δ = 8.28 (d, J = 1.0 Hz, 1H), 8.16 (s, 1H), 7.69 (d, J = 1.9 Hz, 1H), 7.60 - 7.47 (m, 4H), 7.36 (dd, J = 2.0, 8.8 Hz, 1H), 6.03 - 5.96 (m, 1H), 5.00 - 4.96 (m, 1H), 4.43 (dd, J = 5.9, 8.8 Hz, 1H)

**LCMS:** RT =0.462 min, m/z =448.9 $(M+H)^+$.
SFC: RT =1.528 min

*EXAMPLE 1.4 - SYNTHESIS OF COMPOUND (4)*

***SYNTHESIS OF COMPOUND 3***

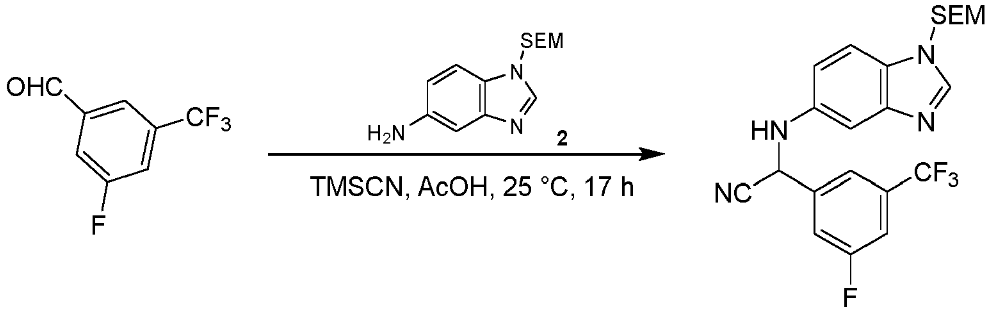


**1** **3**

To a solution of **compound 1** (1 g, 5.21 mmol, 1 eq) and **compound 2** (1.37 g, 5.21 mmol, 1 eq) in AcOH (10 mL) was added TMSCN (1.03 g, 10.41 mmol, 1.30 mL, 2 eq). The mixture was stirred at 25 °C for 17 h. The reaction mixture was diluted with water (10 mL). Then the pH of the mixture was adjusted to 6-7 with $NH_3$. $H_2O$. The resulting mixture was extracted with ethyl acetate (20 mL*3), the combined organic phase was dried with anhydrous $Na_2SO_4$, filtered and concentrated in vacuum to give residue. The residue was purified by silica gel chromatography (100-200 mesh silica gel, Petroleum ether/Ethyl acetate = 3/1, 1/1. TLC (Petroleum ether: Ethyl acetate = 1: 1; Rf = 0.3)) to give **compound 3** (2.2 g, 4.74 mmol, 90.98% yield) as brown oil. LCMS: RT = 0.542 min, m/z = 465.3 $(M+H)^+$.

***SYNTHESIS OF COMPOUND 4***

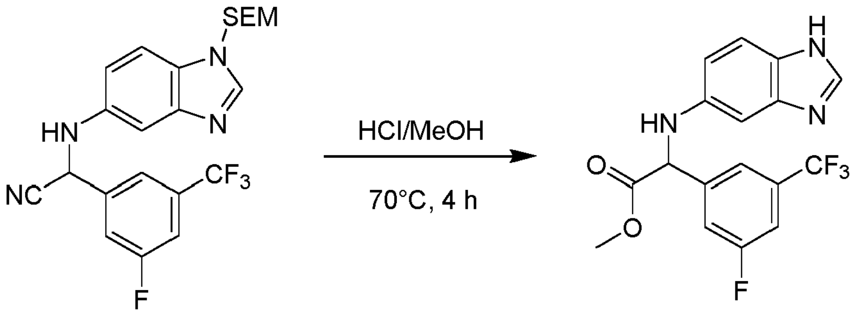


**3** **4**

A mixture of **compound 3** (2.2 g, 4.74 mmol, 1 *eq*) in HCl/MeOH (4 M, 12.56 mL) was stirred at 70 °C for 4 h. The reaction mixture was concentrated in vacuum to give **compound 4** (2 g, crude, HCl) as yellow solid.

***SYNTHESIS OF COMPOUND*** *5*

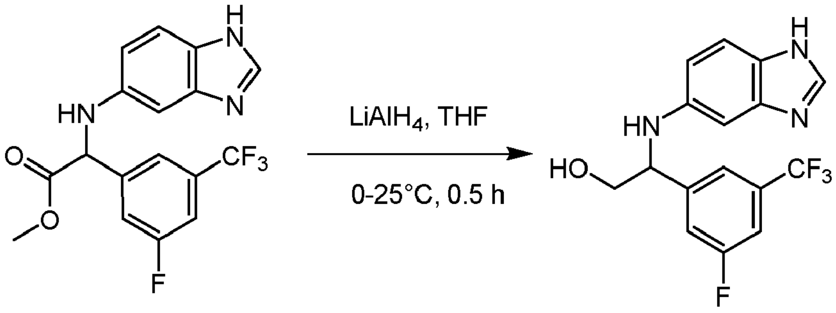


**4** **5**

To a solution of **compound 4** (2 g, 4.95 mmol, 1 eq, HCl) in THF (10 mL) was added $LiAlH_4$ (2 M, 12.38 mL, 5 *eq*) at 0 °C, the mixture was stirred at 25 °C for 0.5 h. The reaction mixture was added into saturated $NH_4Cl$ solution (50 mL) and the resulting mixture was extracted with ethyl acetate (50 mL*3) and the combined organic phase was washed with NaCl saturated solution (50 mL), dried with anhydrous $Na_2SO_4$, filtered and concentrated in vacuum to give **compound 5** (1.3 g, 3.83 mmol, 77.35% yield) as yellow solid. LCMS: RT = 0.410 min, m/z = 340.2 $(M+H)^+$.

***SYNTHESIS OF COMPOUND (4), (4)_PEAK1 AND (4)_PEAK2***

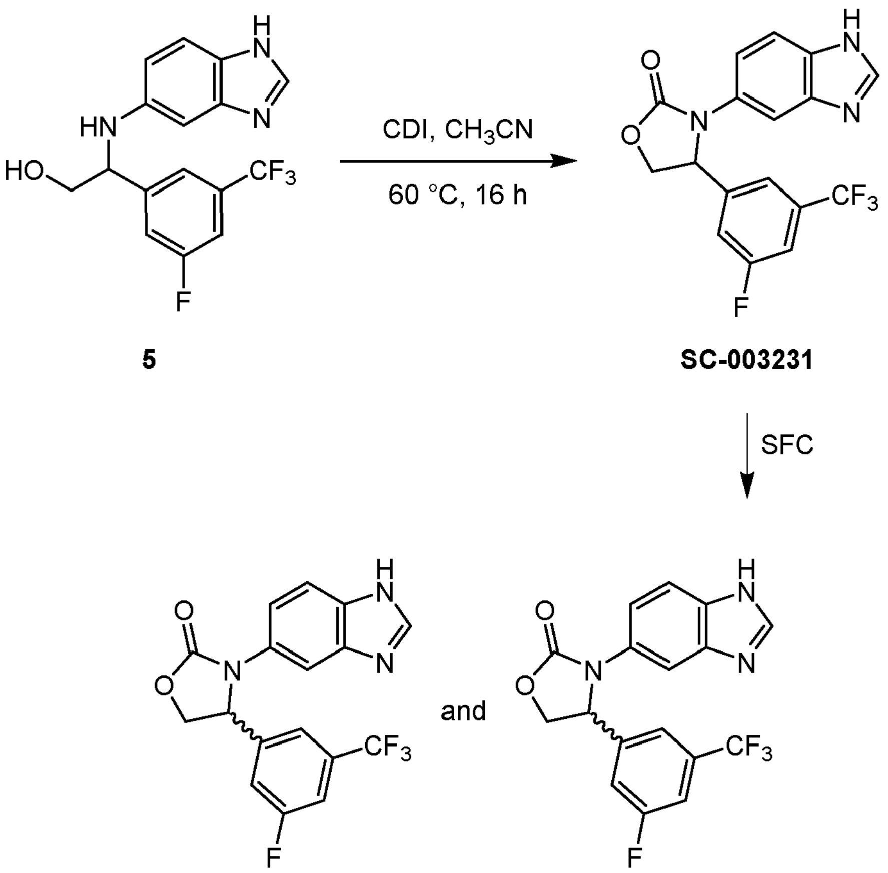


**5** **SC-003231**

and

**(4)_peak1** **(4)_peak2**

To a solution of **compound 5** (1.2 g, 3.54 mmol, 1 eq) in ACN (12 mL) was added CDI (1.15 g, 7.07 mmol, 2 eq). The mixture was stirred at 60 °C for 16 h. The mixture was diluted with water (50 mL) and extracted with EtOAc (50 mL*3). The organic phase was washed with brine (50 mL), dried over $Na_2SO_4$, filtered and concentrated at reduced pressure to give a residue. The residue was purified by prep-HPLC (column: Phenomenex luna C18 150*40 mm* 15um;

mobile phase: [water (FA) -ACN]; gradient: 10%-40% B over 11 min) to give (4) (452.83 mg, 1.10 mmol, 31.13% yield, FA) as off-white solid.

**$^1$H NMR:** (400 MHz, $CD_3OD$) δ = 8.21 (s, 1H), 8.11 (s, 1H), 7.66 (d, *J* = 2 Hz, 1H), 7.61 - 7.54 (m, 2H), 7.50 (br d, *J* = 9.2 Hz, 1H), 7.42 - 7.33 (m, 2H), 5.91 - 5.77 (m, 1H), 4.95 - 4.93 (m, 1H), 4.36 - 4.22 (m, 1H)
**LCMS:** RT = 0.422 min, m/z = 366.1 $(M+H)^+$.

100 mg of **(4)** was purified by SFC (Rt = 1.561 min and Rt = 1.686 min; column: DAICEL CHIRALPAK AD (250 mm*30 mm, 10um); mobile phase: [$CO_2$-EtOH (0.1%$NH_3H_2O$)]; B%: 20%, isocratic elution mode)

To give **(4)_peak1** (40.15 mg, 108.82 μmol, 99% purity) as off-white solid.

**$^1$H NMR:** (400 MHz, $CD_3OD$) δ = 8.35 (s, 1H), 7.71 (d, J = 2 Hz, 1H), 7.62 - 7.55 (m, 2H), 7.50 (br d, J = 9.6 Hz, 1H), 7.45 - 7.35 (m, 2H), 5.90 - 5.79 (m, 1H), 4.93 (s, 1H), 4.35 - 4.26 (m, 1H)

**LCMS:** RT = 0.420 min, m/z = 366.1 $(M+H)^+$.
**SFC:** RT = 1.559 min.

To give **(4)_peak2** (42.17 mg, 115.45 μmol, 100% purity) as off-white solid.

**$^1$H NMR:** (400 MHz, $CD_3OD$) δ = 8.32 (s, 1H), 7.70 (d, *J* = 2 Hz, 1H), 7.58 (d, *J* = 8.4 Hz, 2H), 7.50 (br d, *J* = 9.2 Hz, 1H), 7.42 - 7.35 (m, 2H), 5.90 - 5.79 (m, 1H), 4.93 (s, 1H), 4.35 - 4.27 (m, 1H)

**LCMS:** RT = 0.425 min, m/z = 366.1 $(M+H)^+$.
**SFC:** RT = 1.683 min.

***EXAMPLE 1.5 - SYNTHESIS OF COMPOUND (5)***

***SYNTHESIS OF COMPOUND 2***

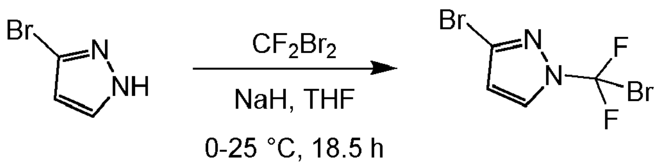


1 2

To a solution of **compound 1** (2 g, 13.61 mmol, 1 eq) in THF (25 mL) was added NaH (653.12 mg, 16.33 mmol, 60% purity, 1.2 eq) at 0 °C. The mixture was stirred at 0 °C for 30 min. Then to the mixture was added dibromo (difluoro) methane (3.43 g, 16.33 mmol, 1.51 mL, 1.2 *eq*) and stirred at 25 °C for 18 hr. The reaction mixture was quenched with saturated $NH_4Cl$ aqueous solution (50 mL), extracted with EtOAc (20 mL*3). The organic phase was separated, washed with brine, dried over anhydrous $Na_2SO_4$, filtered and concentrted under reduced pressure to give a residue. The residue was purified by flash silica gel chromatography ((100-200 mesh silica gel, TLC (Petroleum ether: Ethyl acetate = 5: 1; Rf = 0.7), ISCO®; 20 g SepaFlash® Silica Flash Column, Eluent of 0-0% Ethylacetate/Petroleum ethergradient @ 50 mL/min) to give **compound 2** (3 g, 10.87 mmol, 79.91% yield) as colorless oil. The compound was a mixture of 3-bromo-1-(bromodifluoromethyl)-1H-pyrazole and 5-bromo-1-(bromodifluoromethyl)-1H-pyrazole. $^1$H NMR: (400 MHz, CHLOROFORM-d) δ = 7.74 (d, J = 2.8 Hz, 1H), 6.49 (d, J = 2.8 Hz, 1H)

***SYNTHESIS OF COMPOUND 3***

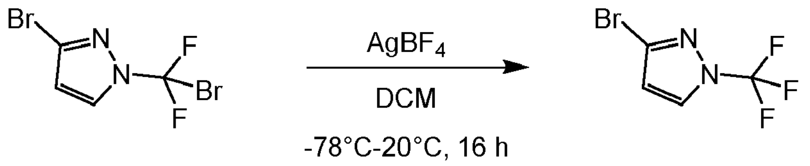


**2** **3**

To a solution of **compound 2** (1 g, 3.62 mmol, 1 *eq*) in DCM (5 mL) was added $AgBF_4$ (1.41 g, 7.25 mmol, 2 *eq*) at -78 °C under $N_2$ atmosphere. The mixture was stirred at 20 °C for 16 hr. The reaction mixture was filtered and washed with saturated NaHCOs aqueous solution (10 mL), the organic phase was separated, washed with brine (5 mL), dried over anhydrous $Na_2SO_4$ **Compound 3** (770 mg, crude) in DCM (5 mL) used directly after work up was obtained as a yellow oil. The compound was a mixture of 3-bromo-1-(trifluoromethyl)-1H-pyrazole and 5-bromo-1-(trifluoromethyl)-1H-pyrazole. $^1H$ NMR (400 MHz, CHLOROFORM-d) $\delta$ = 7.78 (d, J = 2.0 Hz, 1H), 6.55 (br d, J = 2.0 Hz, 1H). (HNMR of the major isomer)

***SYNTHESIS OF COMPOUND 7***

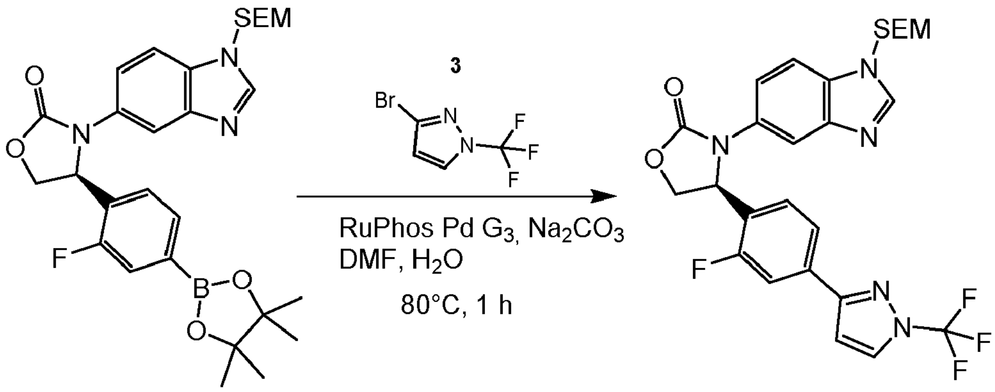


**5** **7**

A mixture of **compound 5** (200 mg, 361.33 μmol, 1 *eq*), **compound 3** (77.68 mg, 361.33 μmol, 1 *eq*), RuPhos Pd Gs (30.22 mg, 36.13 μmol, 0.1 *eq*), NazCOs (76.60 mg, 722.67 μmol, 2 *eq*) and in DMF (3 mL) and $H_2O$ (1 mL) was stirred at 80 °C for 1 hr under $N_2$ atmosphere. The reaction mixture was diluted with brine (20 mL). The aqueous layer was extracted with ethyl acetate (20 mL*3). The combined organic layers dried over anhydrous $Na_2SO_4$, filtered and concentrated under reduced pressure to give a residue. The residue was purified by flash silica gel chromatography ((100-200 mesh silica gel, TLC (Petroleum ether: Ethyl acetate = 1: 2; Rf = 0.05), ISCO®; 12 g SepaFlash® Silica Flash Column, Eluent of 0-88% Ethyl acetate/Petroleum ethergradient @ 40 mL/min) to give **compound 7** (145 mg, 258.19 μmol, 71.45% yield) was obtained as a yellow oil.

***SYNTHESIS OF COMPOUND (5)***

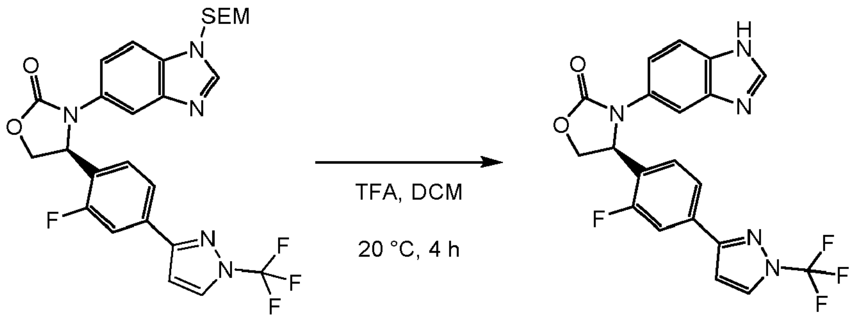


**7** **(5)**

To a solution of **compound 7** (145 mg, 258.19 μmol, 1 *eq*) in DCM (1 mL) was added TFA (1 mL). The mixture was stirred at 20 °C for 4 h. The reaction mixture was concentrated under reduced pressure to give a residue. The residue was diluted with EtOAc (10 mL) and washed with aq.NaHCOs (10 mL), the combined organic layers dried with anhydrous $Na_2SO_4$, filtered and concentrated in vacuum to give a residue. The residue was purified by prep-HPLC (column: Phenomenex Luna C18 150*25 mm*10um; mobile phase: [water (TFA) -ACN]; gradient: 20%-50% B over 9 min) to give **(5)** (crude).

The (5) (crude) was purified by prep-HPLC (column: Phenomenex Luna C18 150*25 mm* 10um; mobile phase: [water (FA) -ACN]; gradient: 15%-45% B over 10 min) to give **(5)** (4.31 mg, 9.69 μmol, 3.75% yield, 97% purity) as a white solid.

**$^1$H NMR:** (400 MHz, $CD_3OD$) δ = 8.19 (d, J = 2.8 Hz, 1H), 8.14 (s, 1H), 7.70 - 7.62 (m, 3H), 7.54 (d, J = 8.8 Hz, 1H), 7.48 (t, J = 8.0 Hz, 1H), 7.35 (dd, J = 2.0, 8.8 Hz, 1H), 6.93 (d, J = 2.8 Hz, 1H), 5.96 (dd, J = 5.6, 9.2 Hz, 1H), 4.96 (t, J = 9.2 Hz, 1H), 4.43 (dd, J = 5.6, 8.8 Hz, 1H)
**LCMS:** RT = 0.444 min, m/z = 432.1 $(M+H)^+$.

***EXAMPLE 1.6 - SYNTHESIS OF COMPOUND (6)***

***SYNTHESIS OF COMPOUND 2***

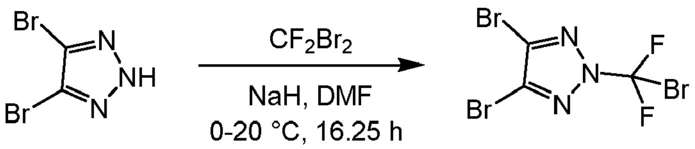


**1** **2**

To a solution of **compound 1** (10.22 g, 45.05 mmol, 1 eq) in DMF (50 mL) was added NaH (1.98 g, 49.56 mmol, 60% purity, 1.1 *eq*) slowly at 0 °C under, after the addition, the mixture was stirred at 0 °C for 0.25 h, then dibromo (difluoro) methane (10.40 g, 49.56 mmol, 4.58 mL, 1.1 eq) was added slowly at 0 °C, the mixture was stirred at 20 °C for 16 h. The mixture was added into saturated $NH_4Cl$ aqueous solution (200 mL) slowly at 0 °C with stirring, the resulting was extracted with Petroleum ether (200 mL*2). The combined organic layer was washed with brine (200 mL*2), dried over $Na_2SO_4$, filtered and concentrated at reduced pressure to give a residue. The residue was purified by silica gel chromatography (1000 mesh silica gel, Petroleum ether/Ethyl acetate = 0/1. TLC (Petroleum ether: Ethyl acetate = 10: 1; Rf = 0.77)) to give **compound 2** (300 mg, 843.26 μmol, 1.87% yield) as colorless oil. $^{13}$C NMR: (100 MHz, $CDCl_3$) δ 130.6 108.7 (t, *J* = 310 Hz), $^{19}$F NMR: (100 MHz, $CDCl_3$) δ -35.4

***SYNTHESIS OF COMPOUND 3***

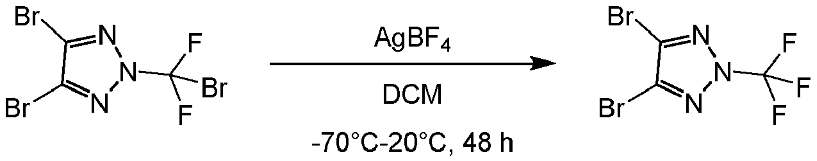


**2** **3**

To a solution of **compound 2** (300 mg, 843.26 µmol, 1 *eq*) in DCM (5 mL) was added $AgBF_4$ (656.64 mg, 3.37 mmol, 4 *eq*) at -70 °C under $N_2$, the mixture was stirred at 20 °C for 48 h under $N_2$ atmosphere. The reaction mixture was washed with saturated NaHCOs aqueous solution (5 mL*3), dried with anhydrous $Na_2SO_4$, filtered and concentrated in vacuum to give **compound 3** (65 mg, crude) as yellow oil. $^{19}F$ NMR: (100 MHz, $CDCl_3$) δ - 62.3

### *SYNTHESIS OF COMPOUND 5*

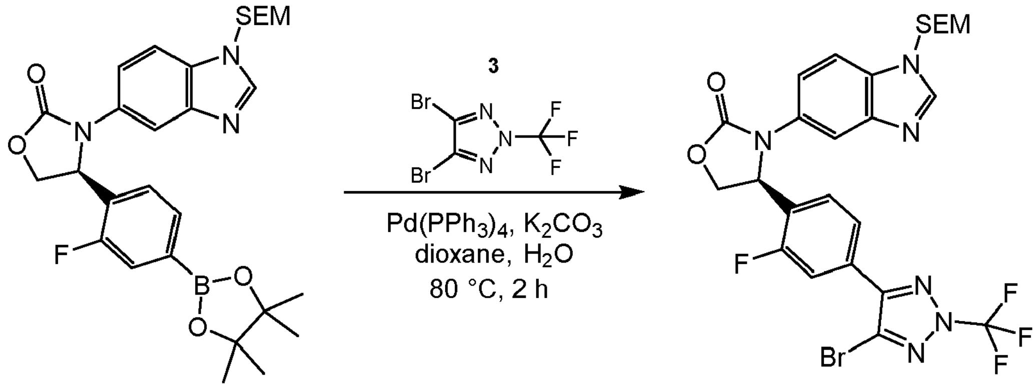


**4** **5**

To a mixture of **compound 3** (21.31 mg, 72.27 µmol, 2 *eq*), **compound 4** (20 mg, 36.13 µmol, 1 *eq*) and $K_2CO_3$ (9.99 mg, 72.27 µmol, 2 *eq*) in $H_2O$ (0.025 mL) and dioxane (0.2 mL) was added $Pd(PPh_3)_4$ (8.35 mg, 7.23 µmol, 0.2 *eq*) under $N_2$. The mixture was stirred at 80 °C for 2 h. The reaction mixture was concentrated in vacuum to give residue.

To a mixture of **compound 3** (42.62 mg, 144.53 µmol, 2 *eq*), **compound 4** (40 mg, 72.27 µmol, 1 *eq*) and $K_2CO_3$ (19.98 mg, 144.53 µmol, 2 *eq*) in $H_2O$ (0.5 mL) and dioxane (1 mL) was added $Pd(PPh_3)_4$ (16.70 mg, 14.45 µmol, 0.2 *eq*) under $N_2$. The mixture was stirred at 80 °C for 2 h. The reaction mixture was concentrated in vacuum to give residue.

The combined residue was purified by prep-TLC (petroleum ether: ethyl acetate = 1/2) to give **compound 5** (40 mg, crude) as colorless oil.

### *SYNTHESIS OF COMPOUND 6*

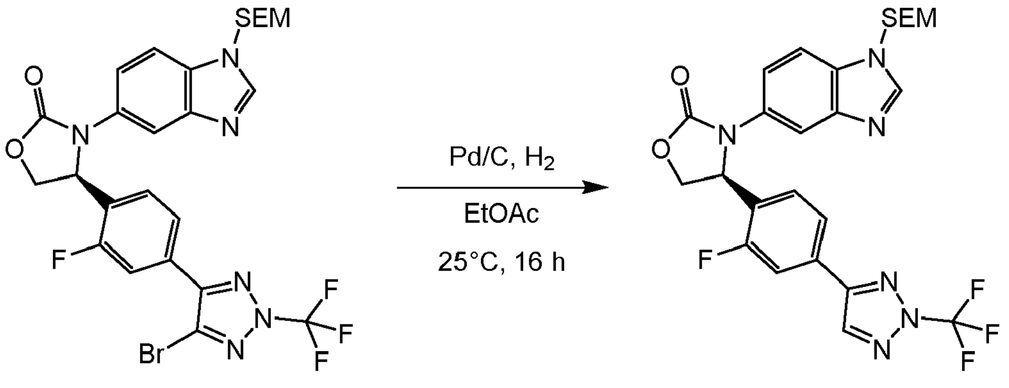


5 6

To a solution of **compound 5** (30 mg, 46.77 μmol, 1 *eq*) in EtOAc (5 mL) was added Pd/C (30.00 mg, 28.19 μmol, 10% purity, 6.03e-1 *eq*) under $N_2$ atmosphere. The suspension was degassed and purged with $H_2$ for 3 times. The mixture was stirred under $H_2$ (15 Psi) at 25 °C for 16 h. The mixture was filtered and concentrated at reduced pressure to give **compound 6** (30 mg, crude) as colorless oil.

***SYNTHESIS OF COMPOUND (6)***

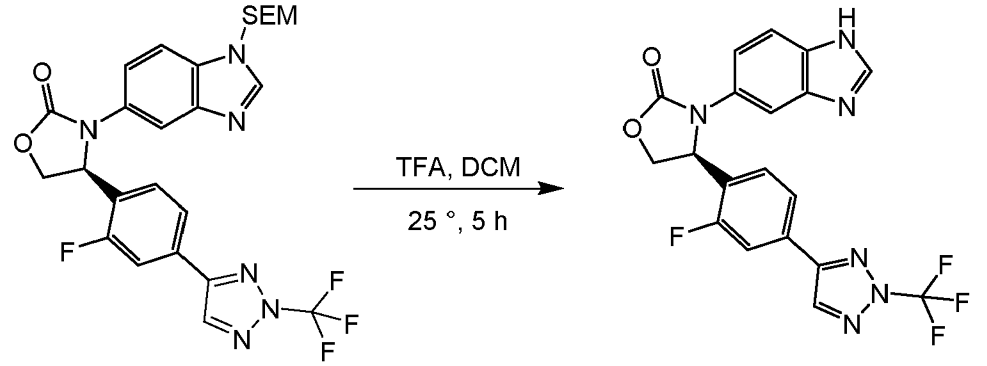


6 **(6)**

To a mixture of **compound 6** (30 mg, 53.32 μmol, 1 *eq*) in $CH_2Cl_2$ (1 mL) was added TFA (0.5 mL). The mixture was stirred at 25 °C for 5 h. The reaction mixture was diluted with water (10 mL) and extracted with ethyl acetate (10 mL*3) and the combined organic phase was washed with saturated NaHCOs solution (10 mL*3), dried with anhydrous $Na_2SO_4$, filtered and concentrated in vacuum to give residue. The residue was purified by prep-HPLC (column: Welch Xtimate C18 150*25 mm*5um; mobile phase: [water (FA) - ACN]; gradient: 20%-40% B over 10 min) to give **(6)** (4.2 mg, 9.71 μmol, 18.22% yield) as white solid.

**$^1$H NMR:** (400 MHz, $CD_3OD$) δ = 8.47 (s, 1H), 8.18 (s, 1H), 7.72 (t, *J* = 10 Hz, 3H), 7.61 - 7.48 (m, 2H), 7.41 - 7.33 (m, 1H), 6.08 - 5.91 (m, 1H), 4.97 (t, *J* = 8.8 Hz, 1H), 4.49 - 4.40 (m, 1H)

**LCMS:** RT = 0.458 min, m/z = 433.2 $(M+H)^+$.
**SFC:** RT =1.925 min.

***EXAMPLE 1.7- SYNTHESIS OF COMPOUND (7)***

***SYNTHESIS OF COMPOUND 2***

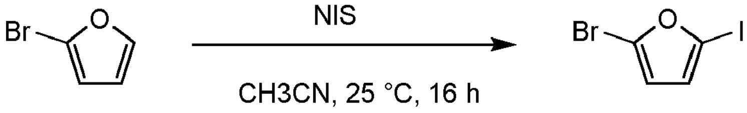


1 2

A mixture of **compound 1** (1 g, 6.80 mmol, 1 *eq*), NIS (1.53 g, 6.80 mmol, 1 *eq*) in ACN (10 mL) and the mixture was stirred at 25 °C for 16 h. The reaction mixture was diluted with water (50 mL) and extracted with ethyl acetate (50 mL*3). The combined organic phase was washed with brine (50 mL*3), dried with anhydrous $Na_2SO_4$, filtered and concentrated in vacuum to give **compound 2** (1 g, crude) as brown oil.

***SYNTHESIS OF COMPOUND 3***

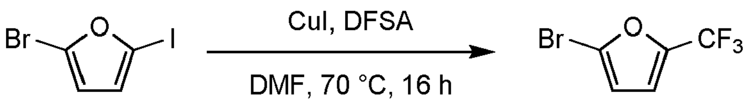


2 3

To a solution of **compound 2** (1 g, 3.66 mmol, 1 eq) in DMF (10 mL) was added CuI (1.05 g, 5.50 mmol, 1.5 *eq*) and methyl 2, 2-difluoro-2-fluorosulfonyl-acetate (1.06 g, 5.53 mmol, 704.05 μL, 1.51 *eq*). The mixture was stirred at 70 °C for 16 hr. The reaction mixture was diluted with water (100 mL) and extracted with Petroleum ether (50 mL*3) and the combined organic phase was washed with brine (50 mL*3), dried with anhydrous $Na_2SO_4$, filtered and concentrated in vacuum to give residue. The residue was purified by silica gel chromatography (100-200 mesh silica gel, Petroleum ether/Ethyl acetate = 1/0, Petroleum ether: Ethyl acetate = 1: 0, Rt = 0.57) to give **compound 3** (5 g, crude, 1 % in petroleum ether) as colorless liquid. $^{19}F$ NMR (400 MHz, $CDCl_3$) δ = -64.60,

***SYNTHESIS OF COMPOUND 5***

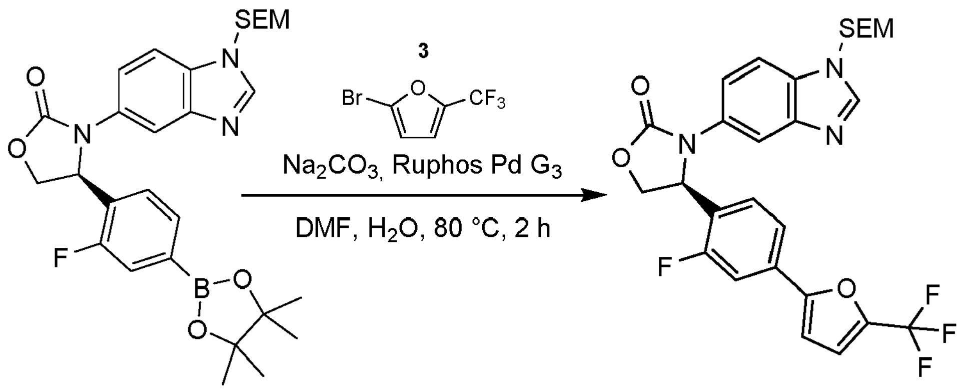


4 5

To a mixture of **compound 3** (5 g, crude, 1 % in petroleum ether), **compound 4** (100 mg, 180.67 μmol, 1 eq) and $Na_2CO_3$ (38.30 mg, 361.33 μmol, 2 eq) in DMF (3 mL) and $H_2O$ (0.5 mL) was added RuPhos Pd Gs (15.11 mg, 18.07 μmol, 0.1 *eq*) under $N_2$. The mixture was stirred at 80 °C for 1 h. The reaction mixture was diluted with water (10 mL) and extracted with ethyl acetate (10 mL*3), the combined organic phase was dried with anhydrous $Na_2SO_4$, filtered and concentrated in vacuum to give residue. The residue was purified by silica gel chromatography (100-200 mesh silica gel, Petroleum ether/Ethyl acetate = 3/1, 1/1. TLC (Petroleum ether: Ethyl acetate = 1: 1; Rf = 0.43)) to give **compound 5** (60 mg, crude) as brown oil.

*SYNTHESIS OF COMPOUND (7)*

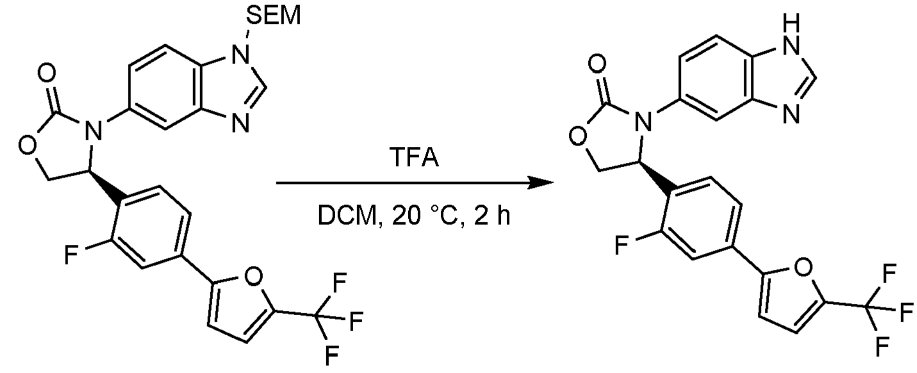


**5** **(7)**

To a solution of **compound 5** (60 mg, 106.84 μmol, 1 eq) in DCM (5 mL) was added TFA (1.25 mL). The mixture was stirred at 25 °C for 0.5 h. The reaction mixture was diluted with water (20 mL) and extracted with ethyl acetate (20 mL*3) and the combined organic phase was washed with saturated NaHCOs solution (20 mL*3), dried with anhydrous $Na_2SO_4$, filtered and concentrated in vacuum to give residue. The residue was purified by prep-HPLC (column: Phenomenex luna C18 150*25 mm* 10um; mobile phase: [water (FA) -ACN]; gradient: 18%-48% B over 10 min) to give **(7)** (3.52 mg, 8.16 μmol, 7.64% yield) as white solid.

**$^1$H NMR:** (400 MHz, $CD_3OD$) δ = 8.13 (s, 1H), 7.66 (d, *J* = 2 Hz, 1H), 7.55 - 7.46 (m, 4H), 7.34 (br d, *J* = 8.8 Hz, 1H), 7.09 - 7.00 (m, 1H), 6.97 (d, *J* = 3.6 Hz, 1H), 6.02 - 5.89 (m, 1H), 4.95 (t, *J* = 8.8 Hz, 1H), 4.48 - 4.39 (m, 1H)

**LCMS:** RT = 0.476 min, m/z = 432.1 $(M+H)^+$.
**SFC:** RT = 2.006 min.

*EXAMPLE 1.8 - SYNTHESIS OF COMPOUND (8)*

*SYNTHESIS OF COMPOUND 3*

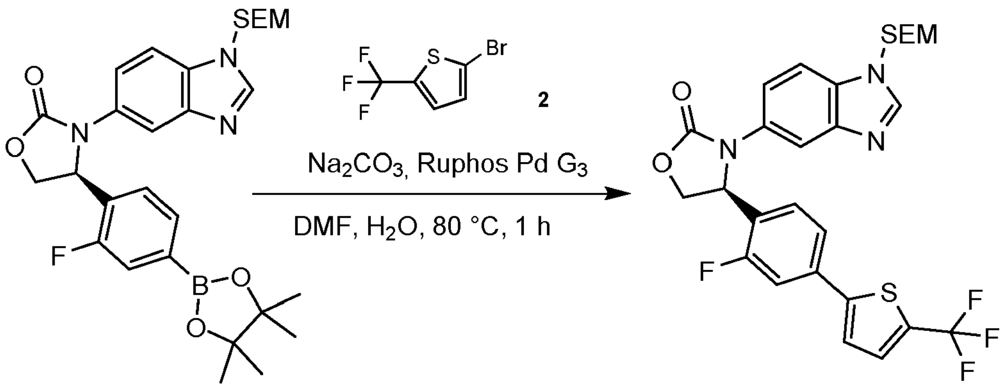


**1** **3**

To a mixture of **compound 2** (22.96 mg, 99.37 μmol, 1.1 *eq*), **compound 1** (50 mg, 90.33 μmol, 1 *eq*) and $Na_2CO_3$ (19.15 mg, 180.67 μmol, 2 *eq*) in $H_2O$ (0.3 mL) and DMF (1.5 mL) was added RuPhos Pd Gs (7.56 mg, 9.03 μmol, 0.1 eq) under $N_2$. The mixture was stirred at 80 °C for 1 h. The reaction mixture was diluted with water (10 mL) and extracted with ethyl acetate (10 mL*3), the combined organic phase was dried with anhydrous $Na_2SO_4$, filtered and concentrated in vacuum to give residue. The residue was purified by silica gel chromatography (100-200 mesh silica

gel, Petroleum ether/Ethyl acetate = 3/1, 1/1. TLC (Petroleum ether: Ethyl acetate = 1: 1; Rf = 0.43)) to give **compound 3** (50 mg, crude) as brown oil.

*SYNTHESIS OF COMPOUND (8)*

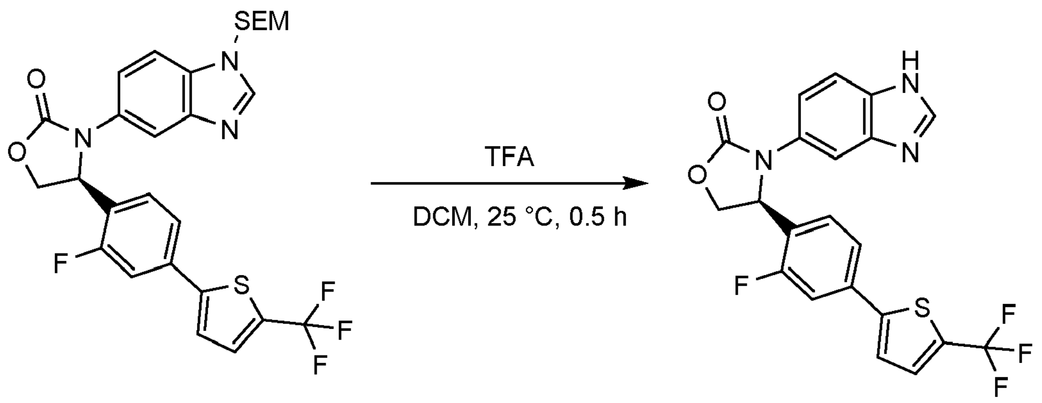


**3** **(8)**

To a solution of **compound 3** (50 mg, 86.56 μmol, 1 eq) in DCM (5 mL) was added TFA (1.25 mL) The mixture was stirred at 25 °C for 0.5 h. The reaction mixture was diluted with water (20 mL) and extracted with ethyl acetate (20 mL*3). The combined organic phase was washed with saturated NaHCOs solution (20 mL*3), dried with anhydrous $Na_2SO_4$, filtered and concentrated in vacuum to give residue. The residue was purified by prep-HPLC (column: Phenomenex luna C18 150*25 mm* 10um; mobile phase: [water (FA) - ACN]; gradient: 23%-53% B over 10 min) to give **(8)** (3.18 mg, 7.11 μmol, 8.21% yield) as off-white solid.

**$^1$H NMR:** (400 MHz, $CD_3OD$) δ = 8.18 (s, 1H), 7.69 (d, *J* = 1.6 Hz, 1H), 7.55 (d, *J* = 8.8 Hz, 1H), 7.52 - 7.43 (m, 5H), 7.39 - 7.33 (m, 1H), 6.01 - 5.90 (m, 1H), 4.96 (t, *J* = 8.8 Hz, 1H), 4.48 - 4.34 (m, 1H)

**LCMS:** RT = 0.484 min, m/z = 448.2 $(M+H)^+$.
**SFC:** RT = 0.845 min.

*EXAMPLE 1.9 - SYNTHESIS OF COMPOUND (9)*

*SYNTHESIS OF COMPOUND 3*

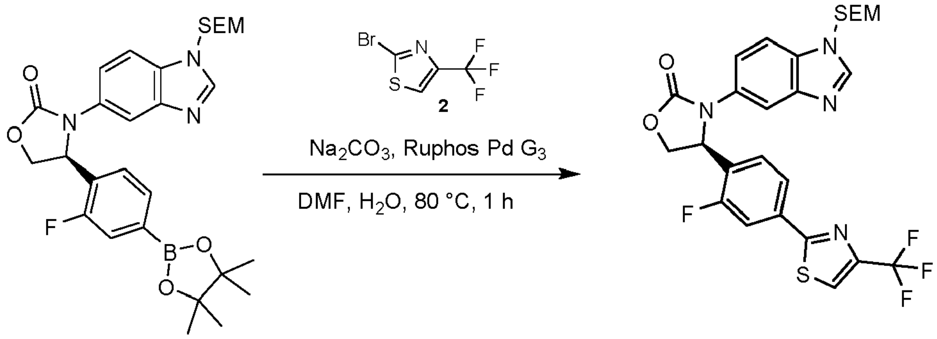


**1** **3**

To a mixture of **compound 2** (23.06 mg, 99.37 μmol, 1.1 *eq*), **compound 1**(50 mg, 90.33 μmol, 1 *eq*) and $Na_2CO_3$ (19.15 mg, 180.67 μmol, 2 *eq*) in $H_2O$ (0.3 mL) and DMF (1.5 mL) was added RuPhos Pd Gs (7.56 mg, 9.03 μmol, 0.1 *eq*) under $N_2$. The mixture was stirred at 80 °C for 1 h. The reaction mixture was diluted with water (10 mL)

and extracted with ethyl acetate (10 mL*3), the combined organic phase was dried with anhydrous $Na_2SO_4$, filtered and concentrated in vacuum to give residue. The residue was purified by silica gel chromatography (100-200 mesh silica gel, Petroleum ether/Ethyl acetate = 3/1, 1/1. TLC (Petroleum ether: Ethyl acetate = 1: 1) to give **compound 3** (50 mg, crude) as brown oil.

***SYNTHESIS OF COMPOUND (9)***

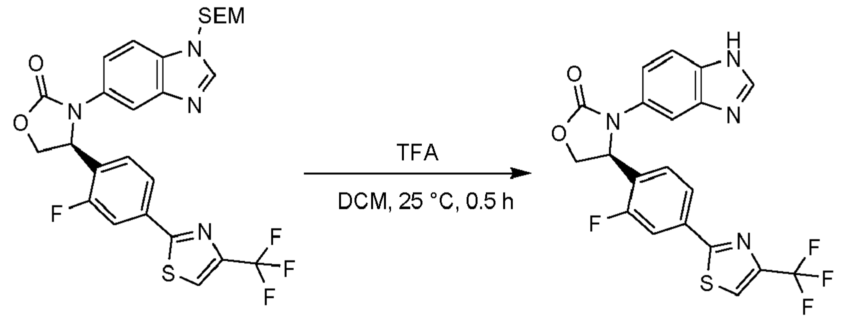


3 (9)

To a mixture of **compound 3** (50 mg, 86.41 μmol, 1 *eq*) in DCM (5 mL) was added TFA (1.25 mL) The mixture was stirred at 25 °C for 0.5 h. The reaction mixture was diluted with water (20 mL) and extracted with ethyl acetate (20 mL*3) and the combined organic phase was washed with saturated NaHCOs solution (20 mL*3), dried with anhydrous $Na_2SO_4$, filtered and concentrated in vacuum to give residue. The residue was purified by prep-HPLC (column: Phenomenex luna C18 150*25 mm* 10um; mobile phase: [water (FA) - ACN]; gradient: 15%-45% B over 10 min) to give **(9)** (3.75 mg, 8.36 μmol, 9.68% yield, 100% purity) as off-white solid.

**$^1$H NMR:** (400 MHz, $CD_3OD$) δ 8.20 (s, 1H), 8.14 (s, 1H), 7.82 - 7.73 (m, 2H), 7.68 (d, *J* = 2 Hz, 1H), 7.60 - 7.50 (m, 2H), 7.39 - 7.33 (m, 1H), 6.05 - 5.92 (m, 1H), 4.97 (t, *J* = 9.2 Hz, 1H), 4.48 - 4.40 (m, 1H)

**$^{19}$F NMR:** (400 MHz, $CD_3OD$) δ 61.6, 115.2
**LCMS:** RT = 0.451 min, m/z = 449.2 $(M+H)^+$.
**SFC:** RT = 1.756 min.

***EXAMPLE 1.10 - SYNTHESIS OF COMPOUND (10)***

***SYNTHESIS OF COMPOUND 2***

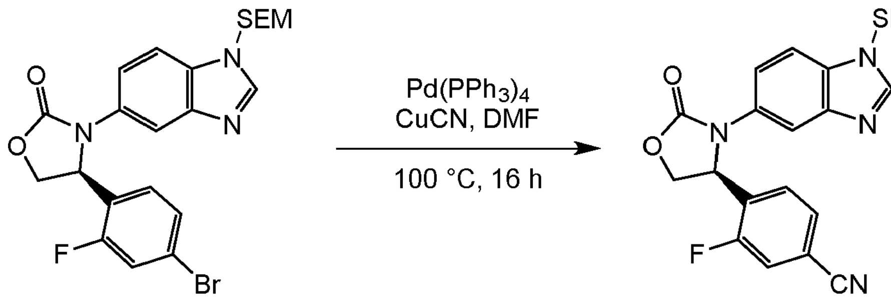


1 2

To a solution of **compound 1** (300 mg, 592.37 μmol, 1 *eq*) in DMF (3 mL) was added CuCN (159.16 mg, 1.78 mmol, 388.21 μL, 3 eq) and $Pd(PPh_3)_4$ (136.90 mg, 118.47 μmol, 0.2 *eq*) under $N_2$. The mixture was stirred at 100 °C for 16 h. The reaction mixture was diluted with water (20 mL) and extracted with ethyl acetate (20 mL*3) and the combined

organic phase was washed with saturated NaHCOs solution (20 mL*3), dried with anhydrous $Na_2SO_4$, filtered and concentrated in vacuum to give residue. The residue was purified by silica gel chromatography (100-200 mesh silica gel, Petroleum ether/Ethyl acetate = 5/1, 0/1. TLC (Petroleum ether: Ethyl acetate = 0: 1; Rf = 0.43)) to give **compound 2** (150 mg, 331.45 μmol, 55.95% yield) as brown oil. LCMS: RT = 0.515 min, m/z = 453.3 $(M+H)^+$.

*SYNTHESIS OF COMPOUND 3*

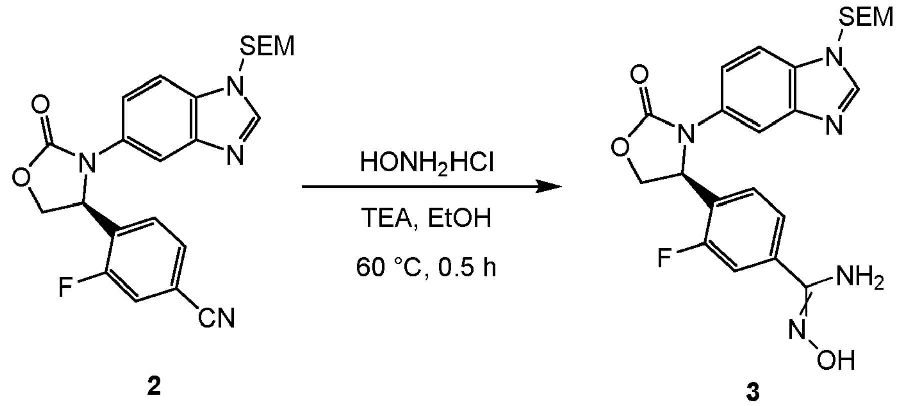


**2** **3**

To a solution of **compound 2** (150 mg, 331.45 μmol, 1 *eq*) in MeOH (1.5 mL) was added hydroxylamine hydrochloride (46.07 mg, 662.91 μmol, 2 *eq*) and TEA (67.08 mg, 662.91 μmol, 92.27 μL, 2 *eq*). The mixture was stirred at 60 °C for 0.5 hr. The reaction mixture was diluted with water (10 mL) and extracted with ethyl acetate (10 mL*3) and the combined organic phase was washed with brine (10 mL*3), dried with anhydrous $Na_2SO_4$, filtered and concentrated in vacuum to give residue. The residue was purified by silica gel chromatography (100-200 mesh silica gel, Petroleum ether/Ethyl acetate = 1/1, 0/1. TLC (Petroleum ether: Ethyl acetate = 0: 1; Rf = 0.19)) to give **compound 3** (50 mg, 102.97 μmol, 31.07% yield) as colorless oil.

*SYNTHESIS OF COMPOUND (10)*

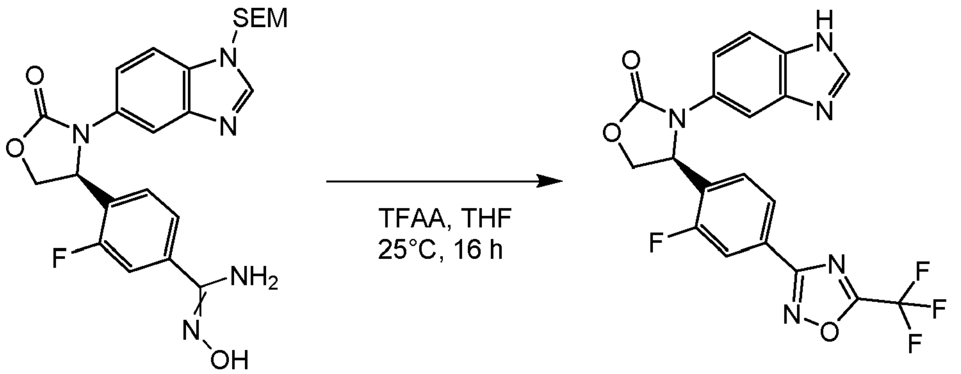


**3** **(10)**

To a solution of **compound 3** (50 mg, 102.97 μmol, 1 *eq*) in THF (0.5 mL) was added TFAA (64.88 mg, 308.91 μmol, 42.94 μL, 3 *eq*). The mixture was stirred at 25 °C for 16 hr. The reaction mixture was diluted with $H_2O$ (20 mL). Then the pH of the mixture was adjusted to 6-7 with NaHCOs. The resulting mixture was extracted with ethyl acetate (30 mL*3), the combined organic phase was dried with anhydrous $Na_2SO_4$, filtered and concentrated in vacuum to give residue. The reaction mixture was purified by prep-HPLC (column: Phenomenex luna C18 150*25 mm* 10um; mobile phase: [water (FA) -ACN]; gradient: 15%-45% B over 10 min) to give **(10)** (10.52 mg, 23.04 μmol, 22.38% yield, 94.9% purity) as white solid.

**$^1$H NMR:** (400 MHz, $CD_3OD$) δ = 8.27 - 8.12 (m, 1H), 7.93 - 7.82 (m, 2H), 7.71 (br s, 1H), 7.64 (t, *J* = 8 Hz,

1H), 7.60 - 7.49 (m, 1H), 7.37 (br d, $J$ = 8.4 Hz, 1H), 6.09 - 6.01 (m, 1H), 4.98 (t, $J$ = 9.2 Hz, 1H), 4.53 - 4.41 (m, 1H)

**$^{19}$F NMR:** (400 MHz, $CD_3OD$) $\delta$ 67.4, 118.7
**LCMS:** RT = 0.455 min, m/z = 434.1 $(M+H)^+$.
**SFC:** RT = 1.614 min.

***EXAMPLE 1.11- SYNTHESIS OF COMPOUND (11)***

***SYNTHESIS OF COMPOUND 2***

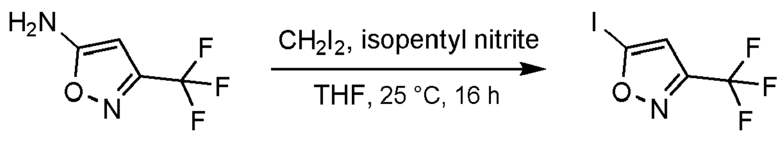


1 2

To a solution of **compound 1** (800 mg, 5.26 mmol, 1 *eq*) in THF (8 mL) was added isopentyl nitrite (924.42 mg, 7.89 mmol, 1.06 mL, 1.5 *eq*) and $CH_2I_2$ (1.69 g, 6.31 mmol, 509.27 $\mu$L, 1.2 *eq*). The mixture was stirred at 25 °C for 16 hr. The reaction mixture was concentrated in vacuum to give residue. The residue was purified by prep-TLC (TLC (Petroleum ether: Ethyl acetate = 1: 0; Rf = 0.70)) to give **compound 2** (1 g, crude) as colorless liquid. $^1$H NMR (400 MHz, $CDCCl_3$) $\delta$ = 6.74 (s, 1H)

***SYNTHESIS OF COMPOUND 3***

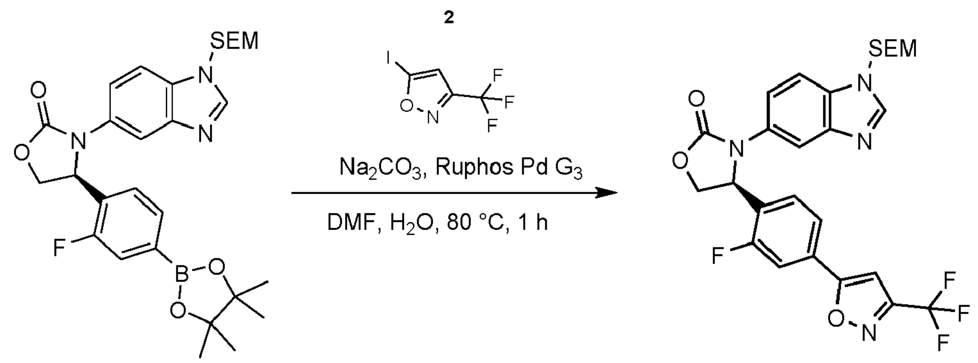


3 4

To a mixture of **compound 2** (261.29 mg, 10%, 9.94 $\mu$mol, 1.1 *eq*), **compound 1** (50 mg, 90.33 $\mu$mol, 1 *eq*) and $Na_2CO_3$ (19.15 mg, 180.67 $\mu$mol, 2 *eq*) in $H_2O$ (0.3 mL) and DMF (1.5 mL) was added RuPhos Pd Gs (7.56 mg, 9.03 $\mu$mol, 0.1 *eq*) under $N_2$. The mixture was stirred at 80 °C for 1 h. The reaction mixture was diluted with water (10 mL) and extracted with ethyl acetate (10 mL*3), the combined organic phase was dried with anhydrous $Na_2SO_4$, filtered and concentrated in vacuum to give residue. The residue was purified by silica gel chromatography (100-200 mesh silica gel, Petroleum ether/Ethyl acetate = 3/1, 1/1. TLC (Petroleum ether: Ethyl acetate = 1: 1; Rf = 0.43)) to give **compound 3** (90 mg, crude) as brown oil.

***SYNTHESIS OF COMPOUND (11)***

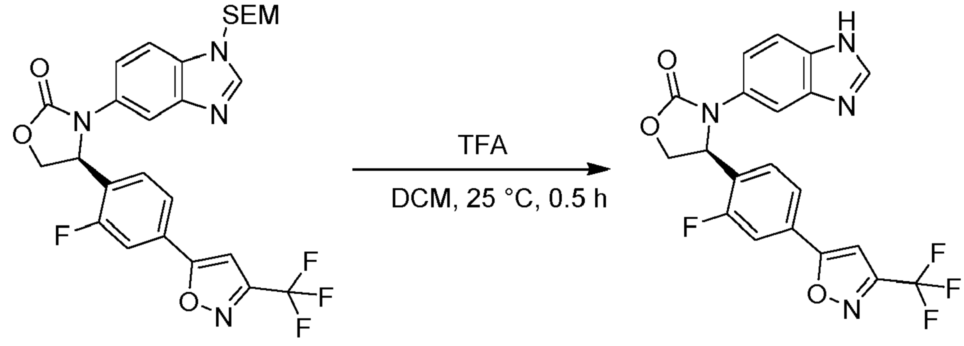


4 (11)

To a mixture of **compound 3** (90 mg, 159.98 μmol, 1 eq) in DCM (5 mL) was added TFA (1.25 mL) The mixture was stirred at 25 °C for 0.5 h. The reaction mixture was diluted with water (20 mL) and extracted with ethyl acetate (20 mL*3) and the combined organic phase was washed with saturated NaHCOs solution (20 mL*3), dried with anhydrous $Na_2SO_4$, filtered and concentrated in vacuum to give residue. The residue was purified by prep-HPLC (column: Phenomenex luna C18 150*25 mm* 10um; mobile phase: [water (FA) -ACN]; gradient: 18%-48% B over 10 min) to give **(11)** (7.04 mg, 16.28 μmol, 10.18% yield) as white solid.

$^1$**H NMR:** (400 MHz, $CD_3OD$) δ = 8.15 (s, 1H), 7.76 - 7.66 (m, 3H), 7.64 - 7.57 (m, 1H), 7.54 (d, *J* = 8.8 Hz, 1H), 7.38 - 7.32 (m, 1H), 7.29 (s, 1H), 6.05 - 5.99 (m, 1H), 4.97 (t, *J* = 9.2 Hz, 1H), 4.46 - 4.41 (m, 1H)

**LCMS:** RT = 0.462 min, m/z = 433.2 $(M+H)^+$.
**SFC:** RT = 2.138 min.

***EXAMPLE 1.12 - SYNTHESIS OF COMPOUND (12)***

***SYNTHESIS OF COMPOUND 3***

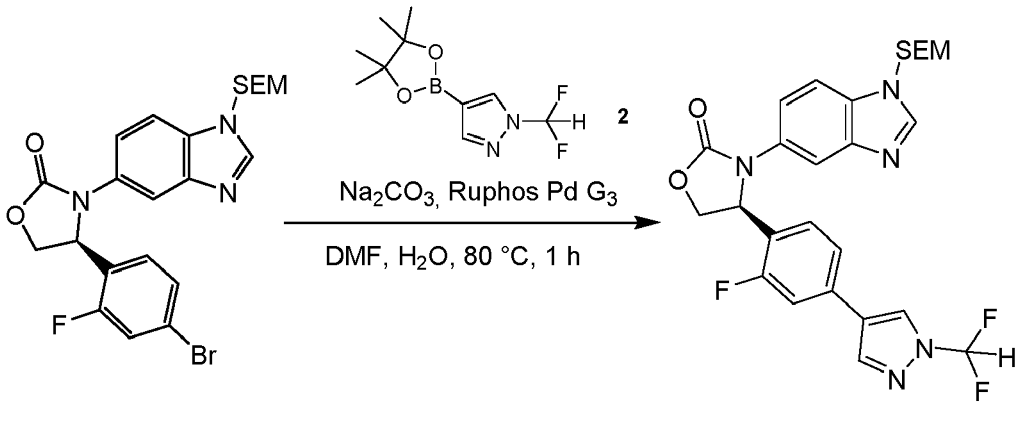


1 3

To a mixture of **compound 2** (24.09 mg, 98.73 μmol, 1 *eq*), **compound 1** (50 mg, 98.73 μmol, 1 *eq*) and $Na_2CO_3$ (20.93 mg, 197.46 μmol, 2 *eq*) in $H_2O$ (0.3 mL) and DMF (1.5 mL) was added RuPhos Pd Gs (8.26 mg, 9.87 μmol, 0.1 *eq*) under $N_2$. The mixture was stirred at 80 °C for 1 h. The reaction mixture was diluted with water (10 mL) and extracted with ethyl acetate (10 mL*3), the combined organic phase was dried with anhydrous $Na_2SO_4$, filtered and concentrated in vacuum to give residue. The residue was purified by silica gel chromatography (100-200 mesh silica gel, Petroleum ether/Ethyl acetate = 3/1, 1/1. TLC (Petroleum ether: Ethyl acetate = 1: 1; Rf = 0.43)) to give **compound 3** (50 mg, 91.98 μmol, 93.16% yield) as brown oil.

*SYNTHESIS OF COMPOUND (12)*

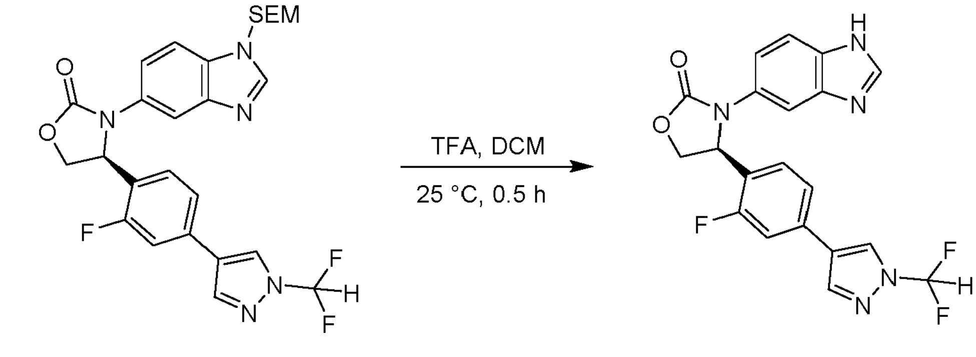


**3** **(12)**

To a mixture of **compound 3** (50 mg, 91.98 μmol, 1 *eq*) in DCM (5 mL) was added TFA (1.25 mL). The mixture was stirred at 25 °C for 0.5 h. The reaction mixture was diluted with water (20 mL) and extracted with ethyl acetate (20 mL*3) and the combined organic phase was washed with saturated NaHCOs solution (20 mL*3), dried with anhydrous $Na_2SO_4$, filtered and concentrated in vacuum to give residue. The residue was purified by prep-HPLC (column: YMC Triart C18 150*25 mm*5um; mobile phase: [water (FA) -ACN]; gradient: 12%-42% B over 10 min) to give **(12)** (14.89 mg, 36.02 μmol, 39.16% yield, 100% purity) as white solid.

**$^1$H NMR:** (400 MHz, $CD_3OD$) δ = 8.44 (s, 1H), 8.16 (br s, 1H), 8.05 (s, 1H), 7.70 - 7.64 (m, 1H), 7.56 - 7.55 (m, 1H), 7.54 (br d, J = 8.6 Hz, 1H), 7.47 (t, J = 60 Hz, 1H), 7.45 - 7.38 (m, 3H), 7.37 - 7.32 (m, 1H), 5.98 - 5.88 (m, 1H), 4.97 - 4.92 (m, 1H), 4.46 - 4.36 (m, 1H)

**LCMS:** RT = 0.410 min, m/z = 414.1 $(M+H)^+$.
**SFC:** RT = 1.056 min.

*EXAMPLE 1.13 - SYNTHESIS OF COMPOUND (13)*

*SYNTHESIS OF COMPOUND 3*

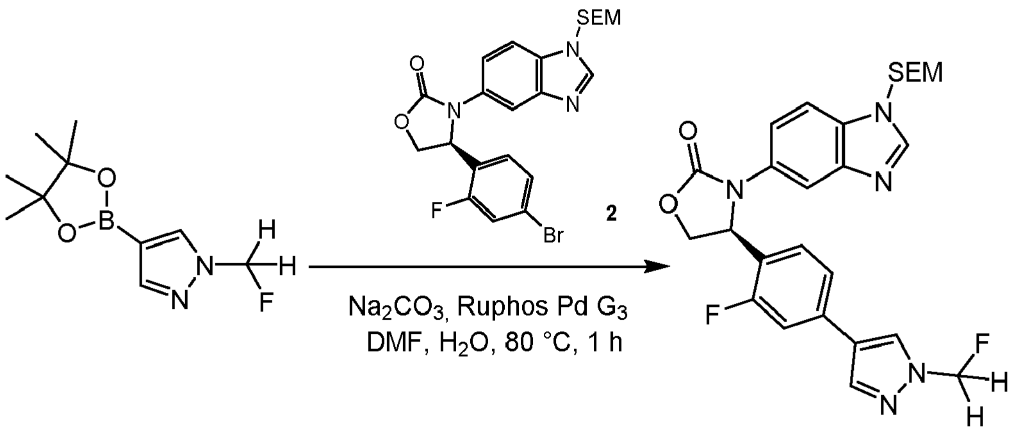


**1** **3**

To a mixture of **compound 1** (22.32 mg, 98.73 μmol, 1 eq), **compound 2** (50 mg, 98.73 μmol, 1 *eq*) and $Na_2CO_3$ (20.93 mg, 197.46 μmol, 2 *eq*) in $H_2O$ (0.3 mL) and DMF (1.5 mL) was added RuPhos Pd Gs (8.26 mg, 9.87 μmol, 0.1 *eq*) under $N_2$. The mixture was stirred at 80 °C for 1 h. The reaction mixture was diluted with water (10 mL) and extracted with ethyl acetate (10 mL*3), the combined organic phase was dried with anhydrous $Na_2SO_4$, filtered and

concentrated in vacuum to give residue. The residue was purified by silica gel chromatography (100-200 mesh silica gel, Petroleum ether/Ethyl acetate = 3/1, 1/1. TLC (Petroleum ether: Ethyl acetate = 1: 1; Rf = 0.43)) to give **compound 3** (50 mg, crude) as brown oil.

***SYNTHESIS OF COMPOUND (13)***

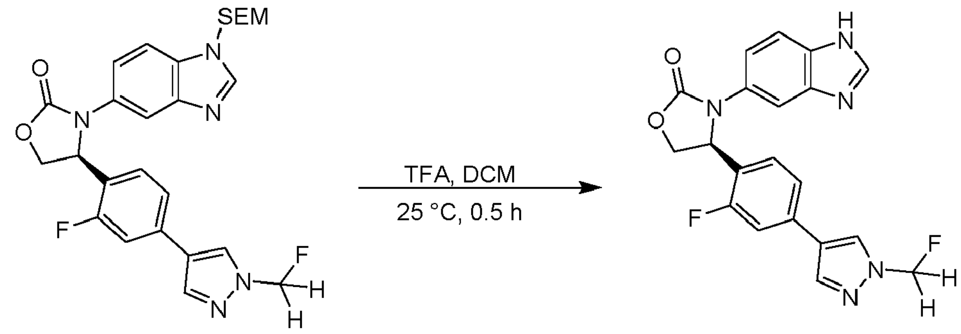


**3** **(13)**

To a solution of **compound 3** (50 mg, 95.13 μmol, 1 eq) in DCM (5 mL) was added TFA (1.25 mL). The mixture was stirred at 25 °C for 0.5 h. The reaction mixture was diluted with water (20 mL) and extracted with ethyl acetate (20 mL*3) and the combined organic phase was washed with saturated NaHCOs solution (20 mL*3), dried with anhydrous $Na_2SO_4$, filtered and concentrated in vacuum to give residue. The residue was purified by prep-HPLC (column: Phenomenex luna C18 150*25 mm* 10um; mobile phase: [water (FA) - ACN]; gradient: 3%-33% B over 10 min) to give **(13)** as white solid (crude). The **(13)** (crude) was purified by prep-HPLC (column: YMC Triart C18 150*25 mm*5um; mobile phase: [water (FA) -ACN]; gradient: 9%-39% B over 10 min) to give **(13)** (2.17 mg, 5.21 μmol, 5.48% yield, 95% purity) as white solid.

**$^1$H NMR:** (400 MHz, $CD_3OD$) $\delta$ = 8.27 (s, 1H), 8.14 (s, 1H), 7.99 (s, 1H), 7.66 (d, *J* = 2 Hz, 1H), 7.53 (d, *J* = 8.8 Hz, 1H), 7.40 - 7.33 (m, 4H), 6.16 - 5.96 (m, 2H), 5.95 - 5.87 (m, 1H), 4.94 (s, 1H), 4.47 - 4.35 (m, 1H)

**LCMS:** RT = 0.391 min, m/z = 396.2 $(M+H)^+$.
**SFC:** RT = 2.102 min.

***EXAMPLE 1.14* - *SYNTHESIS OF COMPOUND (14)***

***SYNTHESIS OF COMPOUND 3***

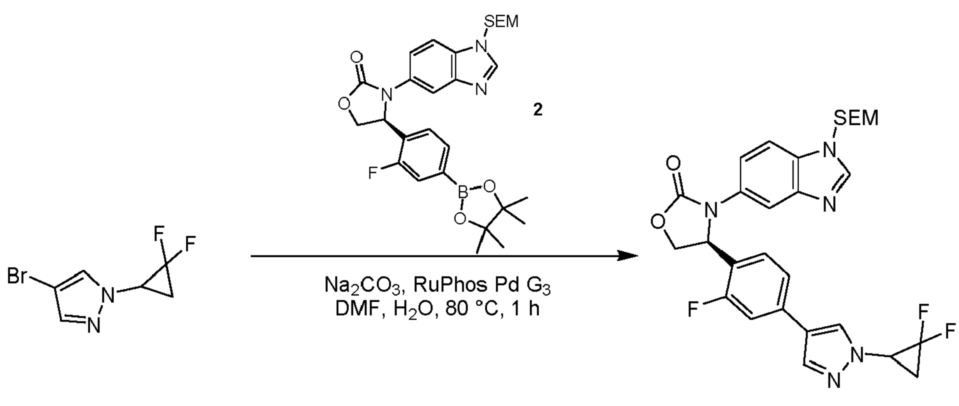


**1** **3**

To a solution of **compound 1** (20.15 mg, 90.33 μmol, 1 eq) and **compound 2** (50 mg, 90.33 μmol, 1 *eq*) in

DMF (1 mL) and $H_2O$ (0.1 mL) was added $Na_2CO_3$ (19.15 mg, 180.67 μmol, 2 *eq*) and RuPhos Pd Gs (7.56 mg, 9.03 μmol, 0.1 *eq*) under $N_2$. The mixture was stirred at 80 °C for 1 hr. The reaction mixture was concentrated under reduced pressure to give residue. The residue was purified by column chromatography (SiOz, Petroleum ether/Ethyl acetate = 1/1 to 0/1, TLC, Petroleum ether/Ethyl acetate = 0/1, $R_f$ = 0.2), the eluent was concentrated under reduced pressure to give **compound 3** (40 mg, crude) as yellow gum. **LCMS:** RT = 0.525 min, m/z = 570.3 $(M+H)^+$.

***SYNTHESIS OF COMPOUND (14)***

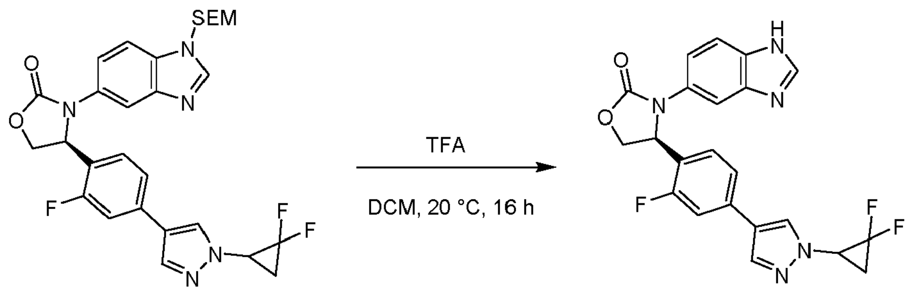


3 (14)

To a solution of **compound 3** (35 mg, 61.44 μmol, 1 *eq*) in $CH_2Cl_2$ (0.5 mL) was added TFA (0.25 mL) and, the mixture was stirred at 20 °C for 2 hr. **Compound 3** was remained, desired mass was detected. The mixture was stirred at 20 °C for 14 h. The reaction mixture was concentrated under reduced pressure to give residue. The residue was purified by prep-HPLC (column: YMC-Actus Triart C18 150*30 mm*7um; mobile phase: [water (FA) - ACN]; gradient: 18%-48% B over 10 min), the eluent was concentrated and freeze dried to give **(14)** (7.04 mg, 15.38 μmol, 25.03% yield, 96% purity) as white solid.

**$^1$H NMR:** (400 MHz, $CD_3OD$) δ = 8.19 - 8.11 (m, 2H), 7.88 (s, 1H), 7.65 (d, *J* = 1.8 Hz, 1H), 7.53 (d, *J* = 8.6 Hz, 1H), 7.40 - 7.31 (m, 4H), 5.90 (dd, *J* = 5.9, 9.2 Hz, 1H), 4.97 - 4.90 (m, 1H), 4.41 (dd, *J* = 6.1, 8.5 Hz, 1H), 4.36 - 4.28 (m, 1H), 2.36 - 2.16 (m, 2H) ;
**LCMS:** RT = 0.421 min, m/z = 440.1 $(M+H)^+$.

***EXAMPLE 1.15 - SYNTHESIS OF COMPOUND (15)***

***SYNTHESIS OF COMPOUND 3***

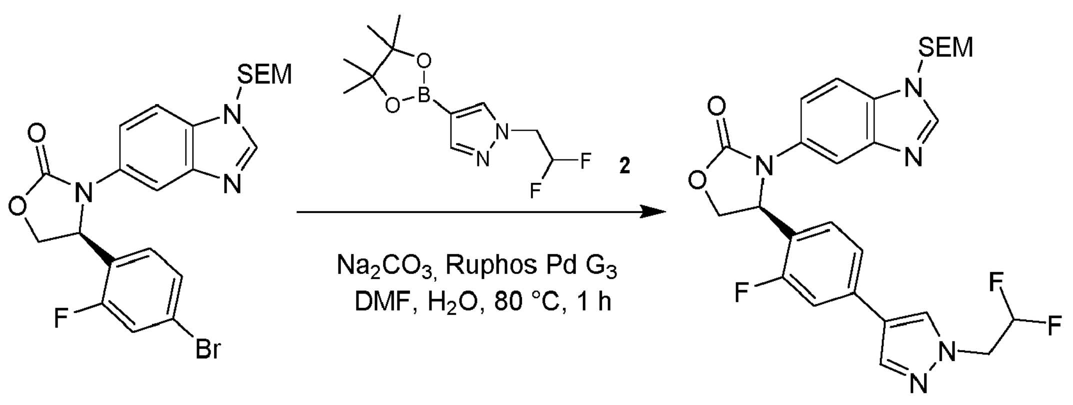


1 3

To a mixture of **compound 2** (25.48 mg, 98.73 μmol, 1 *eq*), **compound 1** (50 mg, 98.73 μmol, 1 *eq*) and $Na_2CO_3$ (20.93 mg, 197.46 μmol, 2 *eq*) in $H_2O$ (0.3 mL) and DMF (1.5 mL) was added RuPhos Pd Gs (8.26 mg, 9.87

μmol, 0.1 *eq*) under $N_2$. The mixture was stirred at 80 °C for 1 h. The reaction mixture was diluted with water (10 mL) and extracted with ethyl acetate (10 mL*3), the combined organic phase was dried with anhydrous $Na_2SO_4$, filtered and concentrated in vacuum to give residue. The residue was purified by silica gel chromatography (100-200 mesh silica gel, Petroleum ether/Ethyl acetate = 3/1, 1/1. TLC (Petroleum ether: Ethyl acetate = 1: 1; Rf = 0.43)) to give **compound 3** (50 mg, crude) as brown oil.

***SYNTHESIS OF COMPOUND (15)***

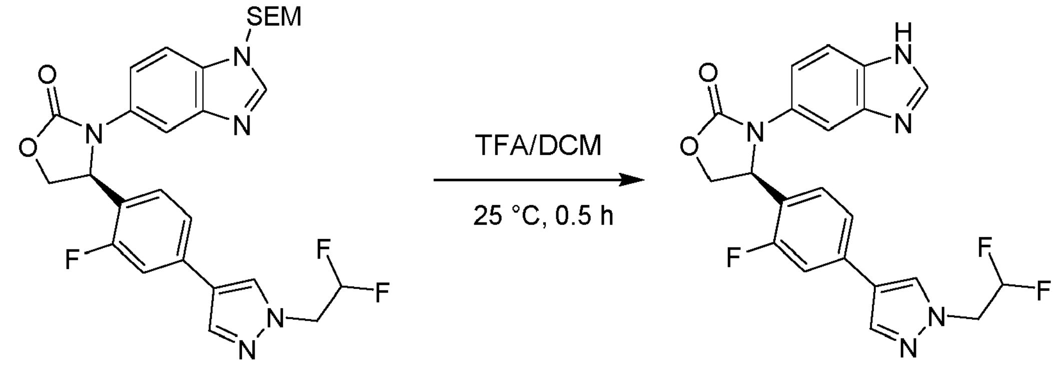


**3** **(15)**

To a mixture of **compound 3** (50 mg, 89.66 μmol, 1 *eq*) in DCM (5 mL) was added TFA (1.25 mL). The mixture was stirred at 25 °C for 0.5 h. The reaction mixture was diluted with water (20 mL) and extracted with ethyl acetate (20 mL*3) and the combined organic phase was washed with saturated NaHCOs solution (20 mL*3), dried with anhydrous $Na_2SO_4$, filtered and concentrated in vacuum to give residue. The residue was purified by prep-HPLC (column: Phenomenex luna C18 150*25 mm* 10um; mobile phase: [water (FA) - ACN]; gradient: 8%-38% B over 10 min) to give **(15)** (16.57 mg, 38.77 μmol, 43.24% yield) as off-white solid.

**$^1$H NMR:** (400 MHz, $CD_3OD$) δ = 8.13 (s, 1H), 8.04 (s, 1H), 7.87 (s, 1H), 7.65 (d, *J* = 2 Hz, 1H), 7.53 (d, *J* = 8.8 Hz, 1H), 7.40 - 7.29 (m, 4H), 6.35 - 6.00 (m, 1H), 5.94 - 5.85 (m, 1H), 4.95 - 4.91 (m, 1H), 4.63 - 4.48 (m, 2H), 4.44 - 4.36 (m, 1H)

**LCMS:** RT = 0.402 min, m/z = 428.2 $(M+H)^+$.

**SFC:** RT = 1.863 min.

***EXAMPLE 1.16*** - ***SYNTHESIS OF COMPOUND (16)***

***SYNTHESIS OF COMPOUND 3***

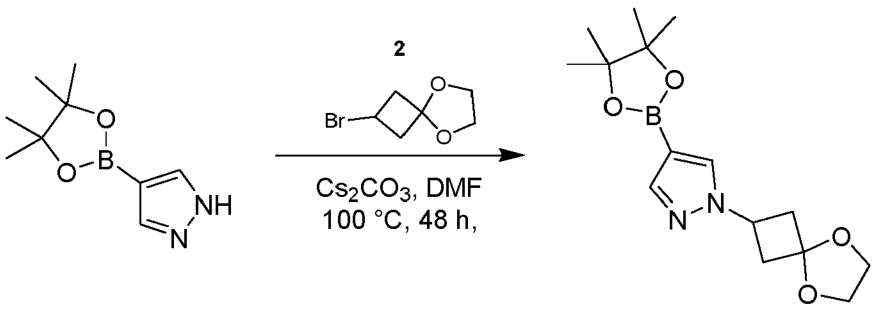


**1** **3**

To a solution of **compound 2** (497.42 mg, 2.58 mmol, 1 *eq*) in DMF (10 mL) was added $Cs_2CO_3$ (1.68 g, 5.15

mmol, 2 *eq*) and **compound 1** (500 mg, 2.58 mmol, 1 *eq*). The mixture was stirred at 100 °C for 48 hr. The reaction mixture was diluted with water (10 mL) and extracted with ethyl acetate (10 mL*3) and the combined organic phase was washed with brine (10 mL*3), dried with anhydrous $Na_2SO_4$, filtered and concentrated in vacuum to give residue. The residue was purified by silica gel chromatography (100-200 mesh silica gel, Petroleum ether/Ethyl acetate = 1/0) to give **compound 3** (200 mg, 653.24 μmol, 25.35% yield) as colorless oil

***SYNTHESIS OF COMPOUND 5***

4

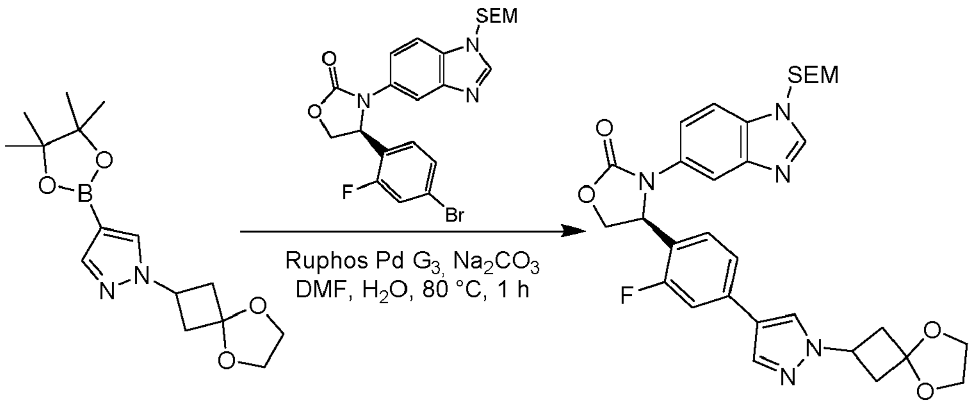


3

5

To a mixture of **compound 3** (181.36 mg, 592.37 μmol, 1 *eq*), **compound 4** (300 mg, 592.37 μmol, 1 *eq*) and $Na_2CO_3$ (125.57 mg, 1.18 mmol, 2 *eq*) in $H_2O$ (0.8 mL) and DMF (4 mL) was added RuPhos Pd Gs (49.54 mg, 59.24 μmol, 0.1 *eq*) under $N_2$. The mixture was stirred at 80 °C for 1 h. The reaction mixture was diluted with water (20 mL). The resulting mixture was extracted with ethyl acetate (20 mL*3), the combined organic phase was dried with anhydrous $Na_2SO_4$, filtered and concentrated in vacuum to give residue. The residue was purified by silica gel chromatography (100-200 mesh silica gel, Petroleum ether/Ethyl acetate = 1/1, 0/1. TLC (Petroleum ether: Ethyl acetate = 0: 1; Rf = 0.43) to give **compound 5** (300 mg, crude) as brown oil. LCMS: RT = 0.508 min, m/z = 606.4$(M+H)^+$.

***SYNTHESIS OF COMPOUND 6***

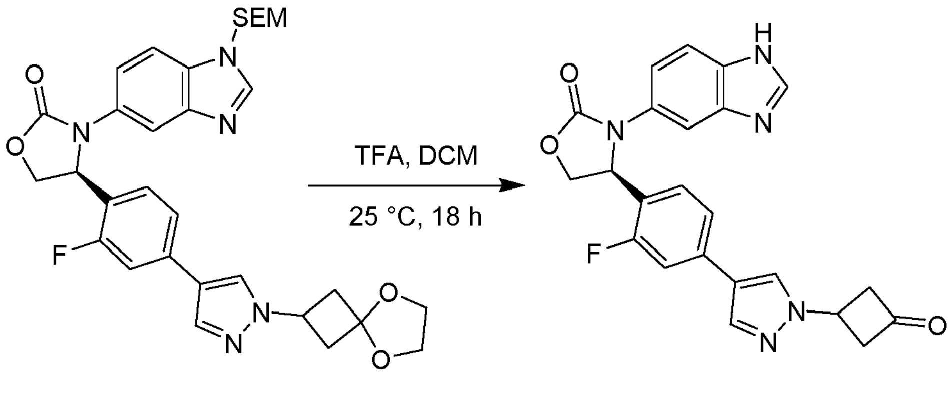


5 6

To a mixture of **compound 5** (300 mg, 495.27 μmol, 1 eq) in DCM (5 mL) was added TFA (1.25 mL). The mixture was stirred at 25 °C for 18 h. The reaction mixture was diluted with water (20 mL) and extracted with ethyl acetate (20 mL*3) and the combined organic phase was washed with saturated NaHCOs solution (20 mL*3), dried with anhydrous

$Na_2SO_4$, filtered and concentrated in vacuum to give **compound 6** (200 mg, crude) as brown oil.

***SYNTHESIS OF (16)***

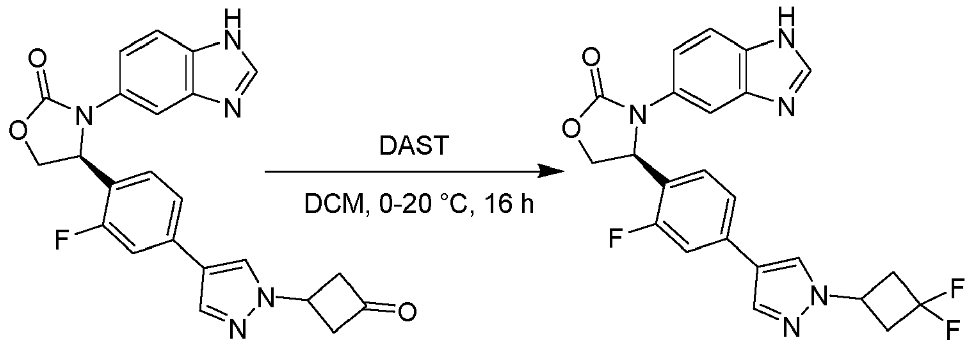


6

**(16)**

To a solution of **compound 6** (100 mg, 231.79 μmol, 1 *eq*) in DCM (2 mL) was added DAST (74.73 mg, 463.59 μmol, 61.25 μL, 2 *eq*) under 0 °C. The mixture was stirred at 20 °C for 16 hr. The mixture was diluted with $H_2O$ (20 mL) and was extracted with ethyl acetate

(20 mL*3), the combined organic phase was dried with anhydrous $Na_2SO_4$, filtered and concentrated in vacuum to give a residue. The residue was purified by prep-HPLC (column: YMC Triart C18 150*25 mm*5um; mobile phase: [water (FA) -ACN]; gradient: 15%-45% B over 10 min) to give white solid. The solid was purified by prep-HPLC (column: Waters Xbridge 150*25 mm* 5um; mobile phase: [water ($NH_4HCO_3$) -ACN]; gradient: 26%-56% B over 9 min) to give **(16)** (4.51 mg, 9.95 μmol, 4.29% yield, 100% purity) as white solid.

**$^1$H NMR:** (400 MHz, $CD_3OD$) δ = 8.13 (s, 1H), 8.09 (s, 1H), 7.88 (s, 1H), 7.65 (d, *J* = 2.0 Hz, 1H), 7.60 - 7.47 (m, 1H), 7.40 - 7.28 (m, 4H), 5.96 - 5.85 (m, 1H), 4.96 - 4.93 (m, 1H), 4.85 - 4.79 (m, 1H), 4.47 - 4.37 (m, 1H), 3.21 - 3.08 (m, 4H)

**LCMS:** RT = 0.524 min, m/z = 454.2$(M+H)^+$.
**SFC:** RT = 1.122 min.

***ABBREVIATIONS***

| | | | |
|---|---|---|---|
| ACN | acetonitrile | LiHMDS | lithium hexamethyldisilazane |
| BAST | bis(2-methoxyethyl)aminosulfur trifluoride | MeOH | Methanol |
| dba | dibenzylidineacetone | NBS | N-bromosuccinimide |
| Bn | Benzyl | NMR | nuclear magnetic resonance spectroscopy |
| DCM | dichloromethane | PCC | pyridinium chlorochromate |
| DIBAL(-H) | diisobutylaluminum hydride | PG | protecting group |
| DIEA | N,N-diisopropylethylamine | Py | Pyridine |
| DMF | N,N-dimethylformamide | RT | Retention time |
| DMSO | dimethylsulfoxice | RuPhos | 2-dicyclohexylphosphino-2',6'-diisopropoxybiphenyl |
| dppf | 1,1'-bis(diphenylphosphino) ferrocene | SEMCI | 2-(trimethylsilyl)ethoxymethyl chloride |

(continued)

| | | | |
|---|---|---|---|
| EtOAc | Ethyl Acetate | SFC | supercritical fluid chromatography |
| HPLC | high performance liquid chromatography | TFA | trifluoroacetic acid |
| IBX | 2-iodoxybenzoic acid | THF | tetrahydrofuran |
| LCMS | Liquid chromatography-mass spectrometry | TLC | Thin-layer chromatography |
| LDA | lithium diisopropylamide | XantPhos | 4,5-bis(diphenylphosphino)-9,9-dimethylxanthene |
| | | XPhos | 2-dicyclohexylphosphino-2',4',6'-triisopropylbiphenyl |

***Example 2 - Synthesis of reference compounds (17)-(19)***

Reference compounds (17), (18) and (19) are not according to the invention, but are instead used to benchmark compounds (1)-(16) according to the invention. Compounds (17), (18) and (19) can be synthesized as described in WO2011029920A1.

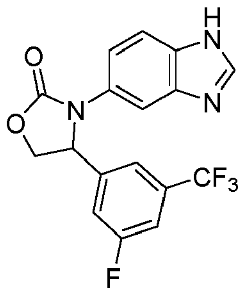

(17)

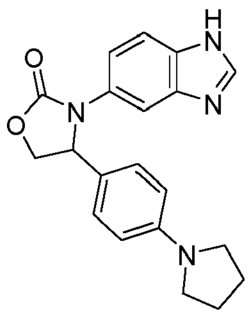

(18)

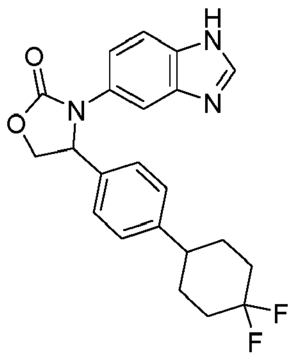

(19)

These compound are presented below either as racemic mixtures ((17)-Rac, (18)-Rac, (19)-Rac), or as either one of two isolated stereoisomers ((17)-ent1 or (17)-ent2; (18)-ent1 or (18)-ent2; (19)-ent1 or (19)-ent2).

***Example 3*** - ***Biological assays***

In order to determine the potency of compounds according to the invention towards inhibiting QPCTL enzymatic activity, $IC_{50}$ values were obtained using a fluorescent enzymatic assay in which glutamine-4-amino-7-methylcoumarine is pyroglutamylated by either isoQC or QC and subsequently measured in a coupled enzymatic detection step (Assays 1 and 2, respectively, below). Finally, a cell-based assay was deployed to measure the cellular potency of compounds according to the invention using a combination of an antibody that specifically binds to isoQC dependent pyroglutamylated CD47, as well as an antibody that binds to CD47 independent of its pyroglutamylation status, thereby controlling for inadvertent downregulation of CD47 itself (Assay 3 below).

***VECTOR GENERATION***

LentiCRISPRv2 vector (Genscript) was cut with PacI and EcoRI restriction enzymes (NEB) for 1 hour at 37°C (New England Biolabs) in CutSmart buffer (NEB) and the backbone was isolated from gel using a Qiaquick Gel Extraction Kit (Qiagen) according to the manufacturer's protocol.

A synthetic DNA fragment (SEQ ID NO: 1) was inserted using NEBuilder HiFi DNA Assembly Master Mix (New England Biolabs) and ensuing GIBSON assembly for 30 minutes at 50°C and transformation into Endura bacteria (Lucigen) to obtain pSCNC-LentiCRISPR.

Subsequently, BsmBI (New England Biolabs) digested pSCNC-LentiCRISPR (1 hour at 55°C in buffer 3.1

(NEB)) was isolated from gel using a Qiaquick Gel Extraction Kit (Qiagen) and oligonucleotides encoding gRNAs targeting QPCTL (SEQ ID NO: 2) or CD47 (SEQ ID NO: 3) were cloned in using a 30 minute GIBSON assembly at 50°C using NEBuilder HiFi DNA Assembly Master Mix (New England Biolabs) and subsequent transformation into Endura cells to obtain pSCNC-LentiCRISPR-QPCTL and pSCNC-LentiCRISPR-CD47 respectively.

***TOTAL CD47/PE-CD47 DUAL STAINING***

Cells were spun for 4 minutes at 1500 rpm and washed once with PBS / 0.5% BSA. Cells were spun down again and the supernatant was removed and incubated in 100 uL antibody staining solution (PBS / 0.5% BSA / 0.1 μg/mL DAPI (4',6-diamidino-2-phenylindole, ThermoFisher Scientific) and a mixture of 1:500 FITC-conjugated anti-human CD47 clone 2D3, which recognizes total CD47 irrespective of its pyroglutamylation state, and 1:500 Alexa647-conjugated anti-human CD47 clone CC2C6, which specifically detects pyroglutamylated CD47) for 2 hours at 4°C. Next, the cells were spun down and washed twice with 2 x 150 μL PBS / 0.5% BSA to remove unbound antibody. Cells were again spun down and taken up in 300 μL FACS buffer before analysis on a Celesta FACS machine (BD Biosciences).

***CREATION OF QPCTL AND CD47 KNOCKOUT CELLS***

KBM7 cells (Kotecki et al., 1999) were infected with lentivirus produced by transfecting pSCNC-LentiCRISPR-QPCTL or pSCNC-LentiCRISPR-CD47 into HEK-293T cells according to standard protocols. After 24 hours incubation, cells were selected with puromycin (Invivogen, 1 μg/mL) for 48 hours. Cells were stained for total CD47 and pE-CD47 and QPCTL k.o. cells were sorted out as negative for pE-CD47, and CD47 k.o. cells as negative for total CD47, using a FACSAria III and FACS Diva software (BD Biosciences).

***ASSAY* 1 - *isoQC POTENCY-FLUORESCENT ENZYMATIC ASSAY***

The Golgi luminal, enzymatically active region of human isoQC (S53-L382; see, e.g., Huang et al., 2011) was obtained by expression in E. coli of an N-terminally GST Enterokinase and C-terminally 6xHis tagged construct in the pET41(+) vector and subsequent purification using Ni-NTA IMAC. Enterokinase digestion ensued, followed by another Ni-NTA IMAC purification, Superdex75 based size exclusion and finally a spin concentration using a 10 kDa molecular weight cutoff. The final protein is formulated in 50 mM Tris-HCl, 150 mM NaCl, 50% Glycerol, pH 7.8.

Pyroglutamylation was measured by conversion of glutamine-4-amino-7-methylcoumarine (H-Gln-AMC, Bachem) to pyroglutamyl-AMC, which is subsequently a specific substrate for pyroglutamylaminopeptidase (pGAPase, Qiagen), releasing free AMC which can be fluorescently detected. For a typical inhibition assay, 7.5 μL aliquots of threefold dilutions of test compound in 50mM Tris pH8 buffer were plated in 384-well plates. 15 μL 20 μM hGln-AMC (diluted from a 40 mM stock dissolved in 25 mM HEPES using 50 mM Tris-HCl 25 pH 8.0) was added to each well. Finally, 7.5 μL, 3 ng/μL recombinant isoQC enzyme was added and the reaction mixture was incubated for 1 hour at 37°C. A 5 minute incubation at 98°C was then performed to denature the isoQC enzyme and stop the reaction.

To detect the amount of pyroglutamyl-AMC formed, a 0.125 U/mL pGAPase enzyme diluted in 50 mM Tris-HCl pH 8.0 containing 10 mM dithiothreitol (DTT) solution was prepared. 25 μL of the pyroglutamylation reaction mixture was transferred into an empty plate and to that, 25 μL of the pGAPase enzyme solution was added. Plates were then incubated for 25 minutes at room temperature before fluorescence readings were taken on an Envision2 plate reader with excitation set at 380 nm and emission at 450 nm. DMSO concentrations during the assay never exceeded 1% and activities were compared to controls containing only the same amount of DMSO and no test compound. Controls were taken along containing pyroglutamylated-AMC (Bachem, dissolved at 10 mM in DMSO) instead of H-Gln-AMC to control for pGAPase inhibition.

IC50 values are calculated using non-linear regression analysis with GraphPad Prism software.

***A SSA Y* 2 - *QC POTENCY-FLUORESCENT ENZYMATIC ASSAY***

Recombinant QC enzyme was produced by bacterial expression of residues 33-361 of uniprot ID *Q16769*, fused to a His tag, codon optimized and cloned into the pET32a plasmid backbone. After scale-up, expression and lysis, protein was purified by Ni-NTA IMAC, dialysed in the presence of Factor Xa, further purified by Ni-NTA reverse IMAC and finally filtrated for size on a Superdex 200 16/60 column. Final protein was concentrated using a spin concentrator at 10 kDa molecular weight cut-off and formulated in 150 mM NaCl, 50 mM Tris-HCl, 50% Glycerol, pH 8.0.

Pyroglutamylation was measured by conversion of glutamine-4-amino-7-methylcoumarine (H-Gln-AMC, Bachem) to pyroglutamyl-AMC, which is subsequently a specific substrate for pyroglutamylaminopeptidase (pGAPase, Qiagen), releasing free AMC which can be fluorescently detected. For a typical inhibition assay, 7.5 μL aliquots of threefold dilutions of test compound in 50 mM Tris pH8 buffer were plated in 384-well plates. 15 μL 20 μM h-Gln-AMC

(diluted from a 40 mM stock dissolved in 25 mM HEPES using 50 mM Tris-HCl pH 8.0) was added to each well. Finally, 7.5 μL, 3 ng/μL recombinant QC enzyme was added and the reaction mixture was incubated for 1 hour at 37°C. A 5 minute incubation at 98°C was then performed to denature the QC enzyme and stop the reaction.

To detect the amount of pyroglutamyl-AMC formed, a 0.125 U/mL pGAPase enzyme diluted in 50 mM Tris-HCl pH 8.0 containing 10 mM dithiothreitol (DTT) solution was prepared. 25 μL of the pyroglutamylation reaction mixture was transferred into an empty plate and to that, 25 μL of the pGAPase enzyme solution was added. Plates were then incubated for 25 minutes at room temperature before fluorescence readings were taken on an Envision2 plate reader with excitation set at 380 nm and emission at 450 nm.

DMSO concentrations during the assay never exceeded 1% and activities were compared to controls containing only the same amount of DMSO and no test compound. Controls were taken along containing pyroglutamylated-AMC (Bachem, dissolved at 10 mM in DMSO) instead of H-Gln-AMC to control for pGAPase inhibition.

IC50 values are calculated using non-linear regression analysis with GraphPad Prism software.

*ASSAY 3 - CELL-BASED POTENCY ASSAY*

In order to test compounds for their effect on pyroglutamylation in live cells, 25000 KBM7 cells per well were seeded in a 96-well U-bottom plate in 150 μl IMDM / 10% FCS / 1% Pen / Strep medium and a 10-point 2-fold dilution range of test compounds. After 72 hours incubation at 37°C, 5% COz, cells were stained for total CD47 and pE-CD47 and analysed on a BD Celesta using FACS Diva software (BD Biosciences). Cells gated for DAPI negativity were recorded and the median fluorescence intensity (MFI) of each sample was transformed to linearly scale between the MFI's of wildtype and CD47 knockout cells (in case of the 2D3 antibody) and the wildtype and QPCTL knockout cells (in case of the CC2C6 antibody). ICso's were calculated by 3 parameter logistic regression of the CC2C6 signal vs. compound concentration, where the top of the sigmoidal curve was fixed at 1, using R and the drc package (http://www.r-project.org, https://cran.r-project.org/web/packages/drc/index.html).

***RESULTS***

Compounds (1)-(16) according to the invention (Table 1) are more potent than the reference compounds (17)-(19) (Table 2) derived from WO2011029920A1. Lower $IC_{50}$ values are obtained for assay 1 and 3 for all compounds (1)-(16) compared to the reference compounds, signifying a higher potency.

| **Table 1: Assays of compounds according to the invention** | | | |
|---|---|---|---|
| | isoQC | QC | Cell |
| | potency (nM) | potency (nM) | potency (nM) |
| | Assay 1 | Assay 2 | Assay 3 |
| Compound # | | | |
| (1) | 1.7 | 10.3 | 92.1 |
| (2) | 4 | 38.9 | 106 |
| (3) | 3.1 | 22.5 | 159 |
| (4) | 5.6 | 10.8 | 378 |
| (5) | 7.7 | 34.7 | 659 |
| (6) | 3.0 | 15.1 | n.y.d. |
| (7) | 10 | 70.1 | 503 |
| (8) | 13.4 | 86.0 | 526 |
| (9) | 9 | 36.1 | 622 |
| (10) | 12.6 | 48.2 | Na |
| (11) | 5.2 | 24.3 | 252 |
| (12) | 3.3 | 7.5 | 157 |
| (13) | 4 | 11.3 | 401 |

(continued)

| Table 1: Assays of compounds according to the invention | | | |
|---|---|---|---|
| | isoQC | QC | Cell |
| | potency (nM) | potency (nM) | potency (nM) |
| | Assay 1 | Assay 2 | Assay 3 |
| Compound # | | | |
| (14) | 3.6 | 12.7 | n.y.d. |
| (15) | 2.7 | 8.0 | 155 |
| (16) | 5.1 | 9.4 | n.y.d. |

In addition, the following reference compounds were prepared for the purpose of comparing enzymatic inhibition potency and cell potency of reference compounds with the compounds described herein.

| Table 2: Assays for control compounds | | | |
|---|---|---|---|
| | isoQC | QC | Cell |
| | potency (nM) | potency (nM) | potency (nM) |
| | Assay 1 | Assay 2 | Assay 3 |
| Compound # | | | |
| (17)-Rac | 1195 | 1730 | >50,000 |
| (17)-ent1 | 10906 | >10,000 | >50,000 |
| (17)-ent2 | 632.1 | 1080 | >50,000 |
| (18)-Rac | 48.4 | 49.1 | 8,510 |
| (18)-ent1 | 1174 | 2530 | >50,000 |
| (18)-ent2 | 23.9 | 31.2 | 3,490 |
| (19)-Rac | 63.7 | 104 | 10,400 |
| (19)-ent1 | 1243 | 4490 | >12,500 |
| (19)-ent2 | 35.8 | 91.2 | 3,730 |

***Example 4 - CACO-2-Based Cell-Permeation and Efflux***

CACO-2 (ATCC, Manassas, VA, USA) monolayers on polyethylene membranes in 96-well insert plates were incubated for 2 hours in 5% C02, 37°C with 2 μM compound tested bidirectionally in duplicate using HBSS with 10 mM HEPES at pH 7.40 ±0.05 as transport buffer. Final DMSO Concentrations were adjusted to less than 1%. All samples were mixed with acetonitrile containing internal standard and centrifuged at 3220 x g for 10 minutes. Subsequently, 100 μL supernatant solution was diluted with 100 μL distilled water for LC/MS/MS analysis. Lucifer yellow rejection assays were applied after the transport assay to ensure CACO-2 cell monolayer integrity.

The apparent permeability coefficient Papp (cm/s) was calculated using the equation:

$$Papp = (dCr/dt) \times Vr / (A \times CO)$$

where dCr/dt is the cumulative concentration of compound in the receiver chamber as a function of time (μM/s); Vr is the solution volume in the receiver chamber (0.075 mL on the apical side, 0.25 mL on the basolateral side); A is the surface area for the transport, i.e., 0.0804 $cm^2$ or the area of the monolayer; CO is the initial concentration in the donor chamber (μM).

| Table 3: CACO-2 assay data | | |
|---|---|---|
| | A2B | B2A |
| | $P_{app}$ ($10^{-6}$ cm/s) | $P_{app}$ ($10^{-6}$ cm/s) |
| Compound # | | |
| (1) | 2.0 | 21.9 |
| (17)-ent2 | 1.5 | 36.8 |
| (18)-ent2 | 2.8 | 18.8 |
| (19)-ent2 | 3.9 | 23.9 |

***Example 5 - Microsomal Stability mouse, human, rat and dog (MLM, HLM, RLM and DLM)***

In order to assess metabolic stability of test compounds, commercially acquired CD-1 mouse liver microsome preparations were incubated in 100 µL at a final concentration of 0.5 mg protein/mL, 1 µM test compound, 0.99% methanol and 0.01% DMSO, after a 10 minute pre-incubation at 37°C and addition of 0.1 volumes 10 U/mL NADPH in 10 mM $MgCl_2$. Reactions were stopped by addition of 300 µL cold acetonitrile containing internal standards at t = 0, 5, 10, 20, 30, and 60 minutes. Samples were submitted to LC-MS/MS analysis subsequently and the slope of linear regression of time versus ln(% Remaining concentration) was derived to calculate t½ as t½= ln(2) /(-Slope). Then $Cl_{int(mic)}$ and $Cl_{int(liver)}$ were calculated as follows:

$$CL_{int(mic)} = \frac{0.693}{\text{In vitro } T_{1/2}} \bullet \frac{1}{\text{mg / mL microsomal protein in reaction system}}$$

$$CL_{int(liver)} = CL_{int(mic)} \bullet \frac{\text{mg microsomes}}{\text{g liver}} \bullet \frac{\text{g liver}}{\text{kg body weight}}$$

Where 45 mg microsomal protein/g liver and 88 g liver/kg mouse bodyweight were used as standardized parameters for mouse microsomes.

The same procedure was performed for human microsomes, SD rat microsomes and (beagle) dog microsomes.

Where 45 mg microsomal protein/g liver and 20 g liver/kg human bodyweight were used as standardized parameters for human microsomes.

Where 45 mg microsomal protein/g liver and 40 g liver/kg rat bodyweight were used as standardized parameters for rat microsomes.

Where 45 mg microsomal protein/g liver and 32 g liver/kg dog bodyweight were used as standardized parameters for human microsomes.

| Table 4: MLM and HLM assay data | | | | |
|---|---|---|---|---|
| | MLM | HLM | RLM | DLM |
| | $Cl_{int(liver)}$ (mL/min/kg) | $Cl_{int(liver)}$ (mL/min/kg) | $Cl_{int(liver)}$ (mL/min/kg) | $Cl_{int(liver)}$ (mL/min/kg) |
| Compound # | | | | |
| (1) | 67.5 | <8.6 | | |
| (17)-ent2 | 182.5 | <8.6 | 49.0 | <13.8 |
| (18)-ent2 | 488.2 | 73.8 | 1066.3 | 32.8 |
| (19)-ent2 | 339.2 | 19.7 | 49.9 | 21.8 |

*Example 6* - *Kinetic Solubility*

To assess the solubility of test compounds 10 μL of a 10 mM solution in DMSO was added to the lower chamber of a whatman miniuniprep (PTFE Filter Media with Polypropylene Housing) vial. 490 μL of 50 mM phosphate buffer (pH 7.4) was added to the lower chamber, the samples were vortexed for at least two minutes and then shaken on a shaker for 24 hours at room temperature at the speed of 880 rpm. The samples were then centrifuged for 20 minutes at 4000 rpm, compressed and the filtrates were injected into a HPLC-UV and LC-MS/MS system to calculate the concentration compared to the standard curve using a ACQUITY UPLC BEH C18 1.7 μm 2.1 * 50 mm column.

| **Table 5: Kinetic solubility** | |
|---|---|
| | Phosphate buffer |
| | Solubility (μM) |
| Compound # | |
| (1) | 121 |
| (17)-ent2 | 39 |
| (18)-ent2 | 60 |
| (19)-ent2 | 42 |

*Example* 7 - *Low Dose Pharmacokinetics*

In order to determine the stability of test compounds in plasma, low-dose pharmacokinetic experiments were carried out in C57Bl/6 mice. Test compounds were dissolved at 0.1 mg/mL in an appropriate vehicle and dosed intravenously at 1 mg per kg bodyweight in three animals per compound. At various (between 5 and 9) timepoints after injection, up to 24 hours, blood samples were drawn, plasma isolated and test compound concentrations were determined using LC-MS/MS. t½ values were subsequently calculated using Phoenix WinNonlin 6.3 software and averaged over the three mice per test compound.

The plasma half-life of (1) injected at a low dose of 1 mg per kg bodyweight ranged from 3.99 to 4.58 hours.

*Example 8* - *In vivo Raji xenograft mouse model*

Anti tumor effects were determined in a model for Burkitt's lymphoma. NSG mice were intravenuously injected with $1 \times 10^6$ Raji human Burkitt's lymphoma cells. Four days after tumor cell injection, animals were treated with compound (1) as a single agent and in combination with Rituximab (anti-CD20 antibody). Compound (1) was administered at 100 mg/kg orally, once daily. Rituximab was dosed at 10 mg/kg intravenously every third day (Q3D). Tumor progression was evaluated by monitoring animal health and body weight measurement. Animals that showed decrease in body weight (more than 15%) and ill health were euthanised. Animals treated with compound in combination with Rituximab, on average, lived 5.5 days longer than animals treated with Rituximab alone. These results indicate that compounds described herein have utility in treating patients with cancer, i.e. with non-Hodgkin's lymphomas such as Burkitt's lymphoma.

*Example 9* - *In vivo Pan02 syngeneic mouse model*

Anti tumor effects were evaluated in the Pan02, pancreatic adenocarcinoma syngeneic mouse tumor model. For this study $3 \times 10^6$ Pan02 cells were injected in the right front flank of C57BL/6 female mice. Tumor size was measured and when tumors reached an average size of 80-120 $mm^3$, treatment was started. Treatment consisted of compound alone, or in combination with the anti PD-L1 antibody (B7-H1). Compound (1) was administered orally at 100 mg/kg once daily and anti-PD-L1 antibody was administered intraperitoneally at 10 mg/kg twice weekly. Tumor progression was monitored by tumor volume measurement using a caliper. The compound did result in tumor growth inhibition by itself and in tumor regression when combined with the anti-PD-L1 antibody.

**Claims**

**1.** A compound represented by formula (I), or a pharmaceutically acceptable salt, hydrate or solvate thereof:

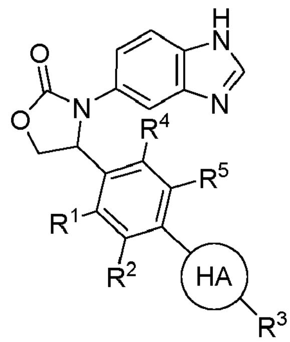

(I)

wherein $R^1$, $R^2$, $R^4$ and $R^5$ are independently H or a halogen,
wherein **HA** is a monocyclic heteroaryl,
wherein $R^3$ is a C1-C4 haloalkyl.

2. The compound, or the pharmaceutically acceptable salt, hydrate or solvate thereof, of claim 1 wherein $R^1$, $R^2$, $R^4$ and $R^5$ are independently H or F.

3. The compound, or the pharmaceutically acceptable salt, hydrate or solvate thereof, of claim 1 or 2, wherein $R^4$ and $R^5$ are H.

4. The compound, or the pharmaceutically acceptable salt, hydrate or solvate thereof, of any one of the preceding claims, wherein $R^1$ is F and $R^2$ is H.

5. The compound, or the pharmaceutically acceptable salt, hydrate or solvate thereof, of any one of the preceding claims, wherein **HA** is a five-membered heteroaryl.

6. The compound, or the pharmaceutically acceptable salt, hydrate or solvate thereof, of claim 5, represented by formula (II):

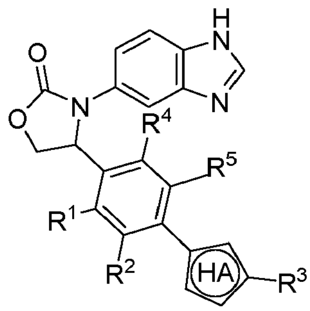

(II).

7. The compound, or the pharmaceutically acceptable salt, hydrate or solvate thereof, of claim 6, represented by formula (III), (IV), (V), or (VI):

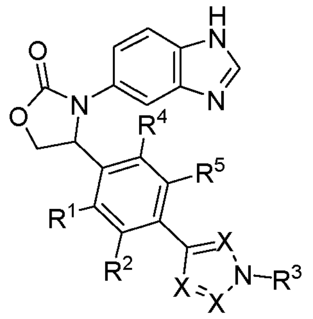

(III)

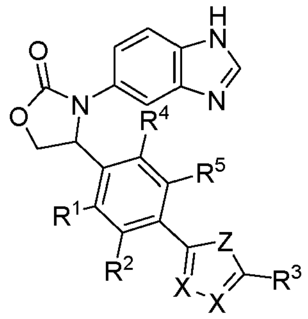

(IV)

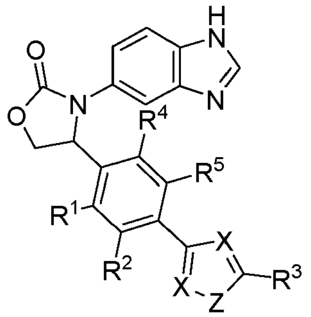

(V)

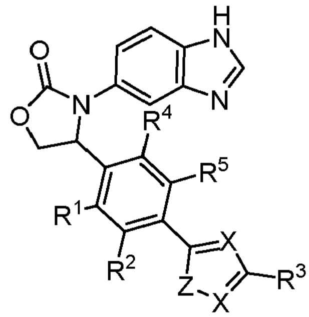

(VI)

wherein each X is independently N or CH,
wherein each Z is independently NH, O or S.

**8.** The compound, or the pharmaceutically acceptable salt, hydrate or solvate thereof, of claim 7, represented by formula (III-a):

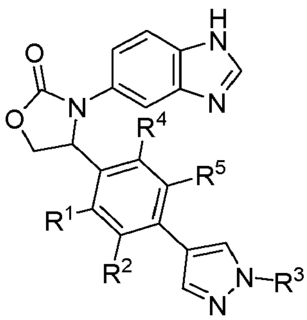

(III-a).

**9.** The compound, or the pharmaceutically acceptable salt, hydrate or solvate thereof, of any one of the preceding claims, wherein $\mathbf{R^3}$ is a C1-C4 fluoroalkyl.

**10.** The compound, or the pharmaceutically acceptable salt, hydrate or solvate thereof, of claim 9, wherein $\mathbf{R^3}$ is $CF_3$.

**11.** The compound, or the pharmaceutically acceptable salt, hydrate or solvate thereof, of any one of the preceding claims, represented by formula (1):

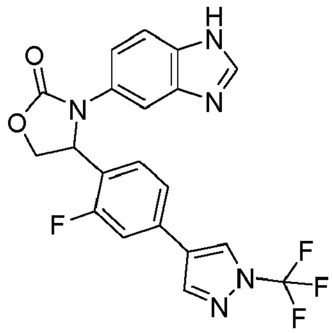

(1).

**12.** The compound, or the pharmaceutically acceptable salt, hydrate or solvate thereof, of any one of the preceding claims, represented by formula (I-S):

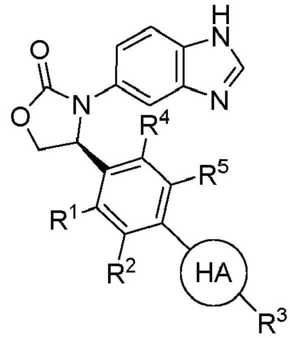

(I-S).

**13.** The compound, or the pharmaceutically acceptable salt, hydrate or solvate thereof, of claim 12, represented by formula (1-S):

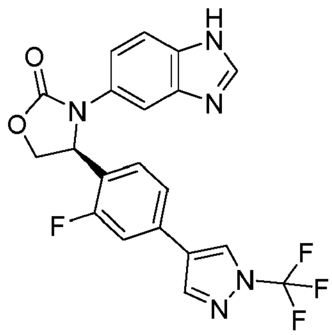

(1-S).

**14.** The compound, or the pharmaceutically acceptable salt, hydrate or solvate thereof, of any one of the preceding claims for use as a medicament.

**15.** The compound, or the pharmaceutically acceptable salt, hydrate or solvate thereof, for use according to claim 14, for use in the treatment of cancer.

Europäisches Patentamt
European Patent Office
Office européen des brevets

# EUROPEAN SEARCH REPORT

Application Number
EP 23 17 0082

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2011/029920 A1 (PROBIODRUG AG [DE]; HEISER ULRICH [DE] ET AL.) 17 March 2011 (2011-03-17) * claim 1; table B * ----- | 1-15 | INV. C07D413/14 C07D417/14 A61P35/00 A61K31/427 A61K31/422 |
| A,D | WO 2021/009068 A1 (SCENIC IMMUNOLOGY B V [NL]) 21 January 2021 (2021-01-21) * compounds FRPPO-144, 147, 148, 149, 150A-C, 151, 164, 165,166, 174, 175, 176, 177. * ----- | 1-15 | |
| A | WO 2014/140279 A1 (PROBIODRUG AG [DE]) 18 September 2014 (2014-09-18) * page 1, line 4 - line 8; claim 1 * ----- | 1-15 | |

TECHNICAL FIELDS SEARCHED (IPC)

C07D
A61P

The present search report has been drawn up for all claims

1

| Place of search | Date of completion of the search | Examiner |
|---|---|---|
| Munich | 28 September 2023 | Bakboord, Joan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 17 0082

This annex lists the patent family members relating to the patent documents cited in the above-mentioned European search report. The members are as contained in the European Patent Office EDP file on
The European Patent Office is in no way liable for these particulars which are merely given for the purpose of information.

28-09-2023

| Patent document cited in search report | Publication date | Patent family member(s) | Publication date |
|---|---|---|---|
| WO 2011029920 A1 | 17-03-2011 | AU 2010294214 A1 | 29-03-2012 |
| | | BR 112012008346 A2 | 15-09-2020 |
| | | CA 2772488 A1 | 17-03-2011 |
| | | CN 102695546 A | 26-09-2012 |
| | | DK 2475428 T3 | 28-09-2015 |
| | | EA 201200474 A1 | 30-10-2012 |
| | | EP 2475428 A1 | 18-07-2012 |
| | | ES 2548913 T3 | 21-10-2015 |
| | | HK 1175135 A1 | 28-06-2013 |
| | | IL 218259 A | 29-09-2016 |
| | | JP 5934645 B2 | 15-06-2016 |
| | | JP 2013504544 A | 07-02-2013 |
| | | KR 20120105421 A | 25-09-2012 |
| | | NZ 598685 A | 31-05-2013 |
| | | PL 2475428 T3 | 31-12-2015 |
| | | SG 178953 A1 | 27-04-2012 |
| | | US 2011092501 A1 | 21-04-2011 |
| | | US 2014065095 A1 | 06-03-2014 |
| | | US 2016039795 A1 | 11-02-2016 |
| | | WO 2011029920 A1 | 17-03-2011 |
| | | ZA 201201366 B | 25-09-2013 |
| WO 2021009068 A1 | 21-01-2021 | AU 2020312647 A1 | 24-02-2022 |
| | | BR 112022000505 A2 | 22-03-2022 |
| | | CA 3147154 A1 | 21-01-2021 |
| | | CN 114466849 A | 10-05-2022 |
| | | EP 3997091 A1 | 18-05-2022 |
| | | IL 289784 A | 01-03-2022 |
| | | JP 2022542540 A | 05-10-2022 |
| | | KR 20220054293 A | 02-05-2022 |
| | | US 2023077723 A1 | 16-03-2023 |
| | | WO 2021009068 A1 | 21-01-2021 |
| WO 2014140279 A1 | 18-09-2014 | AU 2014230249 A1 | 08-10-2015 |
| | | BR 112015023454 A2 | 05-12-2017 |
| | | CA 2904465 A1 | 18-09-2014 |
| | | CN 105263927 A | 20-01-2016 |
| | | CN 108218849 A | 29-06-2018 |
| | | DK 2970235 T3 | 23-09-2019 |
| | | EA 201500930 A1 | 29-04-2016 |
| | | EP 2970235 A1 | 20-01-2016 |
| | | ES 2750645 T3 | 26-03-2020 |
| | | HK 1216531 A1 | 18-11-2016 |
| | | JP 6454651 B2 | 23-01-2019 |
| | | JP 2016513632 A | 16-05-2016 |
| | | KR 20150128928 A | 18-11-2015 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 23 17 0082

This annex lists the patent family members relating to the patent documents cited in the above-mentioned European search report. The members are as contained in the European Patent Office EDP file on
The European Patent Office is in no way liable for these particulars which are merely given for the purpose of information.

28-09-2023

| Patent document cited in search report | Publication date | Patent family member(s) | Publication date |
|---|---|---|---|
| | | KR 20210091830 A | 22-07-2021 |
| | | MX 367855 B | 09-09-2019 |
| | | NZ 712352 A | 29-07-2016 |
| | | PL 2970235 T3 | 31-12-2019 |
| | | SG 11201506609V A | 29-09-2015 |
| | | US 2016031869 A1 | 04-02-2016 |
| | | WO 2014140279 A1 | 18-09-2014 |
| | | ZA 201506156 B | 29-11-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021009068 A
- WO 2011029920 A1


### Non-patent literature cited in the description


- **BERGE et al.** Pharmaceutically Acceptable Salts. *J. Pharm. Sci,* 1977, vol. 66, 1-19
- **T. GREEN ; P. WUTS.** Protective Groups in Organic Synthesis. John Wiley and Sons, 2006
- Remington: The Science and Practice of Pharmacy. Lippinott Williams and Wilkins, 2005
- Remington: The Science and Practice of Pharmacy. Pharmaceutical Press, 2012
- Handbook of Pharmaceutical Excipients. Pharmaceutical Press, 2012
- **KOTECKI et al.** *KBM7 cells,* 1999
- **HUANG et al.** *S53-L382,* 2011